# EUROPEAN PATENT SPECIFICATION

(11) **EP 3 471 743 B1**
(45) Date of publication and mention of the grant of the patent: **06.08.2025**
(21) Application number: 17814207.1
(22) Date of filing: 16.06.2017
(51) Int. Cl.: A61K 35/12, A61K 35/28, A61P 7/00, C12N 5/0789, C12N 5/10, G01N 33/50, G01N 33/569

(54) **STRATEGIES TO ASSESS AND/OR PRODUCE CELL POPULATIONS WITH PREDICTIVE ENGRAFTMENT POTENTIAL**
STRATEGIEN ZUR BEURTEILUNG UND/ODER ERZEUGUNG VON ZELLPOPULATIONEN MIT PRÄDIKTIVEM TRANSPLANTATPOTENZIAL
STRATÉGIES POUR ÉVALUER ET/OU PRODUIRE DES POPULATIONS CELLULAIRES AYANT UN POTENTIEL DE GREFFE PRÉDICTIF

(30) Priority: 17.06.2016 US 201662351761 P; 01.12.2016 US 201662428994 P
(43) Date of publication of application: 24.04.2019
(73) Proprietor: Fred Hutchinson Cancer Center, Seattle, WA 98109 (US)
(72) Inventor: RADTKE, Stefan, Seattle, Washington 98109 (US); ADAIR, Jennifer E., Seattle, Washington 98109 (US); KIEM, Hans-Peter, Seattle, Washington 98109 (US)
(74) Representative: Hobson, David James
(86) International application number: PCT/US2017/037967
(87) International publication number: WO 2017/218948

(56) References cited:
- WO-A1-2016/041080
- US-A1- 2012 164 118
- US-A1- 2013 274 138
- US-A1- 2014 369 973
- US-A1- 2015 064 150
- US-A1- 2016 168 590
- D WISNIEWSKI ET AL: "Further phenotypic characterization of the primitive lineage- CD34+CD38-CD90+CD45RA- hematopoietic stem cell/progenitor cell sub-population isolated from cord blood, mobilized peripheral blood and patients with chronic myelogenous leukemia", LEUKEMIA, vol. 1, no. 9, 1 September 2011 (2011-09-01), London, pages 1 - 11, XP055266958, ISSN: 0887-6924, DOI: 10.1038/bcj.2011.35
- JOS DOMEN ET AL: "Bone Marrow (Hematopoietic) Stem Cells | stemcells.nih.gov", NIH STEM CELL INFORMATION PAGE, 1 January 2016 (2016-01-01), pages 1 - 16, XP055643738, Retrieved from the Internet <URL:https://stemcells.nih.gov/info/Regenerative_Medicine/2006Chapter2.htm> [retrieved on 20191118]
- PETERSON CHRISTOPHER: "Long-term multilineage engraftment of autologous genome-edited HSCs in nohuman primates", BLOOD, vol. 127, no. 20, 28 January 2016 (2016-01-28), pages 2416, XP055643826, Retrieved from the Internet <URL:https://ashpublications.org/blood/article/127/20/2416/35281/Longterm-multilineage-engraftment-of-autologous> DOI: 10.1182/blood-2015-09-672337
- STEFAN RADTKE ET AL: "A distinct hematopoietic stem cell population for rapid multilineage engraftment in nonhuman primates", SCIENCE TRANSLATIONAL MEDICINE, vol. 9, no. 414, 1 November 2017 (2017-11-01), US, pages eaan1145, XP055643183, ISSN: 1946-6234, DOI: 10.1126/scitranslmed.aan1145
- DE GREEF GEORGINE E.: "Lymphocyte and Hematopoietic Stem and Progenitor Cell (HSPC) Subsets Mobilized By Either Plerixafor or G-GCSF: A Retrospective Comparison of Grafts Harvested in Healthy Allogeneic Stem Cell Donors | Blood | American Society of Hematology", BLOOD, 2 December 2006 (2006-12-02), XP055796711, Retrieved from the Internet <URL:https://ashpublications.org/blood/article/124/21/2451/95528/Lymphocyte-and-Hematopoietic-Stem-and-Progenitor> [retrieved on 20210419], DOI: 10.1182/blood.V124.21.2451.2451
- NIGHTINGALE ET AL.: "Transient gene expression by nonintegrating lentiviral vectors", MOL THER, vol. 13, 23 March 2006 (2006-03-23), pages 1121 - 1132, XP005469386

## Description

### FIELD OF THE DISCLOSURE

The present disclosure provides strategies to assess and/or produce cell populations with predictive engraftment potential. The cell populations can be used for a variety of therapeutic and research purposes.

### BACKGROUND OF THE DISCLOSURE

Hematopoietic stem cells (HSC) are the basis for all therapeutic cord blood, bone marrow and mobilized peripheral blood stem cell transplantation approaches, and blood stem cell gene therapy approaches. HSC transplantation can be curative in many non-malignant and malignant disorders, however, even in transplant centers, the current gold standard for isolation, manipulation and expansion of HSC is through use of the CD34 protein on the cellular surface of HSC. Singular use of the CD34 HSC cell surface marker leads to a mixed or heterogeneous population of cells that have distinct phenotypes and characteristics. Due to the heterogeneity of the cell population, there are several major limitations with using CD34 to identify HSCs (1) the absolute numbers of CD34-expressing cells in blood and bone marrow therapeutic products do not quantitatively correlate with clinical kinetics of patient hematopoietic recovery, which leads to unpredictable clinical results, sometimes resulting in engraftment failure, (2) the CD34-expressing cell phenotype does not predict self-renewal, proliferative capacity or blood cell lineage potential, which is required for a normal and balanced hematopoietic compartment, including the immune system, and (3) the numbers of CD34-expressing cells isolated from blood and bone marrow products require large volumes of reagents for manipulation, which can be cost prohibitive if genetic manipulation of the HSCs is required. For example, the volume of genetic manipulation components (e.g., lentivirus) required to transfer a therapeutic gene of interest can encompass up to 25% of a standard production lot of these components. Thus, the field has widely recognized that there continues to be an unmet need to identify a more enriched, true HSC population. Others have identified alternative HSC protein surface markers to enrich for true HSCs, however, they still fail to predict engraftment potential.
Wisniewski D et al (2011), Blood Cancer J., 1(9):e36 describes further phenotypic characterization of the primitive lineage-CD34+CD38-CD90+CD45RA- hematopoietic stem cell/progenitor cell sub-population isolated from cord blood, mobilized peripheral blood and patients with chronic myelogenous leukemia. Domen et al (2016), NIH Stem Cell Information Page, pages 1-16 describes bone marrow (hematopoietic) stem cells. Peterson et al (2016), Blood, 127, 20, 2416-2426 describes long-term multilineage engraftment of autologous genome-edited hematopoietic stem cells in nonhuman primates. De Greef et al (2014), Blood, 124, 21, 2451 describes lymphocyte and hematopoietic stem and progenitor cell subsets mobilized by either plerixafor or G-GCSF.

### SUMMARY OF THE DISCLOSURE

The invention is defined by the claims. The present disclosure provides methods as claimed to isolate and optionally genetically-modify or formulate cell populations with predictive engraftment potential in transplantation. The cell populations can be used in the development and deployment of therapies, among other uses. Methods described herein allow processing and isolation of cells with particular marker profiles that contribute to the predictability of engraftment.

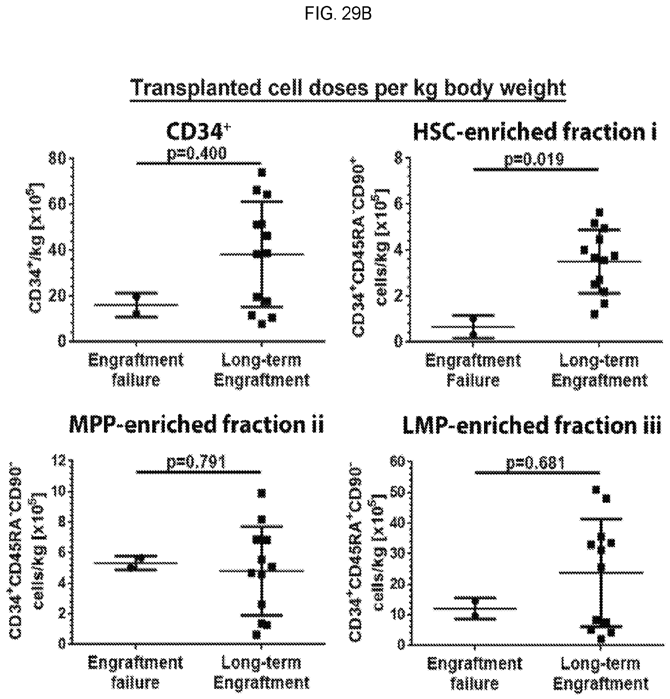

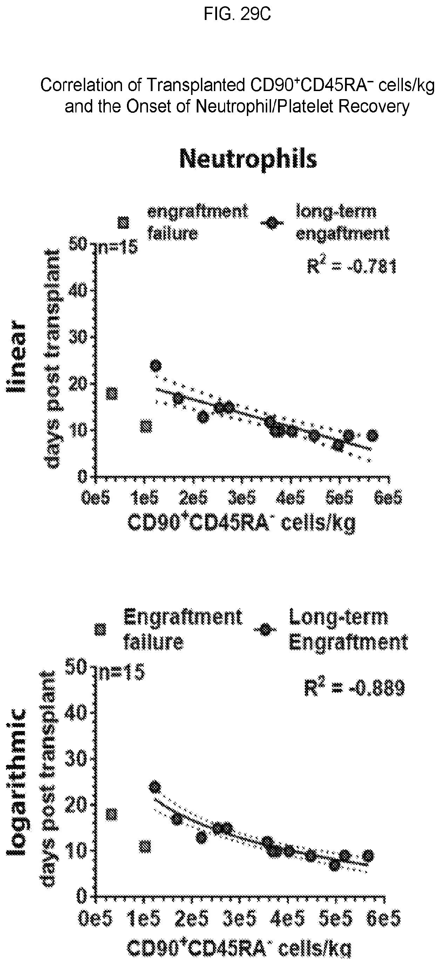

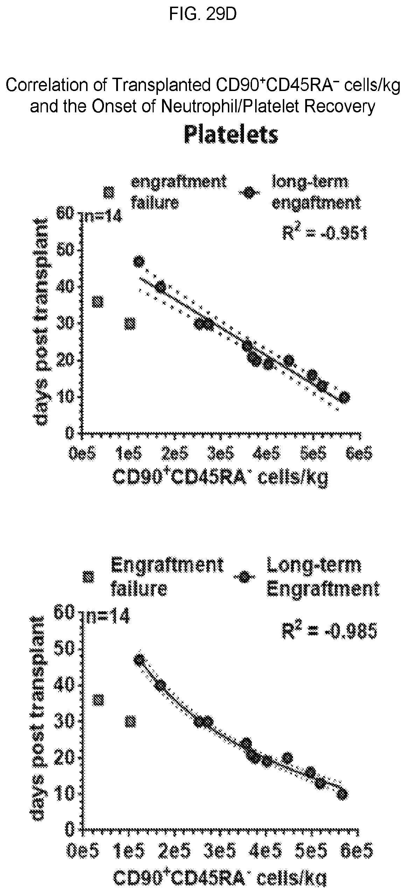

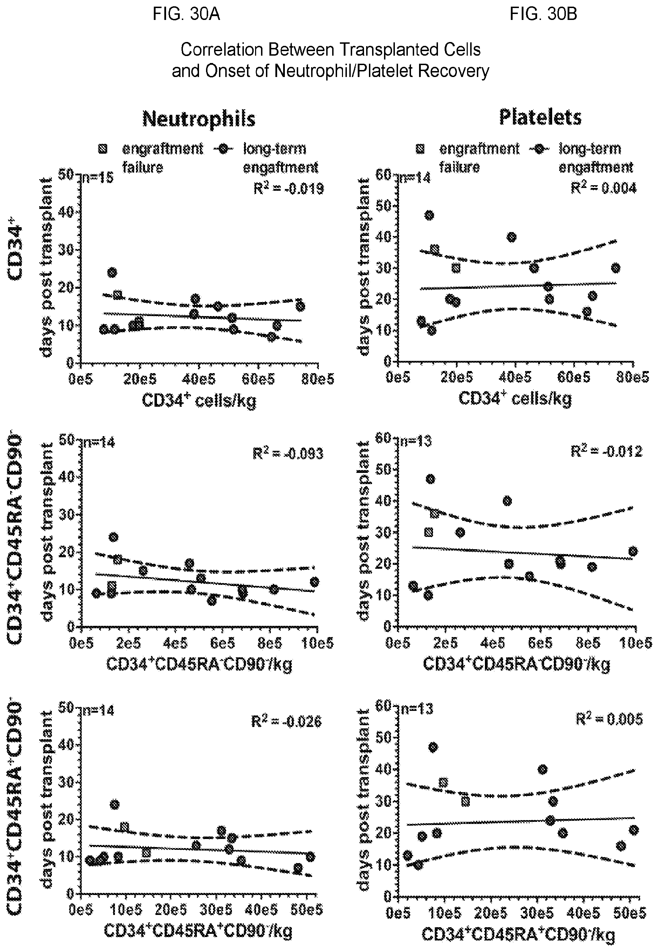

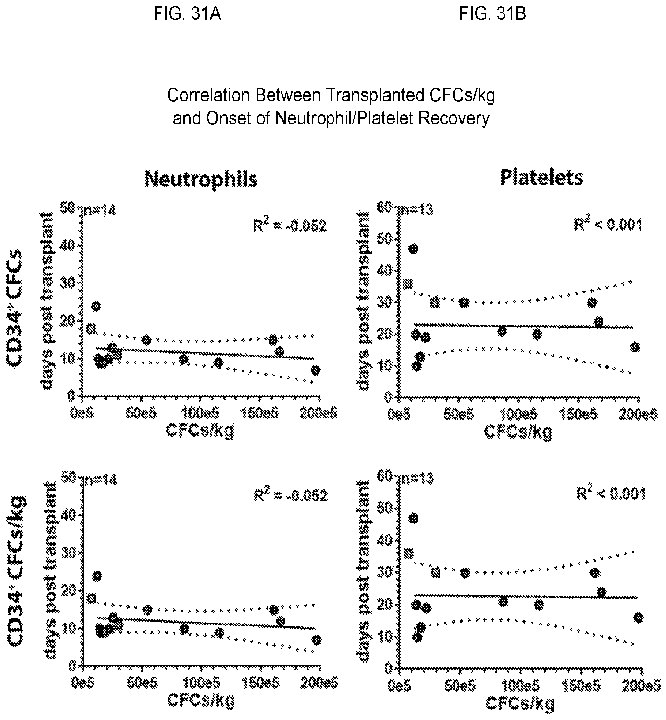

In particular embodiments, populations of HSC with predictive engraftment potential can be isolated by selecting for CD34⁺ cells from the cell source, and sorting for the CD34⁺cell population which is negative for the cell marker CD45RA and positive for the cell marker CD90 on the cell surface which is conserved between humans and nonhuman primates. In particular embodiments, populations of HSC with predictive engraftment potential can be isolated by using the additional cell surface markers of CD133 (human) or CD117 (non-human primate). Each of the foregoing subsets of CD34⁺ cells have self-renewing capacity, multi-lineage potential, long-term engraftment capability and, most importantly, predictably correlate (quantitatively and/or logarithmically) with hematopoietic recovery after transplantation. As an overview of the predictive nature of engraftment of the developed cell populations, compare FIGs. 29C and 29D (developed cell populations) to FIGs. 30A, 30B, 31A, 31B (prior art cell populations). A statistical method known as the Spearman's Rank Correlation coefficient (R²), was used to quantify the significance of the correlation between the two independent variables, cell number and duration/time to recovery post-transplant, as measured by neutrophils (FIG 29C) and platelets (FIG 29D). The absolute R² value of 1 means there is a perfect correlation whereas a value of 0 means there is no correlation between the variables. In light of the analysis performed, only the CD34⁺CD45RA⁻ CD90⁺ population, as compared to CD34⁺, the current gold standard, as well as CD34⁺CD45RA⁻ CD90⁻ and CD34⁺CD45RA⁺CD90⁻ demonstrates a very strong logarithmic correlation with neutrophil and platelet recovery (FIGs 29C and 29D). Furthermore, performing a statistical method known as the Student's T-Test, only the CD34⁺CD45RA⁻CD90⁺ population compared to CD34⁺ as well as CD34⁺CD45RA⁻CD90⁻ and CD34⁺CD45RA⁺CD90⁻ predicts engraftment failure versus successful long-term engraftment (FIG 29B). The predictive engraftment potential of the cell populations produced according to the current disclosure, as well as the frequency of these cell types within the CD34-expressing cell pool dramatically (a) reduces the cost of goods for manufacturing of blood stem cell therapeutics, including gene therapies, (b) provides a quantitative measure of graft potency and a (c) basis for improvement in manipulation and expansion of HSC. As one example, in therapeutic uses, cells isolated solely based on expression of CD34 are clinically administered at a dose of 5-10 million cells / kg. Cell populations of the current disclosure predictably engraft at cell doses as low as 122,000 cells / kg. In addition, to lower cost of goods, the reduced number of cells required for engraftment minimize the volume of cells that need to be administered to the patient which can minimize adverse events upon administration and has the potential to improve clinical outcomes as compared to transplantation with bulk CD34+ cells.

Moreover, the engineering procedures described herein can be applied to embryonic stem cell (ESC), pluripotent stem cell (PSC) and induced pluripotent stem cell (iPSC) therapeutics. These phenotypes can be used to generate engineered cell therapeutics or could be used as a target population for differentiation of previously engineered stem cells such as ESC, PSC and iPSC. The phenotypes described could serve as a measure of success and potency of such engineered cell therapeutics. Additionally, non-engineered cell therapeutics such as platelets, macrophages, red blood cells or other blood cells, could be generated from the cell populations defined herein.

### BRIEF DESCRIPTION OF THE FIGURES

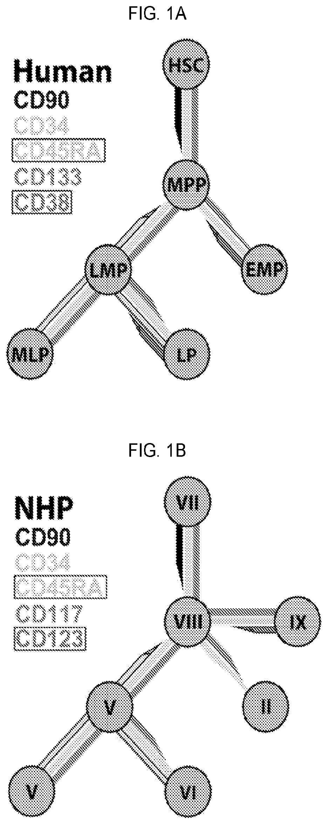

FIGs. 1A and 1B. Conserved lineage relationships in human and nonhuman primates (NHP). (1A) Revised model of human hematopoiesis proposing HSCs (CD34⁺CD45RA⁻ CD90⁺CD133⁺) giving rise to MPPs (CD34⁺CD45RA⁻CD90⁻CD133⁺), followed by a segregation of lympho-myeloid potentials into the CD34⁺CD45RA⁺CD133⁺ cell fraction and erythro-myeloid lineages (EMP) into CD34⁺CD45RA⁻CD133^{low} populations (Görgens *et al.,* 2014; Görgens *et al.,* 2013). (1B) Similar lineage relationships were observed in the NHP using identical cell surface markers but replacing CD133 with CD117, as there is no species cross-reactive CD133 antibody available for NHP. Additional assessment of CD123 expression allowed separation of erythro-myeloid (Population II) and megakaryocyte progenitors (Population IX).

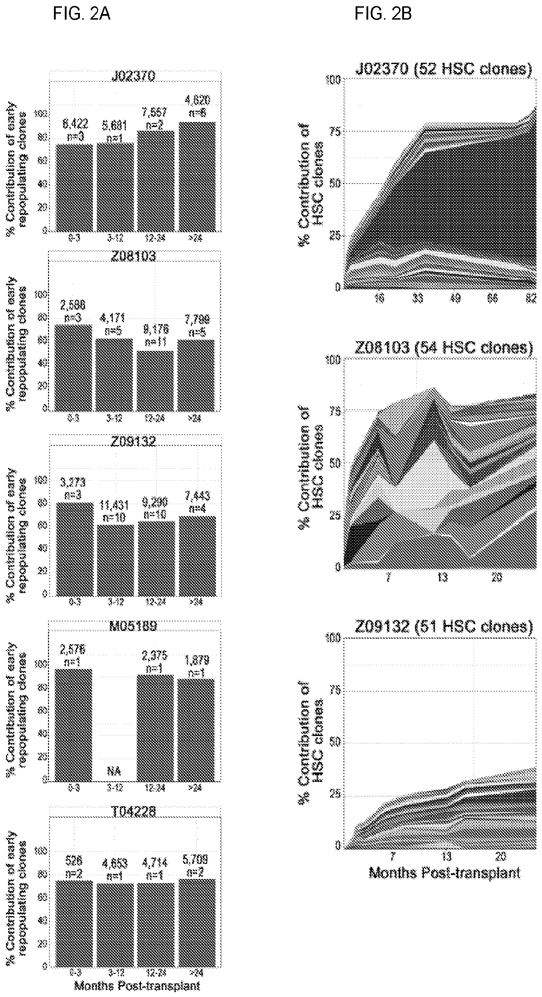

FIGs. 2A and 2B. Persistence of early-engrafting stem-cell like clones over years after transplant. (2A) *In vivo* hematopoietic cell clone tracking data for five retrospectively analyzed animals followed for more than 2 years after myeloablative transplant of lentiviral (LV) gene modified autologous CD34⁺ cells. Bars represent the contribution of clones identified in peripheral blood (PB) <3 months after transplant (first bar in each graph), over time. The number above each bar is the total number of clones identified in the time period; n = number of individual blood samples analyzed during that time period. NA: no samples available within this time period. (2B) Longitudinal contributions of abundant HSC clones (maximum clone contribution > mean maximum contribution for all HSC clones identified in the animal). Each colored band represents one HSC clone. Band width corresponds to the clone frequency (y-axis). To be called an HSC clone, the clone signature (LV integration locus), had to be present in one short-lived and one long-lived mature blood cell lineage and present at more than a single time period of analysis, one of which had to be later than 6 months post-transplant.

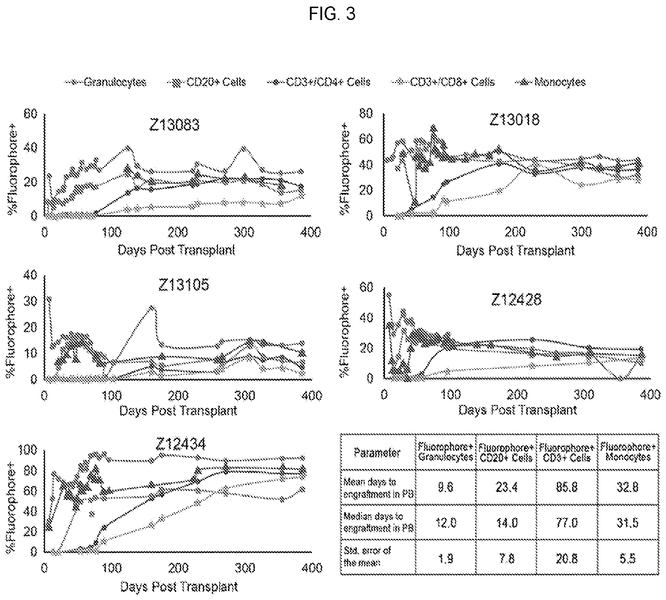

FIG 3. (Related to FIGs. 2A, 2B). Initial multilineage engraftment occurs within 3 months after autologous, myeloablative transplant in NHP. The level of fluorophore⁺ PB subsets including granulocytes (gray dot), monocytes (gray triangle), B cells (CD20⁺ cells; gray box) and T cells (CD3⁺/CD4⁺ cells (black dot) and CD3⁺/CD8⁺ cells (gray star)) measured by flow cytometry over 1+ year after transplant in the myeloablative setting in five macaques. The table summarizes engraftment data as numeric values. The day of engraftment for each lineage is defined as the first day ≥1% fluorophore⁺ cells were observed in PB with consecutive increases over the next three measurements.

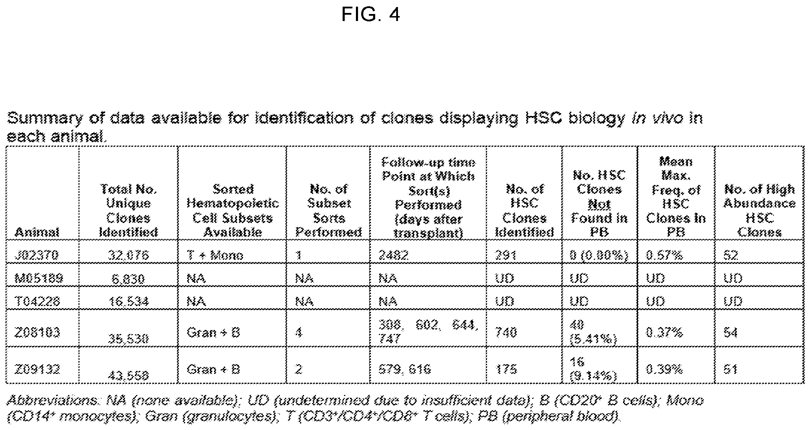

FIG 4. (Related to FIGs. 2A, 2B). Summary of data available for identification of clones displaying HSC biology *in vivo* in each animal.

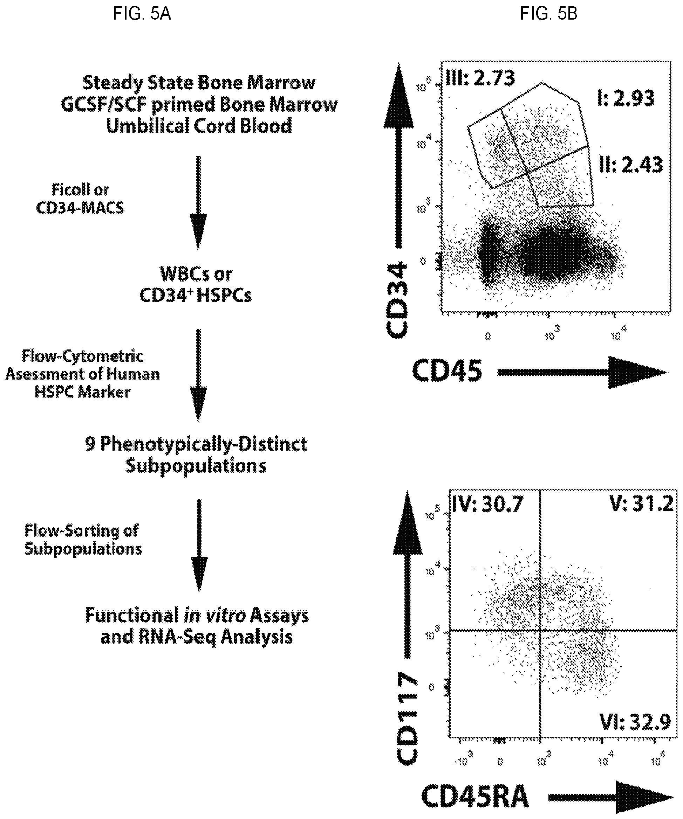

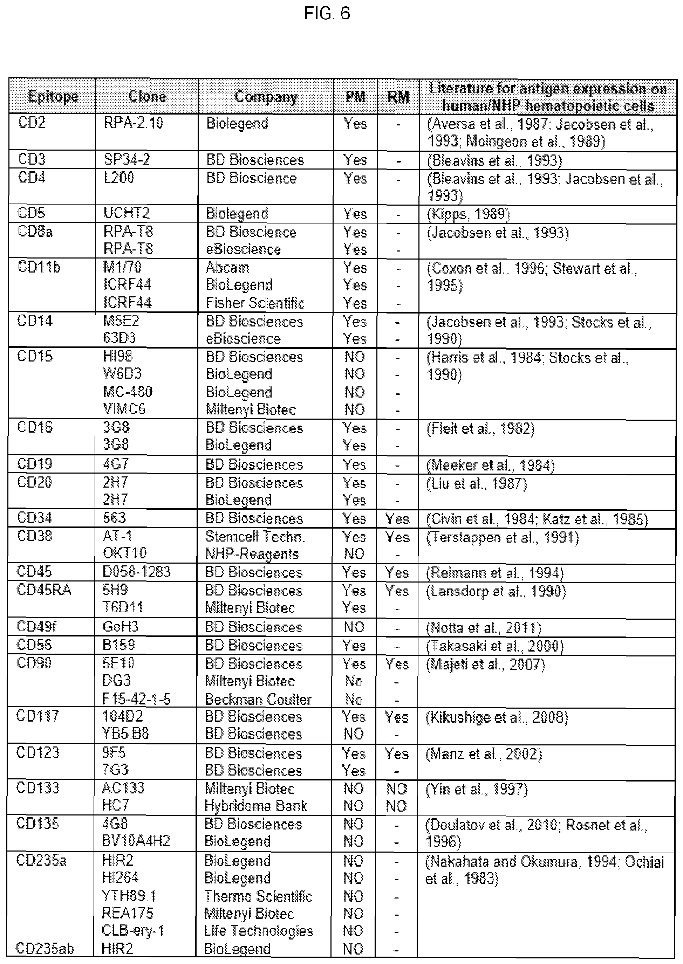

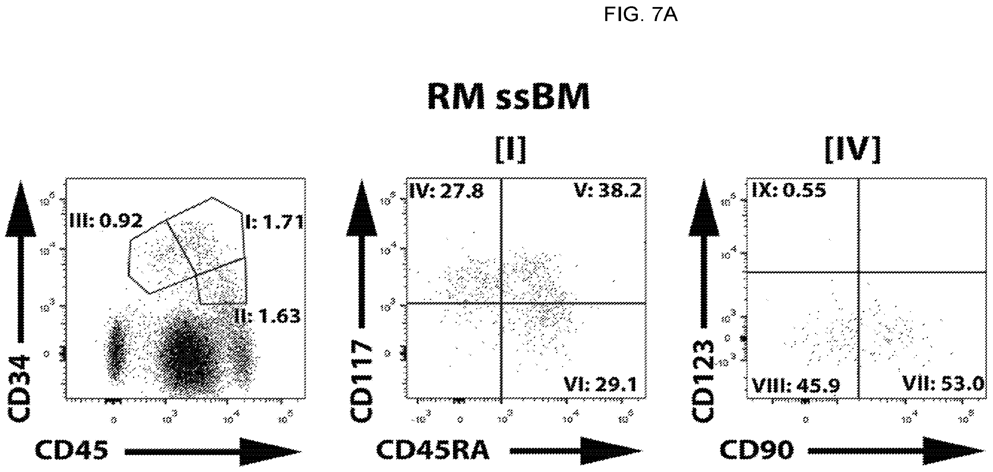

FIGs. 5A-5F. Identification of phenotypically defined pigtail macaque (PM) steady state bone marrow (ssBM)-derived CD34⁺ subpopulations. (5A) Experimental schema. White blood cells (WBCs) or CD34⁺ hematopoietic stem and progenitor cells (HSPCs) were enriched by Ficoll or magnetic-assisted cell-sorting (MACS), respectively. Expression of human HSPC markers was analyzed by flow-cytometry and identified subpopulations were separated by flow-sorting for functional *in vitro* assays as well as RNA-sequencing (RNA-seq). (5B) Cell surface expression of CD45 vs. CD34, CD45RA vs. CD117, and CD90 vs. CD123 on PM ssBM-derived WBC (int = intermediate). CD45RA/CD117 expression is gated on CD34^{high}CD45^{int} (I) populations; CD90/CD123 analysis is shown for CD34^{high}CD45^{int}CD45RA⁻CD117⁺ (IV) cells. (5C) Phenotypically distinct subpopulations were labeled from I-IX for further use throughout the disclosure. (5D) Average frequency of CD34-subpopulations I-IX in ssBM-derived WBCs. (mean ± SEM) (5E) CD34^{high}CD45^{int} (I), CD45RA⁻CD117⁺ (IV) and CD90⁺CD123⁻ (VII) cells were sort-purified (upper row, post sort) and cultured in StemSpan supplemented with either recombinant human stem cell factor (SCF), recombinant human thrombopoietin (TPO) and recombinant human Fms-related Tyrosine Kinase 3 ligand (FLT3-L) (I and VII) or SCF and recombinant human interleukin 3 (IL-3) (IV). Arising progeny were analyzed on day three (lower row, post culture). (5F) Alteration of cell surface marker expression patterns on HSPC subpopulations. See also FIG. 6 and FIGs. 7A-7H.
FIG. 6 (Related to FIGs. 5A-5F) Cross-species reactivity of fluorochrome-conjugated antibodies with PM and RM unmanipulated ssBM-derived white blood cells and HSPCs.

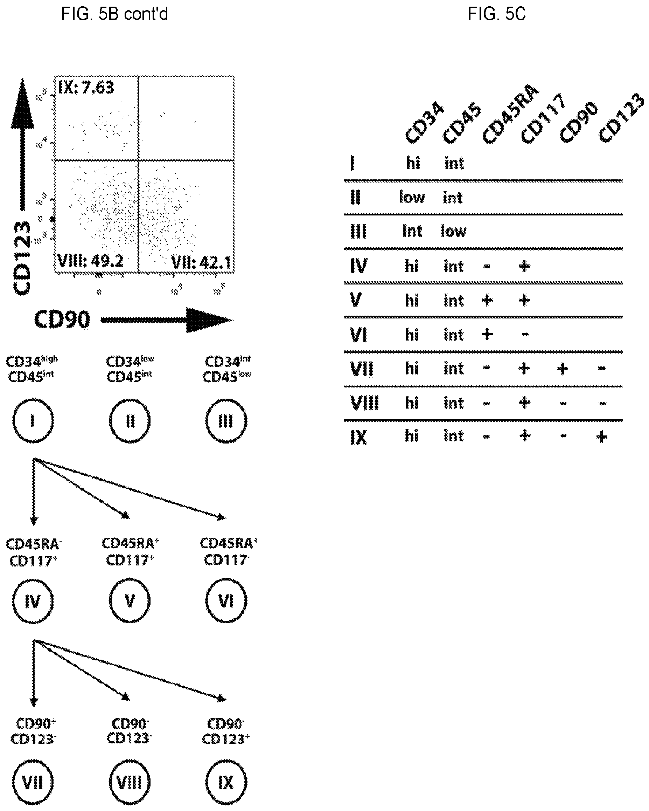

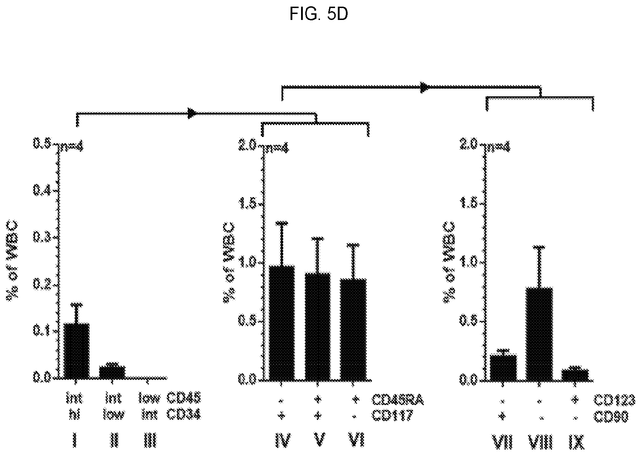

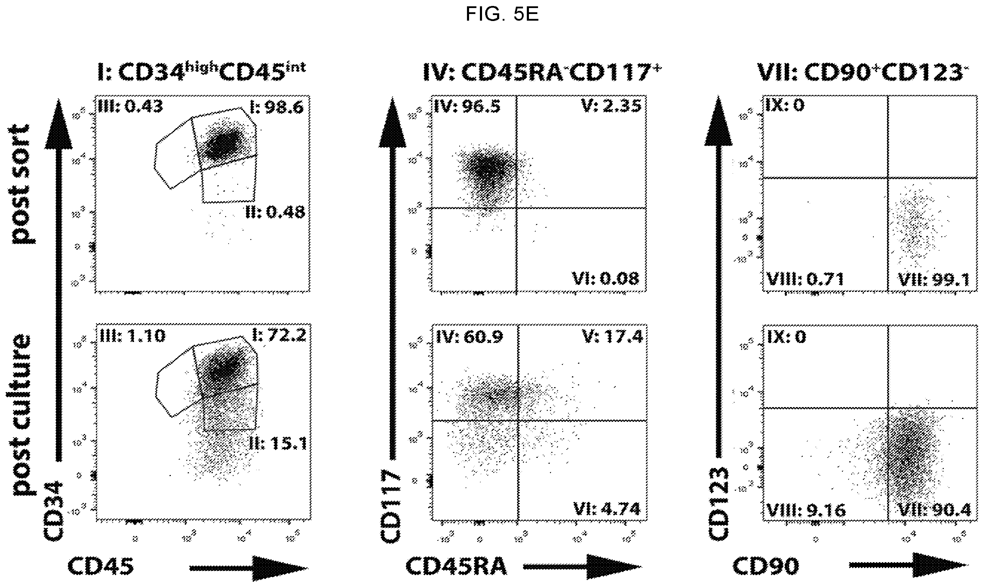

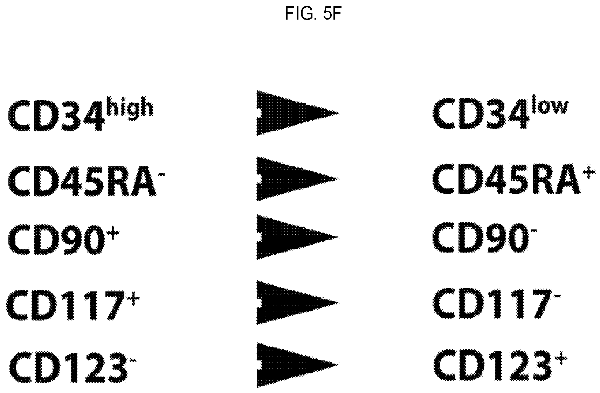

FIGs. 7A-7H (Related to FIGs. 5A-5F). HSPC composition and *ex vivo* expansion of sort-purified HSPC subpopulations from different stem cell sources in RM and PM. (7A-7C) Cell surface expression of CD45 vs. CD34, CD45RA vs. CD117 and CD90 vs. CD123 on (7A) RM ssBM, (7B) PM growth factor primed bone marrow (pBM) and (7C) PM umbilical cord blood (UCB) WBCs. Within shown stem cell sources, CD45RA/CD117 expression is gated on CD34^{high}CD45^{int} (I) populations; CD90/CD123 analysis is shown for CD34^{high}CD45^{int}CD45RA⁻CD117⁺ (IV) cells. (7D-7F) Average frequency of CD34-subpopulations I-IX (defined in FIG. 5) in (7D) RM ssBM WBCs, (7E) PM pBM and (7F) PM UCB. (7G) CD34^{low}CD45^{int} (II), CD34^{int}CD45^{low} (III), CD45RA⁺CD117⁺ (V), CD45RA⁺CD117⁻ (VI), CD90⁻CD123⁻ (VIII) and CD90⁻CD123⁺ (IX) populations from PM ssBM were sort-purified (upper row, post sort) and cultured in StemSpan supplemented with either recombinant human SCF, recombinant human TPO and recombinant human FLT3-L (II, III, VIII and IX) or SCF and recombinant human IL-3 (V and VI). Arising progeny were analyzed on day 3 (lower row, post culture). (7H) Fold expansion (total height of bars) and phenotypical composition of progeny (coded, stacked) derived from sort-purified PM ssBM CD34-subpopulations cultured in StemSpan supplemented with either SCF, TPO and FLT3-L (I, II, III, VII, VIII and IX) or SCF and IL-3 (IV, V and VI) for 3 days. (All values given as mean ± SEM).

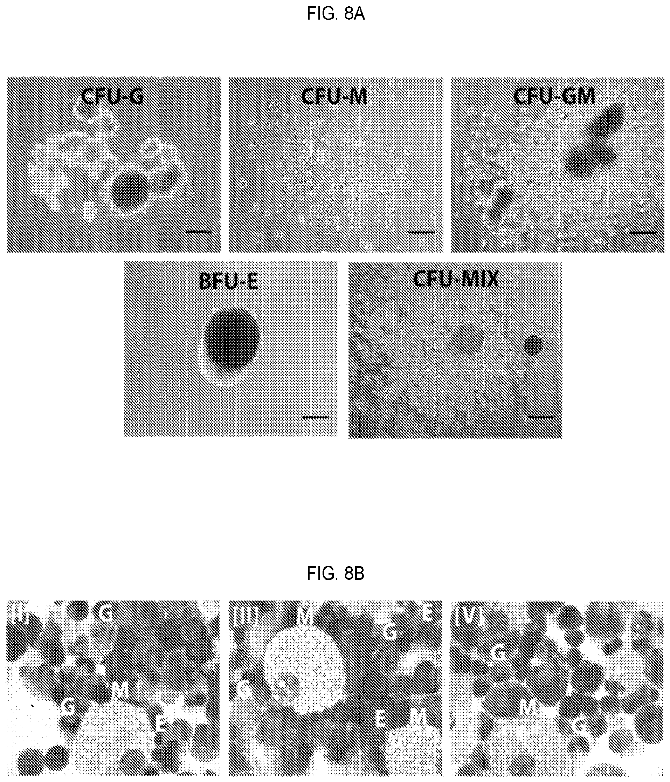

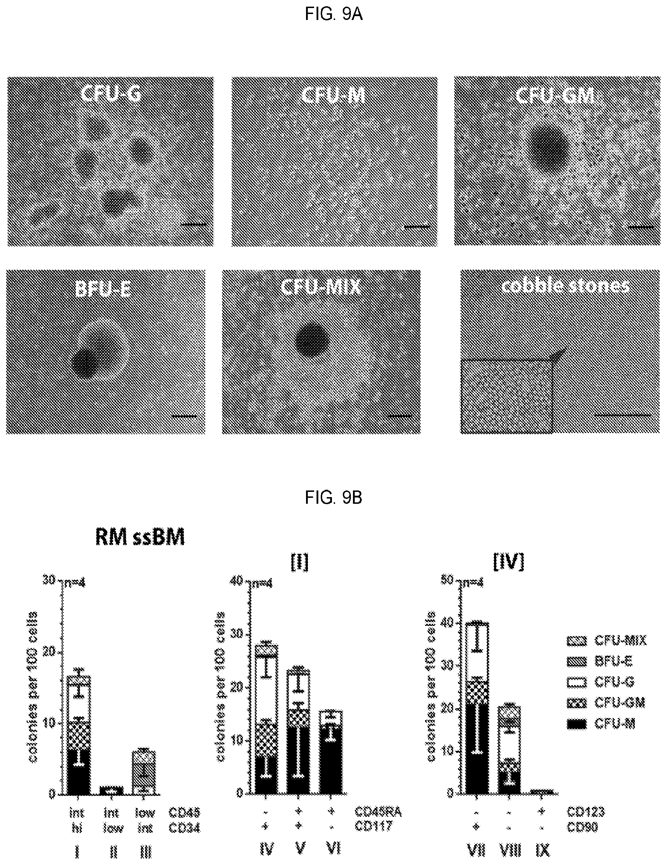

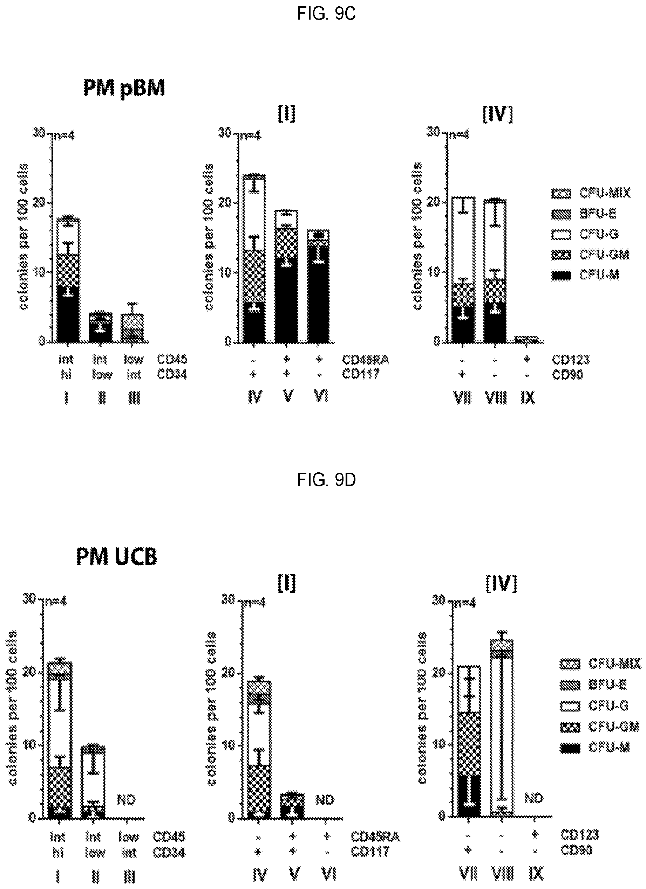

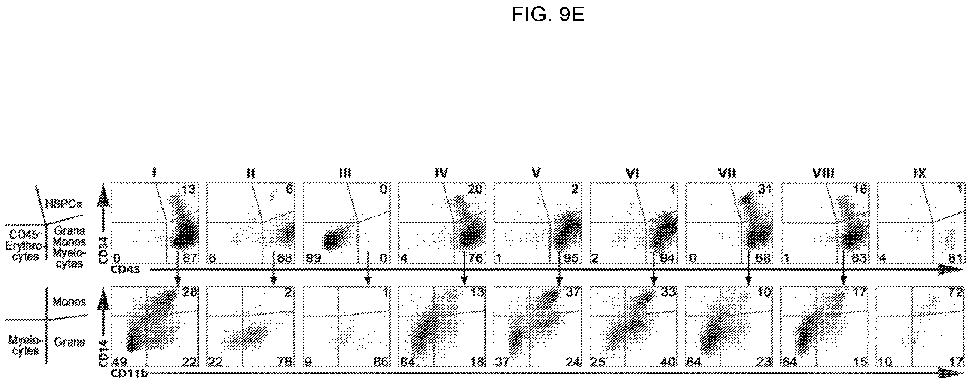

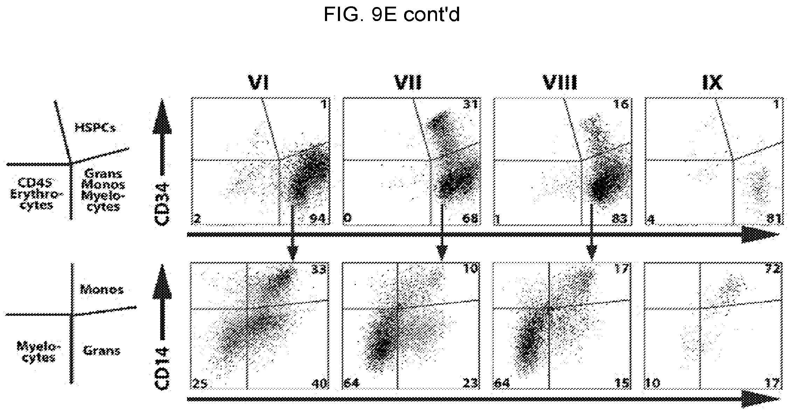

FIGs. 8A-8E. Restriction of erythro-myeloid differentiation potentials into CD45RA⁻ /CD34^{low} populations and segregation of megakaryocytic potentials into CD123⁺ populations. (8A) Representative images of different colony-subtypes of sort-purified PM ssBM-derived CD34⁺ cells. Arising colonies were identified as colony forming unit-(CFU) granulocyte (CFU-G), macrophage (CFU-M), granulocyte-macrophage (CFU-GM), and burst forming unit-erythrocyte (BFU-E). Colonies including erythroid and myeloid cells were scored as CFU-MIX. (scale bar = 100 µm) (8B) CFU assays were harvested, cells washed twice with PBS, concentrated via cytospin on glass slides, and stained with Hematoxylin and Eosin in order to discriminate monocytes/macrophages (M), granulocytes (G), erythrocytes (E) progenitors, and undifferentiated progenitor cells. Representative images of cytospin-enriched subsets for granulocytes, monocytes/macrophages, erythrocytes and myelocytes in Population I, erythrocytes and monocytes in Population II and granulocytes, monocytes/macrophages and myelocytes in Population V. (8C) Colony-forming frequency of previously defined CD34-subpopulations I-IX. The different colony-subtypes are coded as indicated. (8D) Representative images of myeloid (upper picture, scale bar 100µm) and megakaryocytic (lower picture, scale bar 100µm, inlet 40x magnification) colonies generated in MegaCult assays. (8E) Frequency of progenitor cells with myeloid (black bars) and megakaryocyte (gray bars) colony-forming potential within CD34-subpopulations I-IX.(mean ± SEM) See also FIGs. 9A-9F.
FIGs. 9A-9F. (Related to FIGs. 8A-8E). Colony-forming potential of freshly isolated and cultured HSPC subpopulations from different stem cell sources in RM and PM. (9A) Representative images of different colony-subtypes of sort-purified RM ssBM-derived CD34⁺ cells. Arising colonies were identified as CFU-G, CFU-M, CFU-GM, and BFU-E. Colonies including erythroid and myeloid cells were scored as CFU-MIX (scale bar = 100 µm). (9B-9D) Colony-forming frequency of (9B) RM ssBM, (9C) PM pBM and (9D) PM UCB-derived CD34-subpopulations I-IX. The different colony-subtypes are legend-coded as indicated. (9E) To confirm the discrimination of colony-types in primary colony-forming cell (CFC) assays from freshly isolated NHP-HSPCs, total colonies were harvested, washed and stained for flow cytometry. CFC assay contained erythrocytes (CD45⁻), HSPCs (CD34⁺CD45⁺), monocytes (CD11b⁺CD14⁺CD34⁻CD45⁺) and granulocytes (CD11b⁺CD14⁻CD34⁻CD45⁺). (9F) Colony-forming frequency of CD34-subpopulations from PM ssBM cultured in StemSpan media supplemented with either SCF, TPO and FLT3-L (I, II, III, VII, VIII and IX) or SCF and IL-3 (IV, V and VI) for 3 days.

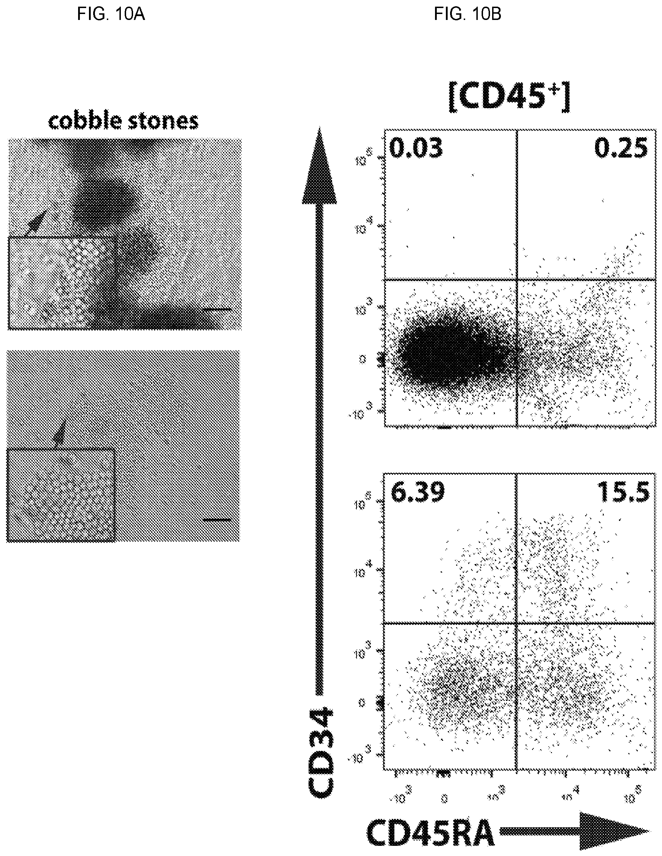

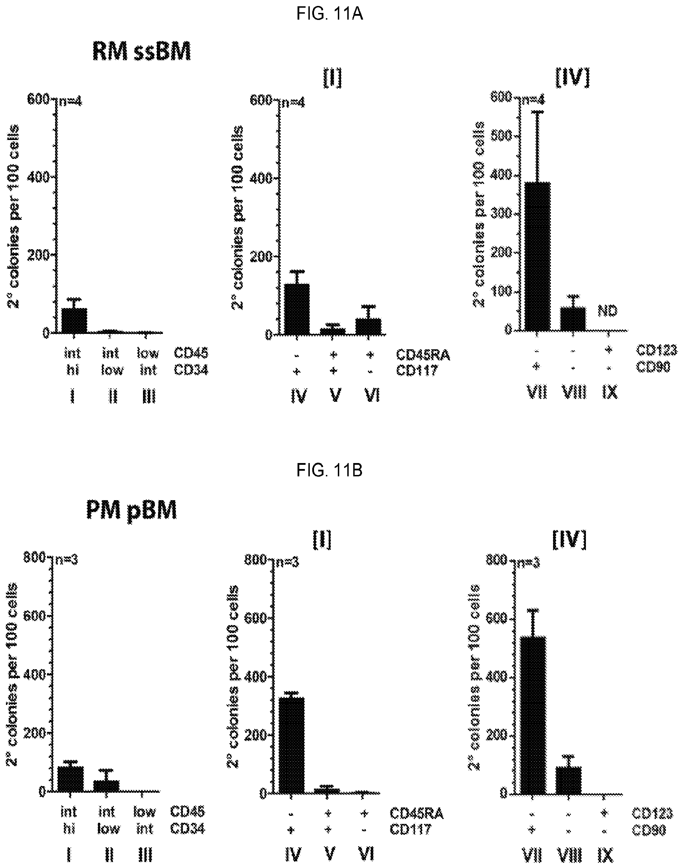

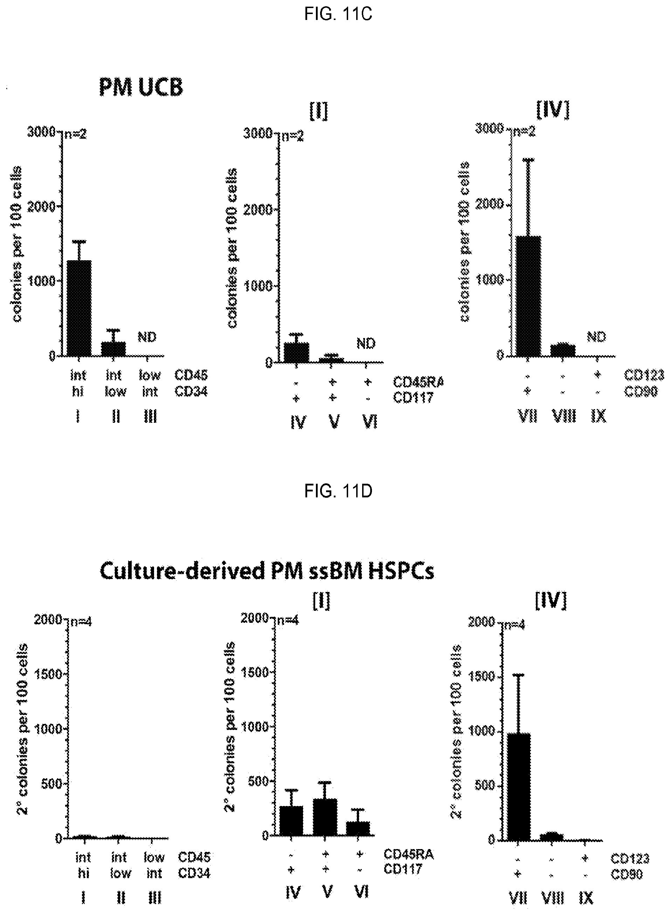

FIGs. 10A-10D. Enrichment of primitive HSPCs in CD90⁺ populations. (10A) Representative images of cobblestone area forming cell (CAFC) colonies. Cobblestones are either associated with differentiated colonies (upper picture, scale bar 100µm) or autonomous (lower picture, scale bar 20µm, inlay 100x magnification). (10B) Cobblestones associated with colonies (upper row) and autonomous (lower row) were harvested for analysis. After exclusion of non-hematopoietic cells and erythrocytes (CD45⁻), expression of the progenitor markers CD34 and CD45RA were analyzed flow-cytometrically. (10C) Single cobblestones from primary CFC assays of Populations VII and VIII were harvested, washed, and re-plated to evaluate secondary colony-forming potential. Within secondary CFC assays, myeloid (G/M/GM) and erythroid/erythro-myeloid (BFU-E/CFU-MIX) differentiation potential of CAFC was evaluated. (10D) Total primary CFC assays were harvested from four independent experiments, washed, and cells re-plated to evaluate secondary colony-forming potential of CD34-subpopulations I-IX. Within secondary CFC assays, only total colony-forming potential was evaluated without discrimination of colony-subtypes. (mean ± SEM). See also FIGs. 11A-11D.
FIGs. 11A-11D (Related to FIGs. 10A-10D). Secondary colony-forming potential of freshly isolated and cultured HSPC subpopulations from different stem cell sources in RM and PM. Primary CFC assays of (11A) RM ssBM, PM (11B) pBM, (11C) UCB, and (11D) culture-derived PM ssBM CD34-subpopulations were harvested, washed and cells re-plated to evaluate secondary colony-forming potential. Within secondary CFC assays, total colony-forming potential was evaluated without discrimination of colony-subtypes.

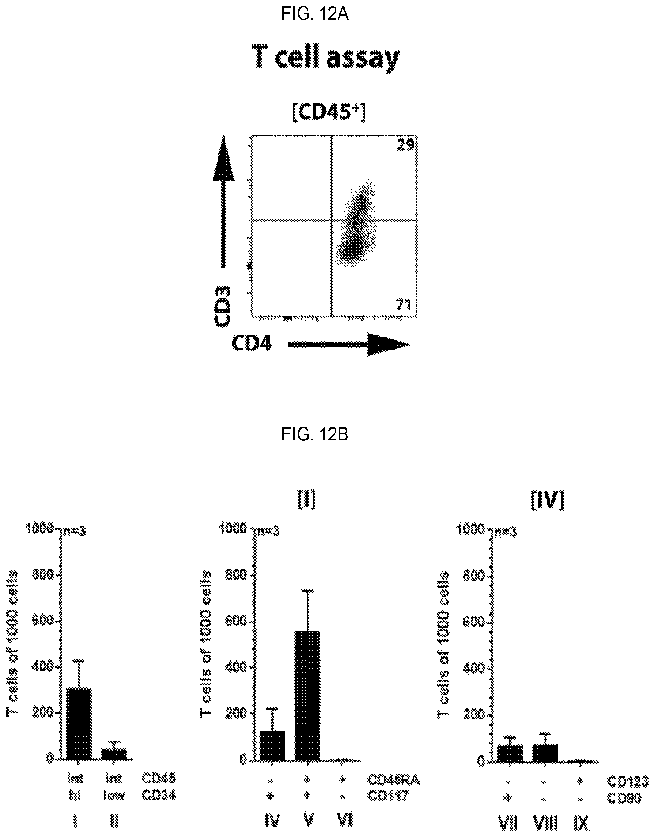

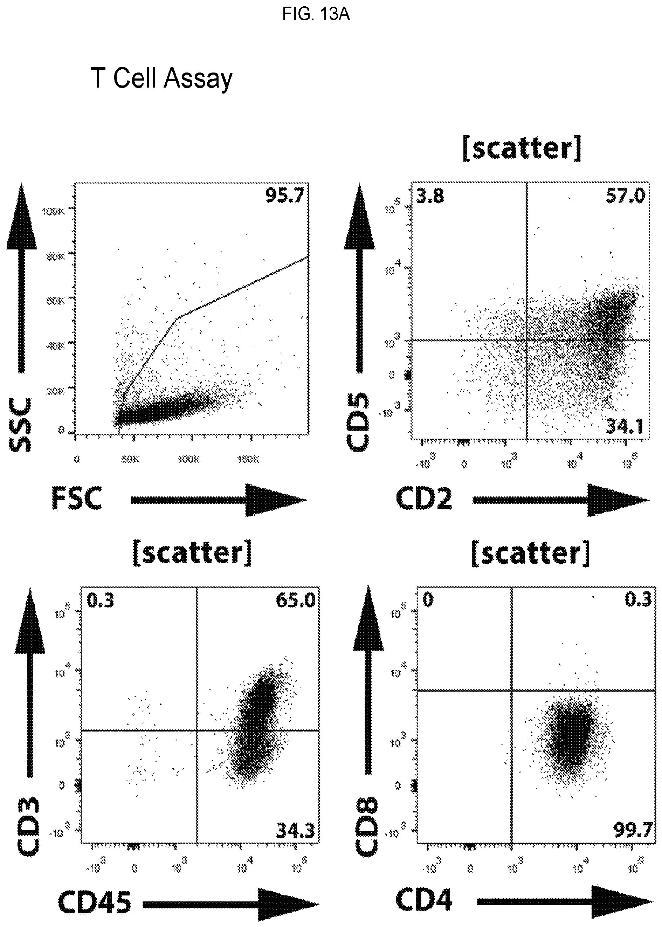

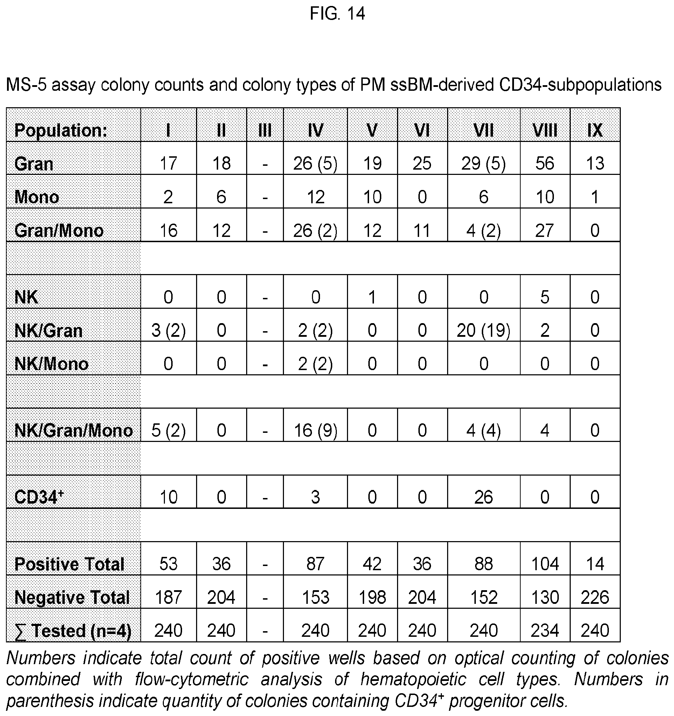

FIGs. 12A-12E. Segregation of T and NK cell potentials into CD45RA⁺ cell fractions. (12A) PM ssBM-derived CD34-subpopulations were sort-purified and introduced into an *in vitro* T cell assay. Representative flow cytometric analysis of CD3⁺CD4⁺ T cells. A detailed gating-strategy for the T cell analysis is shown in FIG. 13A. (12B) Quantification of phenotypical T cells derived from 1000 sort-purified progenitor cells. (12C) Experimental strategy for the clonogenic multi-lineage MS-5 differentiation assay. MS-5 stroma cells were seeded in 96-well plates 5 days prior to single cell deposition of CD34⁺ subpopulations. Cells were expanded/differentiated in H5100 media supplemented with SCF, TPO, recombinant human interleukin 7 (IL-7), recombinant human granulocyte colony stimulating factor (G-CSF), granulocyte/macrophage colony stimulating factor (GM-CSF) and macrophage colony stimulating factor (M-CSF) for 35 days with a weekly 50% medium exchange. After 34 days, wells containing hematopoietic colonies were microscopically enumerated. On day 35 of culture, positive and negative wells were harvested, and hematopoietic cells flow cytometrically analyzed for the expression of macrophage (CD11b⁺CD14⁺), granulocyte (CD11b⁺CD14⁻), NK cell (CD56⁺), and B cell (CD20⁺) markers. (12D) Representative flow cytometric analysis of hematopoietic colonies containing myeloid or lymphoid, and mixed myeloid-lymphoid blood cells. Upper row (CD11b vs. CD14) is gated on CD45⁺ NHP cells to exclude MS-5 stroma cells (not shown). The lower row (CD20 vs. CD56) is gated on CD11b⁻CD14⁻ non-myeloid cells from the upper panel. Numbers in flow-plots indicate percentage of marker positive cells. Individual wells of a 96-well plate with a minimum of five CD45⁺ NHP cells were counted as positive (FIG. 14). (12E) Frequency of progenitor cells in PM ssBM-derived CD34-subpopulations I-IX with clonogenic myeloid (granulocyte, macrophage, granulocyte-macrophage), lymphoid (NK cell, B cell), and lympho-myeloid (granulocyte-NK, monocyte-NK, granulocyte-monocyte-NK) differentiation potential (mean ± SEM). See also FIGs. 13A-13C and FIG. 14.
FIGs. 13A-13C (Related to FIGs. 12A-12E). Enrichment of primitive CD34⁺ HSPCs with clonogenic multi-lineage potential in CD45RA⁻CD90⁺ cell fractions. (13A) Detailed gating-strategy for the flow cytometric analysis and quantification of *in vitro* differentiated mature and immature T cells. Within T cell assays hematopoietic cells expressing the T cell marker CD3, CD4 or CD8 were gated and quantified separately. (13B) Representative flow cytometric analysis of colonies with myeloid, lymphoid or myeloid/lymphoid blood cells containing CD34⁺ progenitor cells in the MS-5 assay. Upper row (CD11b vs. CD14) is gated on CD45⁺ NHP cells to exclude MS-5 stroma cells (data not shown). The second row (CD20 vs. CD56) is gated on CD11b⁻CD14⁻ cells from the upper panel. The bottom row (CD20 vs. CD34) is gated on CD11b⁻CD14⁻CD20⁻CD56⁻ hematopoietic cells. Numbers in flow-plots indicate percentage of marker positive cells. Individual wells of a 96-well plate with a minimum of five CD45⁺ NHP cells were counted as positive (FIG. 14, number in parentheses). (13C) Frequency of progenitor cells in PM ssBM-derived CD34-subpopulations I-IX with myeloid, lymphoid or lympho-myeloid differentiation potential also containing undifferentiated CD34⁺ HSPCs. (mean ± SEM).
FIG. 14 (Related to FIGs. 12A-12E and FIGs. 13A-13C). MS-5 assay colony counts and colony types of PM ssBM-derived CD34-subpopulations.

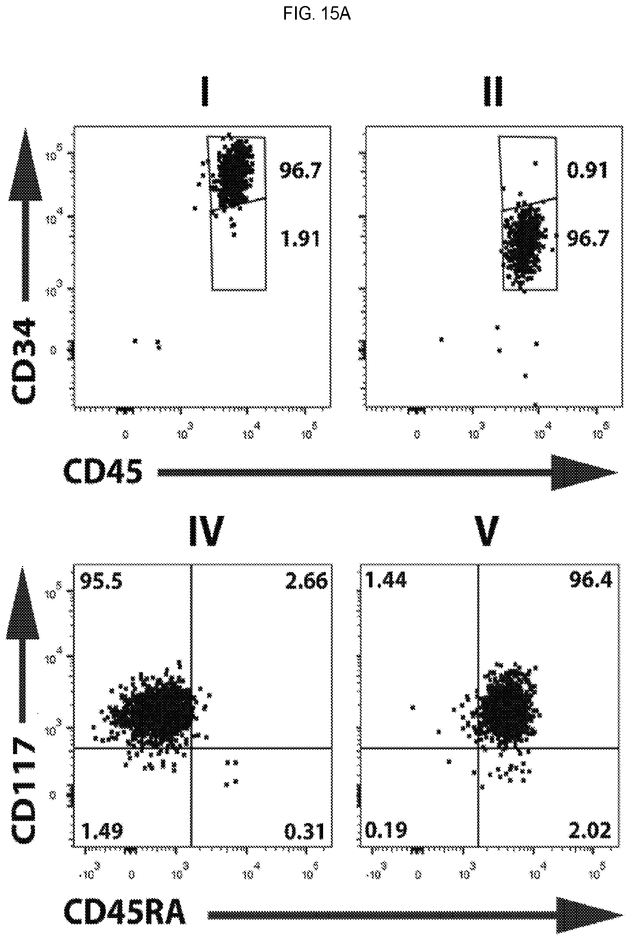

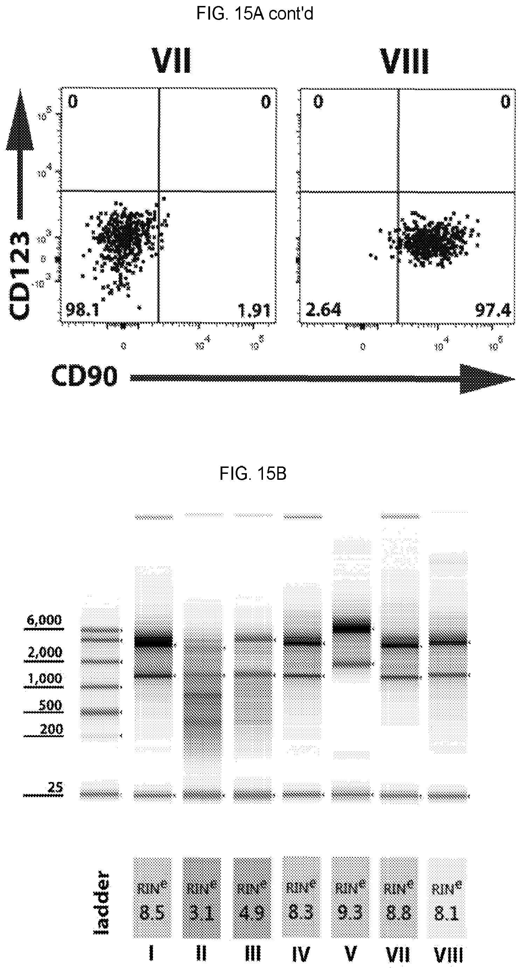

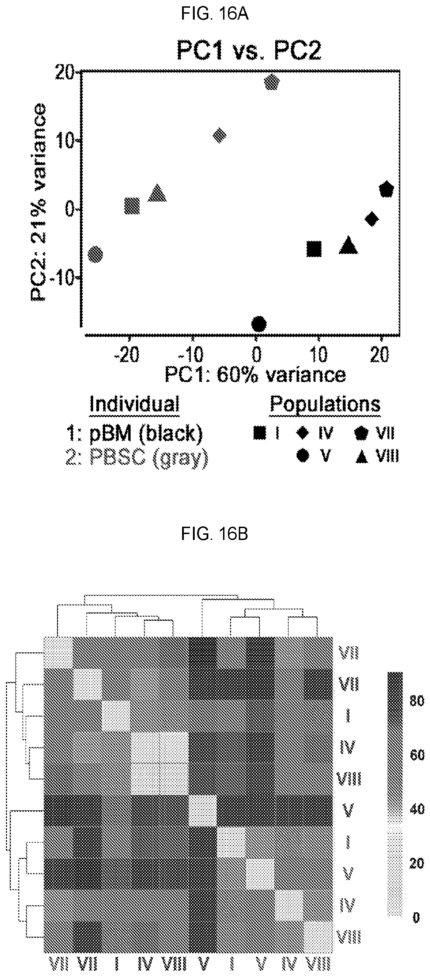

FIGs. 15A-15D (Related to FIGs. 16A-16C). Quality control of cell sorting, RNA extraction and RNA sequencing for transcriptome analysis of NHP HSPC subpopulations. (15A) Flow cytometric analysis of sort-purified NHP HSPC subpopulations I, II, IV, V, VII and VIII. (15B) Concentration and integrity of isolated RNA from sort-purified HSPC subpopulations were tested by electrophoresis calculating the ratio of the ribosomal subunits 28S/18S (RIN^{e} score: RNA integrity number). Samples with degraded RNA showing a RIN^{e} score < 8.0 were excluded from RNA sequencing. (15C) Total number of reads (black bars) as well as reads mapping to the RM (gray bars) and human (white bars) genomes of RNA sequencing data from pBM- and PBSC-derived HSPC subpopulations. (15D) Total number of reads mapped to the human genome located within an exon of an annotated gene for pBM- (black bars) and PBSC-derived (white bars) HSPC subpopulations.

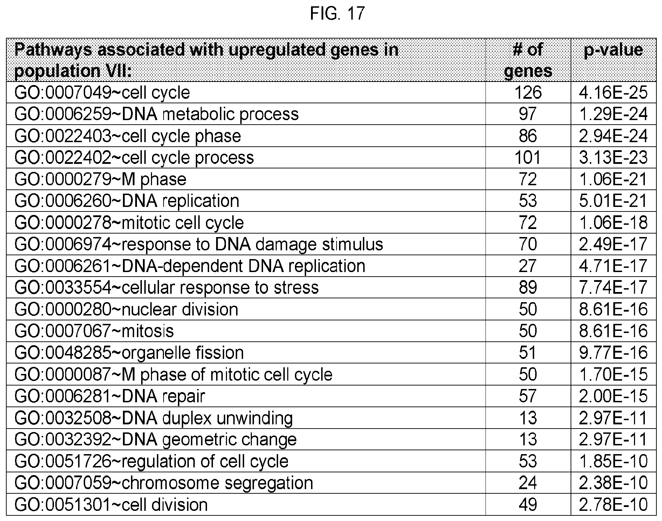

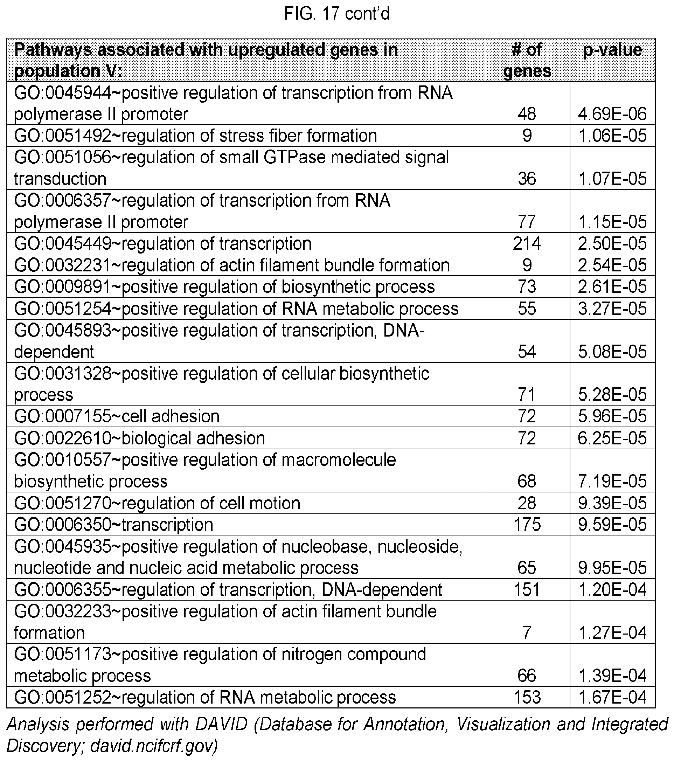

FIGs. 16A-16C. RNA-seq of NHP HSPC subpopulations. (16A) Principle component (PC) analysis and (16B) unsupervised hierarchical clustering of PM pBM- (black) and PBSC-derived (gray) HSPC subpopulations. (16C) To identify differentially expressed genes in the NHP HSPC subpopulations, a pairwise comparison of RNA expression levels was performed. Up/down-regulated genes (p<0.05) in the scatter plots are highlighted in light gray. Corresponding numbers of differentially expressed genes (↑) as well as pathways (→) associated with identified genes (DAVID analysis) are given for each comparison in the opposing half of the table. See also FIGs. 15A-15D and FIG. 17.
FIG. 17. (Related to FIGs. 16A-16C) Top 20 pathways upregulated in Populations VII and V.

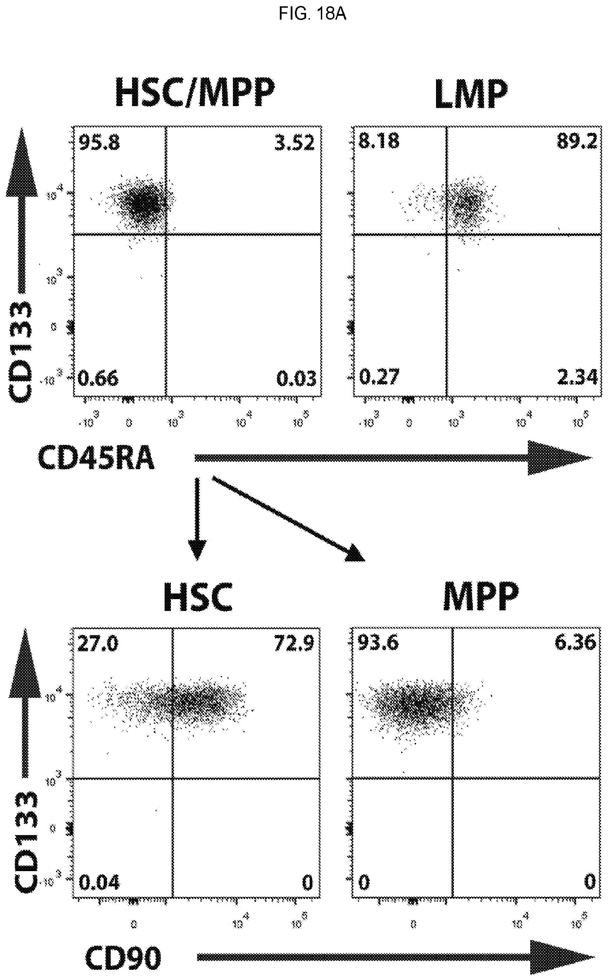

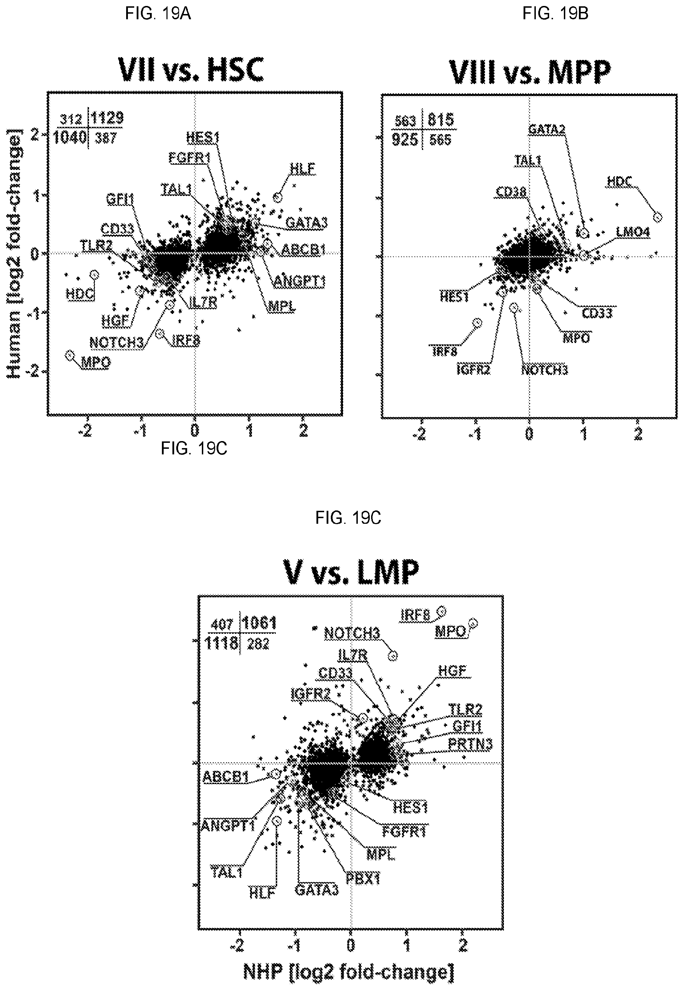

FIGs. 18A-18D (Related to FIGs. 19A-19C). Quality control of cell sorting, RNA extraction and RNA sequencing for transcriptome analysis of human HSPC subpopulations. (18A) Flow cytometric analysis of sort-purified human HSC-, MPP- and LMP-enriched (lympho-myeloid progenitors) cell fractions. (18B) Concentration and integrity of isolated RNA from sort-purified HSPC subpopulations was tested by electrophoresis calculating the ratio of the ribosomal subunits 28S/18S (RIN^{e} score: RNA integrity number). (18C) Total number of reads (black bars) and reads mapping to the human genome (white bars) of RNA sequencing data from PBSC-derived HSPC subpopulations. (18D) Total number of reads mapped to the human genome located within an exon of an annotated gene.
FIGs. 19A-19C. Conservation of RNA expression patterns in corresponding human and NHP HSPC subpopulations. (19A-19C) For the comparison of RNA expression levels in corresponding human and NHP HSPC subpopulations, the log₂ fold-change of gene expression was calculated for each species and gene individually. The number of genes for each quadrant is given in the upper left corner of the scatter plot, with genes showing the same pattern (lower left and upper right quadrant) highlighted in bold. Key regulators for distinct hematopoietic lineages are tagged and highlighted (Notta *et al.,* 2015; Paul *et al.,* 2015). (19A) VII vs. HSC; (19B) VIII vs. MPP; (19C) V vs. LMP. See also FIGs. 18A-18D.

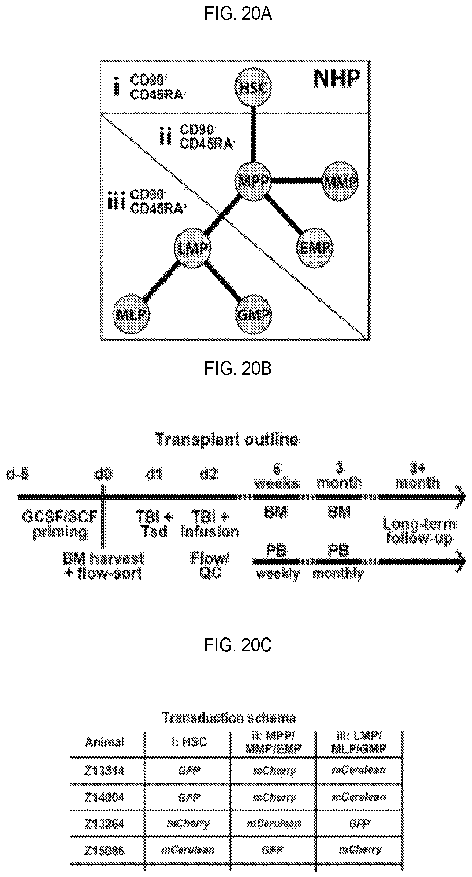

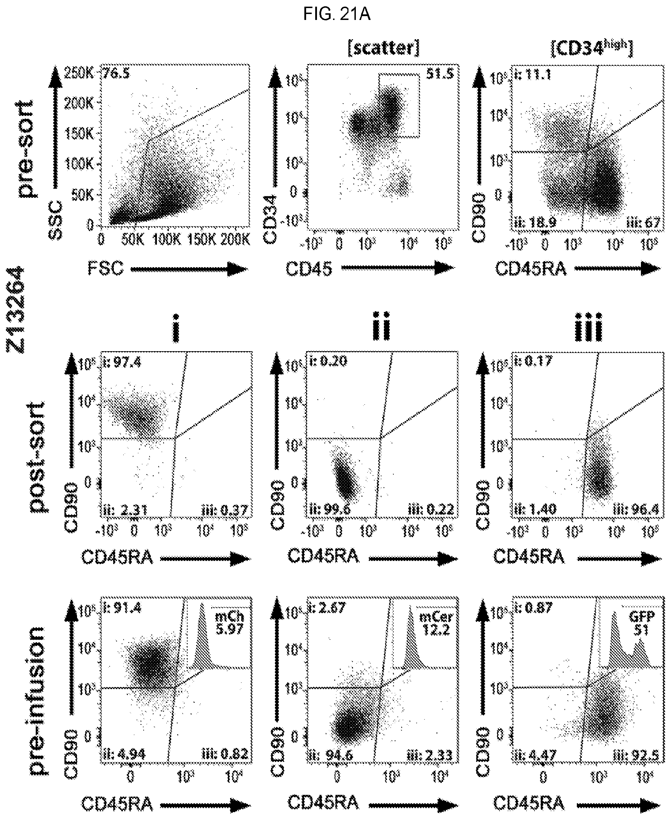

FIGs. 20A-20F. Efficient separation and lentiviral transduction of HSPC subpopulations for competitive repopulation experiments in an autologous NHP transplant model. (20A) Hierarchical organization of HSPCs in the nonhuman primate. Within CD34-enriched fractions of GCSF/SCF-primed bone marrow (BM) aspirates, 3 groups of phenotypically-distinct HSPCs were separated by flow cytometric sorting based on the cell surface expression of CD45RA and CD90. CD34⁺CD45RA⁻CD90⁺ fractions (i) are enriched for true HSCs, CD34⁺CD45RA⁻CD90⁻ fractions (ii) for MPPs (multipotent progenitors), MMPs (myelo-megakaryocytic progenitor) and EMPs (erythro-myeloid progenitors) and CD34⁺CD45RA⁺CD90⁻ fractions (iii) for LMPs (lympho-myeloid progenitors), MLP (multi-lymphoid progenitors) and GMP (granulocyte-macrophage/monocyte progenitor). (20B) Experimental schema and time line for the autologous stem cell transplant including time points for total body irradiation (TBI: 4x 255cGy), flow-sort, transduction, BM draws and long-term follow-up. (20C) A total of four animals were used for autologous stem cell transplants. For the first two animals (Z13314 and Z14004) fraction i was transduced with lentiviral vectors encoding for GFP (green fluorescent protein), fraction ii with mCherry (mCh), and fraction iii with mCerulean (mCer). For the third animal (Z13264) colors were rotated with fraction i transduced with mCh, fraction ii with mCer, and fraction iii with GFP. For the fourth animal (Z15086), fraction i was transduced with mCer, fraction ii with GFP, and fraction iii with mCh. (20D) Representative flow cytometric quality control of the CD34-enriched cell fraction (pre-sort), flow-sorted fractions i, ii, and iii (post-sort) and lentiviral transduced fractions i, ii, and iii (pre-infusion) for Z14004. The transduction efficiency (% cells expressing respective fluorochrome) of fraction i, ii, and iii at 24 hours after transduction is indicated in the inlaid histogram in the top right corner of the pre-infusion flow plots (bottom row). (20E) Flow-sorted cells from fraction i, ii, and iii from the CD34-enriched cell fraction (pre-sort), flow-sorted (post-sort) and transduced (pre-infusion) were introduced into CFC assays. After 14 days the frequency of primary CFC assays (1^{st}) was determined counting arising colonies identified as CFU-G, CFU-M, CFU-GM, and BFU-E. Colonies including erythroid and myeloid cells were scored as CFU-MIX. The different colony-subtypes are coded as indicated in the figure legend shown in (20F). Total primary CFC assays were harvested, washed, and cells re-plated to evaluate secondary CFC potential (2^{nd}). Within secondary CFC assays, only total CFC potential was evaluated without discrimination of colony-subtypes. (20F) Individual erythroid, myeloid and erythro-myeloid colonies from primary CFC assays in (20E) were extracted for detection of integrated lentiviral elements in the genome by polymerase chain reaction (PCR). The efficiency of transduction for each colony-subtype in fraction i, ii, and iii is visualized individually (coded, N/A = not available). See also FIGs. 21A-21F.
FIGs. 21A-21F (Related to FIGs. 20A-20F). Efficient separation and lentiviral transduction of HSPC subpopulations for competitive repopulations experiments in an autologous NHP transplant model. (21A, 21C, 21E) Representative flow cytometric quality control of the CD34-enriched cell fraction (pre-sort), flow-sorted fractions i, ii, and iii (post-sort) and lentiviral transduced fractions i, ii, and iii (pre-infusion) for Z13264, Z13314 and Z15086. The transduction efficiency (% cells expressing respective fluorochrome) of fraction i, ii, and iii is indicated in the histogram-inlay in the top-right corner of the pre-infusion flow plots (bottom row). (21B, 21D, 21F) Flow-sorted cells from fraction i, ii, and iii from the CD34-enriched cell fraction (pre-sort), flow-sorted (post-sort) and transduced (pre-infusion) were introduced into CFC assays. After 14 days the frequency of primary CFC assays (1^{st}) was determined counting arising colonies identified as CFU-G (white), CFU-M (black), CFU-GM (black-white checkered), and BFU-E (dark gray). Colonies including erythroid and myeloid cells were scored as CFU-MIX (gray-white checkered). The different colony-subtypes are coded as indicated in the figure legend. Total primary CFC assays were harvested, washed, and cells re-plated to evaluate secondary CFC potential (2^{nd}). Within secondary CFC assays, only total CFC potential was evaluated without discrimination of colony-subtypes.

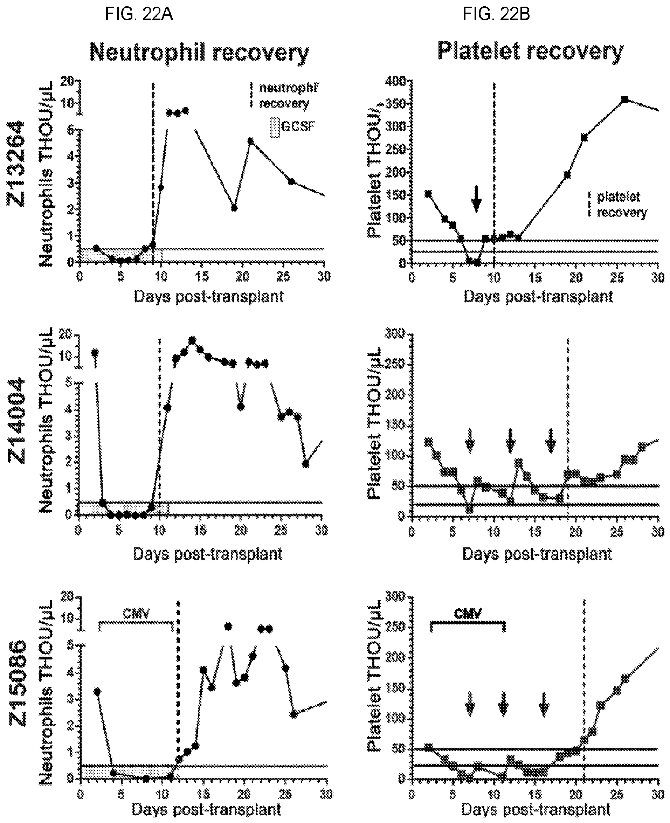

FIGs. 22A-22E. Neutrophil/platelet recovery, long-term multi-lineage engraftment and bone marrow reconstitution, clinical hallmarks for engraftment success, is exclusively driven by CD90⁺CD45RA⁻ cell fractions in the NHP transplant model. Short-term follow-up of (22A) neutrophil and (22B) platelet recovery following myeloablative total body irradiation and administration of lentivirus gene modified autologous hematopoietic stem cells. (22A) The day of neutrophil engraftment (dashed line) was defined using the clinical standards of a minimum of 500 neutrophils per µl PB (solid horizontal line) for a duration of at least 3 consecutive days. The duration of GCSF administration was not considered in this definition and is indicated by the gray bar in each graph. (22B) The day of platelet engraftment (dashed line) was defined as a minimum of 20,000 platelets per µl PB (lower solid horizontal line) for a duration of at least 7 consecutive days, and trending toward a self-sustained increase in platelet counts without transfusion reaching greater than 50,000 platelets per µl (upper solid horizontal line). (22C) Long-term follow-up of gene-marking in white blood cells (WBCs). In animal Z13264 fraction i was transduced with mCh, ii with mCer, and iii with GFP, in Z14004 fraction i expressed GFP, ii mCh, and iii mCer and in Z15086 fraction i expressed mCer, fraction ii expressed GFP, and fraction iii expressed mCh. (22D) Flow cytometric analysis of gene-marked granulocytes (CD11b⁺CD14⁻), monocytes (CD11b⁺CD14⁺), B cells (CD11b⁻CD20⁺), T cells (CD11b⁻CD3⁺), NK cells (CD11b⁻CD16⁺), platelets (CD45⁻CD61⁺) and erythrocytes (CD45⁻) in the PB of a control animal (upper row) and Z14004 (lower row). (22E) Frequency of granulocytes (black triangle), monocytes (black squares), T cell (red circle), B cells (green triangle) and NK cells (yellow squares) in the PB of Z13264, Z14004, and Z15086 (left three graphs) and frequency of gene-marked cells in each blood-lineage (right three graphs).

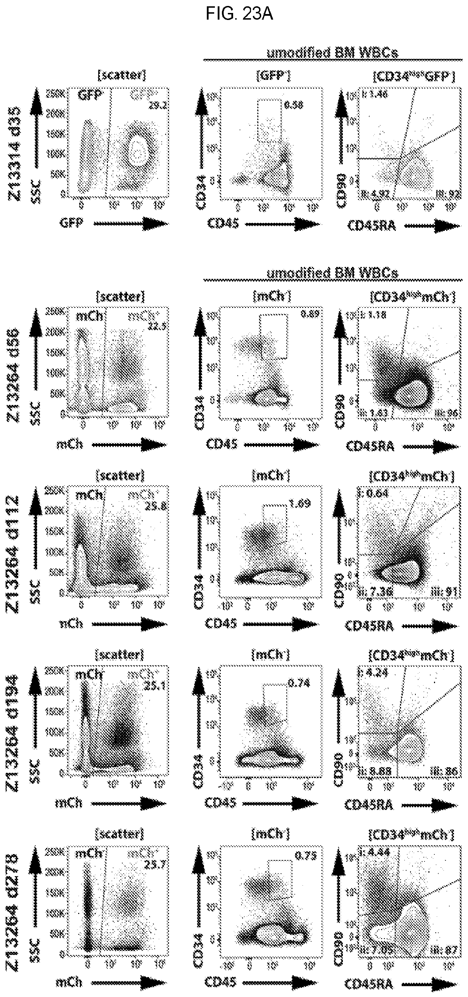

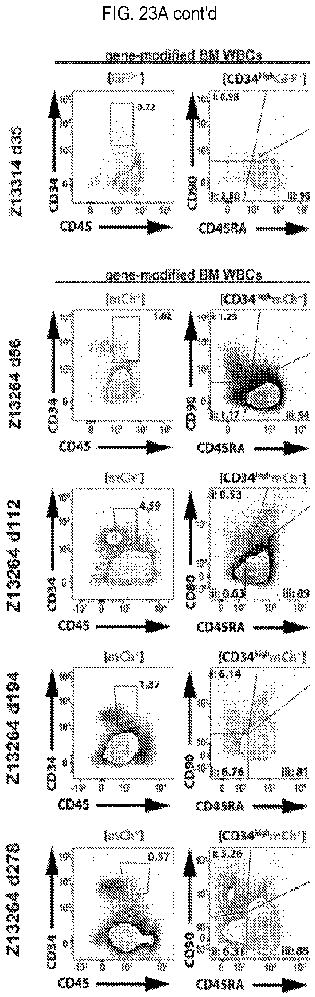

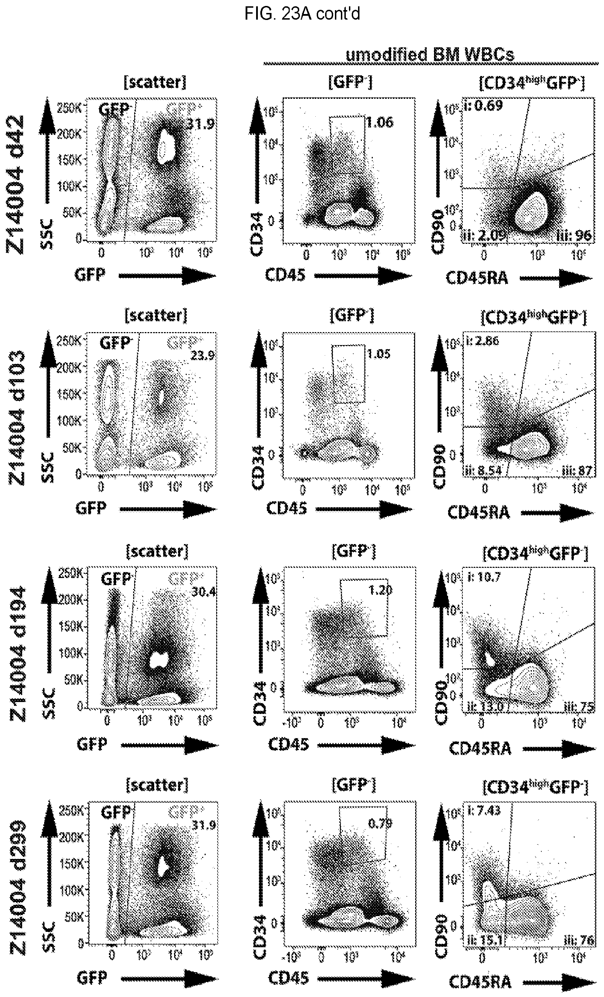

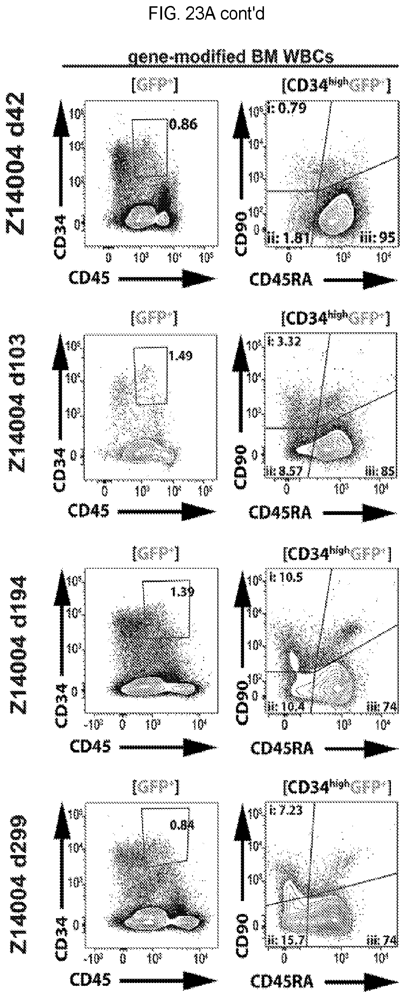

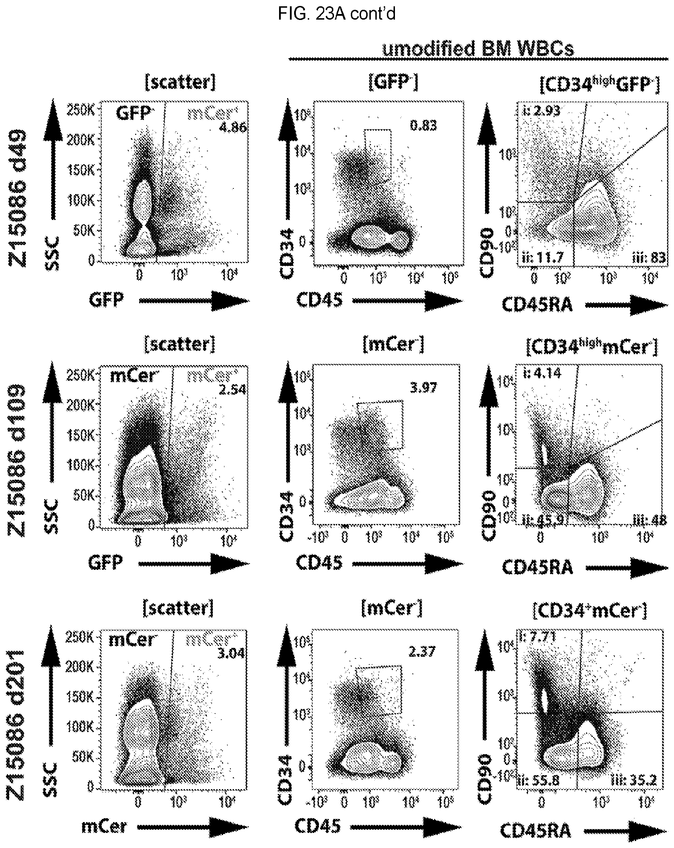

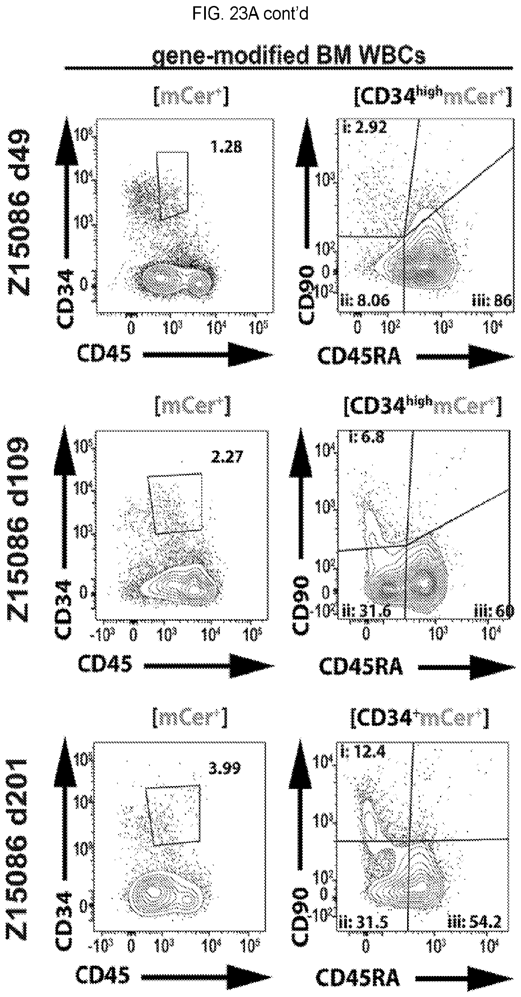

FIGs. 23A-23C. Flow cytometric analysis of engrafted HSPCs in the repopulated bone marrow of Z13314, Z13264, Z14004, and Z15086 at various time points (6 weeks, 3 months, 6 months and 9 months) after transplant. (23A) BM-resident white blood cells (WBCs, far left plots) demonstrate similar frequencies of gene-marking compared to PB-WBCs. Unmodified and gene-modified BM-WBCs contained equivalent frequencies of CD45^{int}CD34⁺ HSPCs (2^{nd} and 4^{th} plot from the left), with similar proportions of unmodified and gene-modified HSPCs from fraction i, ii, and iii (3^{rd} and 5^{th} plot from the left). (23B) Unmodified and gene-modified BM-derived CD34⁺ and HSPCs of fraction i+ii, i, ii, and iii from Z13314, Z13264, Z14004, and Z15086 were sort-purified and introduced into CFC assays. After 14 days the frequency of primary CFC assays was determined counting arising colonies identified as CFU-G, CFU-M, CFU-GM, and BFU-E. Colonies including erythroid and myeloid cells were scored as CFU-MIX. The different colony-subtypes are coded as indicated in the figure legend. (23C) Frequencies of unmodified and gene-modified fraction i, ii, and iii in the bone marrow stem cell niche in Z13314, Z13264, Z14004, and Z15086 over time compared to the stem cell product before *ex vivo* manipulation (d0). The different fractions are coded as indicated in the figure legend.

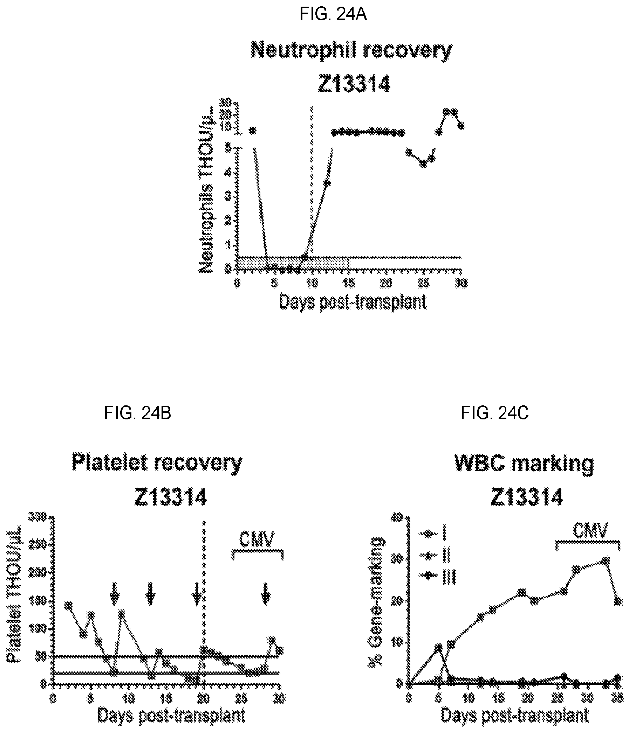

FIGs. 24A-24D. Neutrophil/platelet recovery and long-term multi-lineage engraftment in Z13314, Z13264, Z14004, and Z15086. Short-term follow-up of (24A) neutrophil and (24B) platelet recovery following myeloablative total body irradiation and administration of lentivirus gene modified autologous HSC for animal Z13314. (24A) The day of neutrophil engraftment (dashed line) and (24B) the day of platelet engraftment (dashed line) were as defined in relation to FIG. 22A, 22B. (24C) Long-term follow-up of genetic modification using fluorophores to mark and easily interrogate the status of the respective populations in white blood cells (WBCs). In animal Z13314 fraction i expressed GFP, ii mCh, and iii mCer. (24D) Long-term follow-up of neutrophil and platelets in Z13264 (upper two graphs), Z14004 (middle two graphs), and Z15086 (lower two graphs).

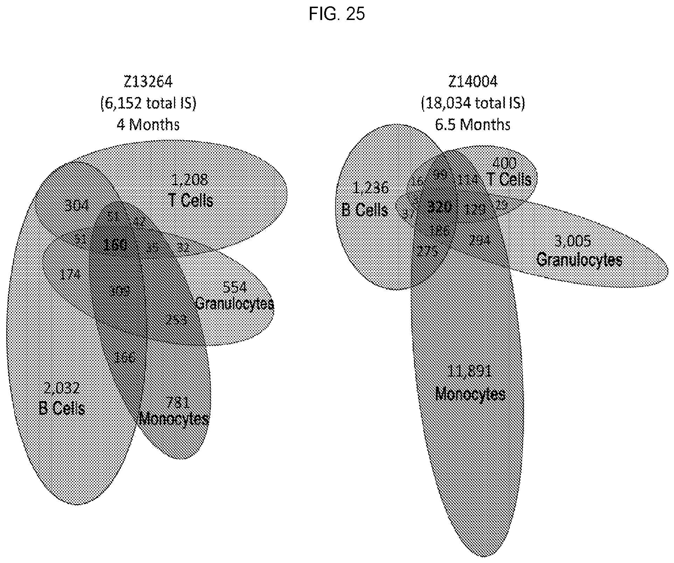

FIG. 25. An alternative method to longitudinally follow genetic modification is to perform clone tracking by insertion site analysis (ISA) confirms early, multi-lineage hematopoietic engraftment of CD34⁺CD45RA⁻CD90⁺ cells. Venn diagrams illustrate the number of shared clone signatures (IS) between fluorophore and cell surface marker sorted PB cell lineages in two animals at 4 months (Z13264) and 6.5 months (Z14004) after transplant. Fluorophore⁺ fraction i cells were mCherry⁺ (Z13264) or GFP⁺ (Z14004). Subsets include B cells (CD20⁺), T cells (CD3⁺), granulocytes (CD11b⁺CD14⁻) or monocytes (CD11b⁺CD14⁺).

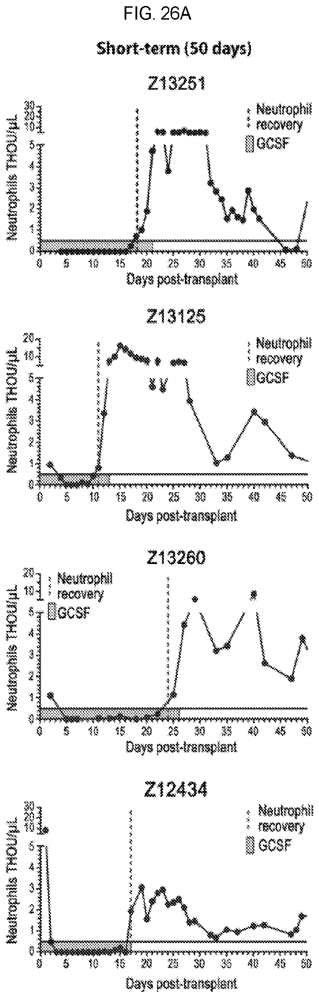

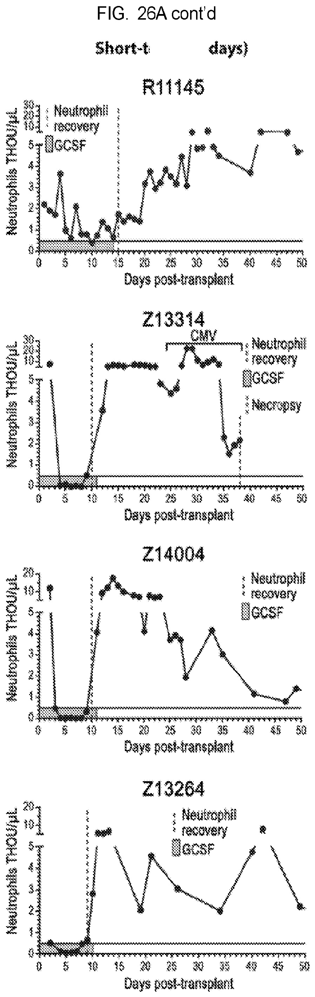

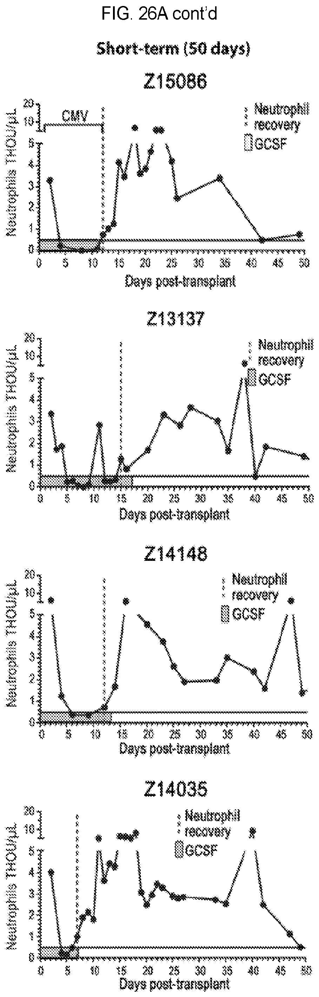

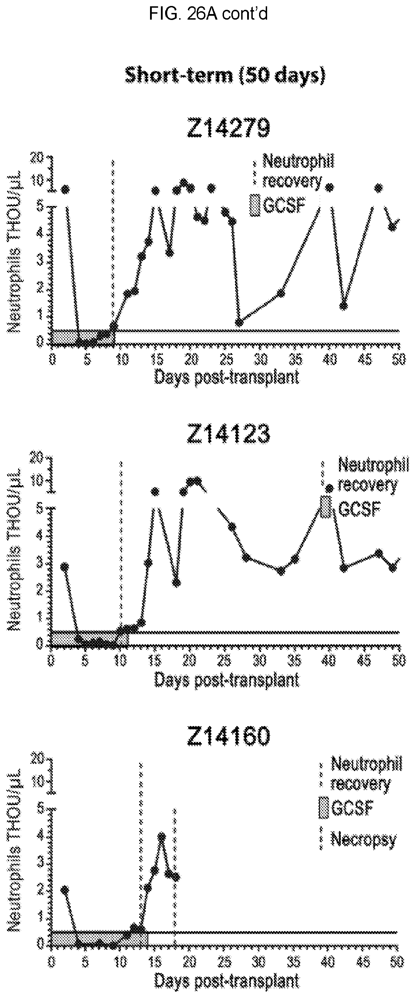

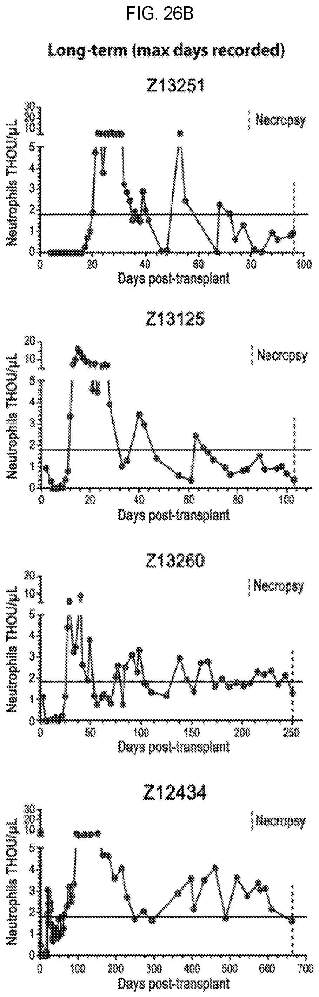

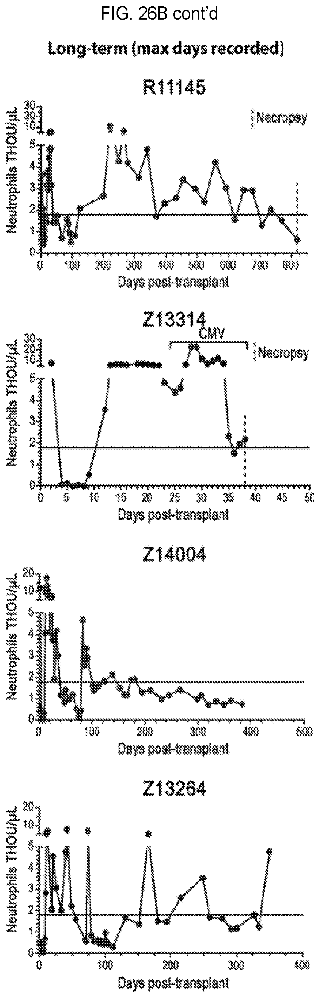

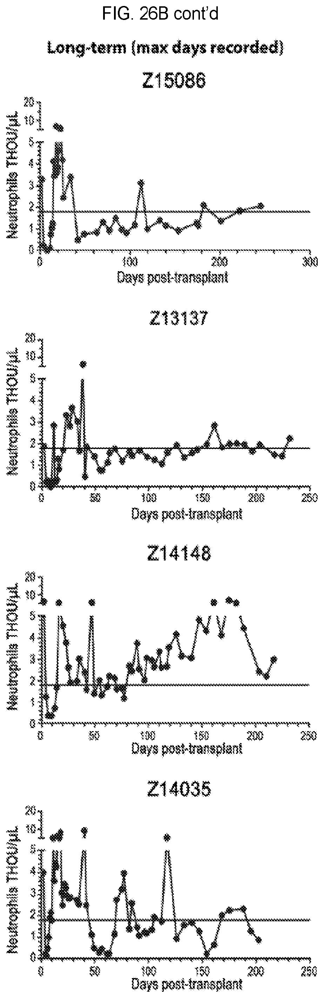

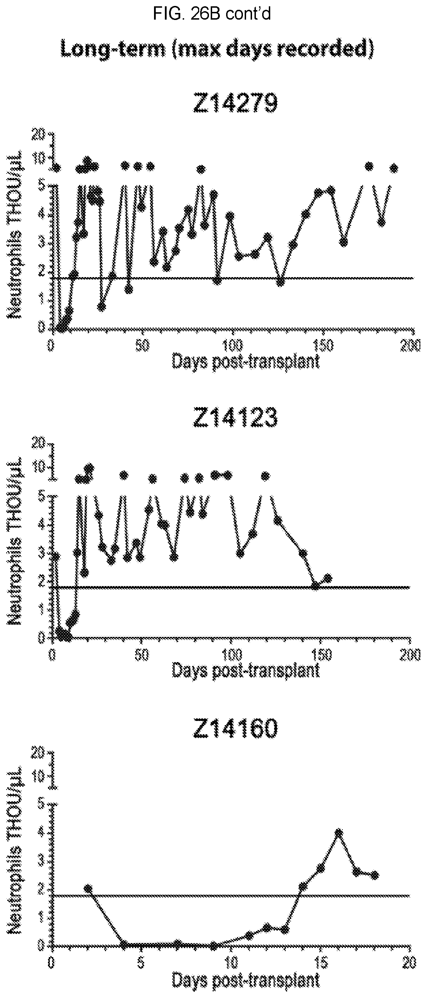

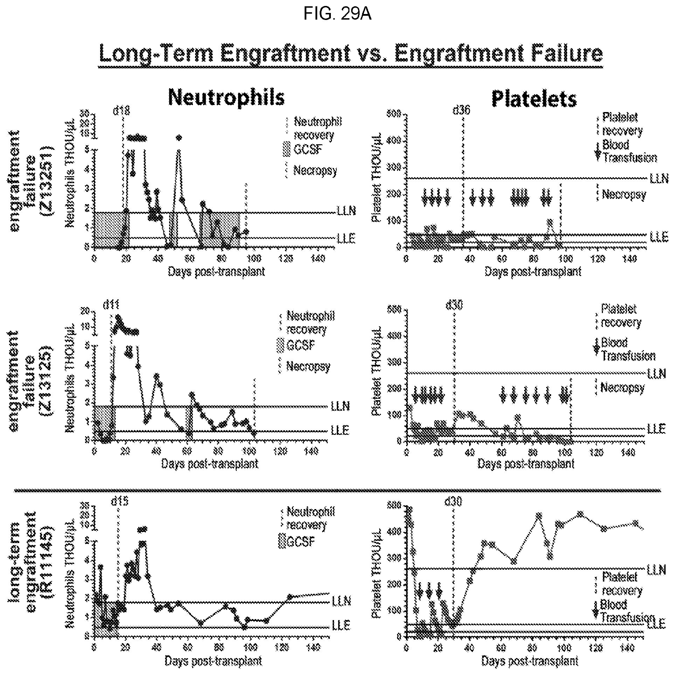

FIGs. 26A and 26B (Related to FIGs. 29A-29D). Short-term and long-term follow-up of neutrophil counts after autologous stem cell transplant in the nonhuman primate. The neutrophil counts of 15 individual nonhuman primates were tracked over time following myeloablative total body irradiation and lentiviral mediated genetic modification of autologous HSC transplant. (26A) The day of neutrophil engraftment (dashed vertical line) was plotted as defined FIG. 22A, 22B. (26B) Long-term follow-up (maximum number of days recorded) of neutrophil counts in the PB of transplanted nonhuman primates. The horizontal black bar indicates the average lower limit of neutrophil counts in healthy and historically, successfully engrafted primates (1,800/µl).
Due to cytomegalovirus (CMV) infection and early euthanasia, no long-term follow-up is available for animal Z13314. Due to engraftment failure requiring early euthanasia, no long-term follow-up is available for animals Z13251 and Z13125. Due to kidney failure, no long-term follow-up is available for animal Z14160. The day of necropsy is indicated by the dashed vertical line in FIG. 26B.

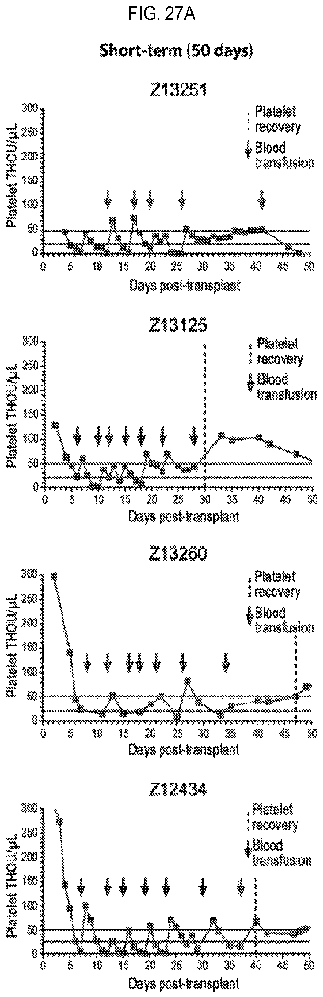

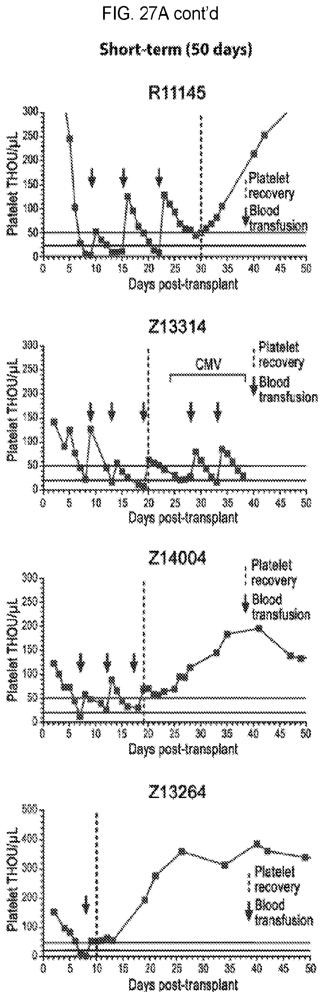

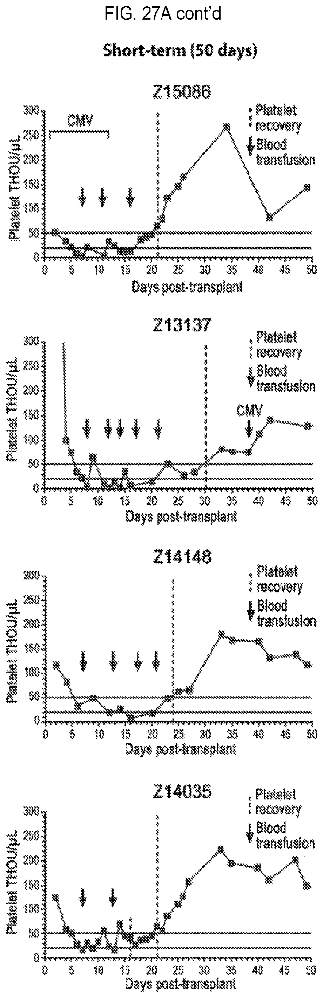

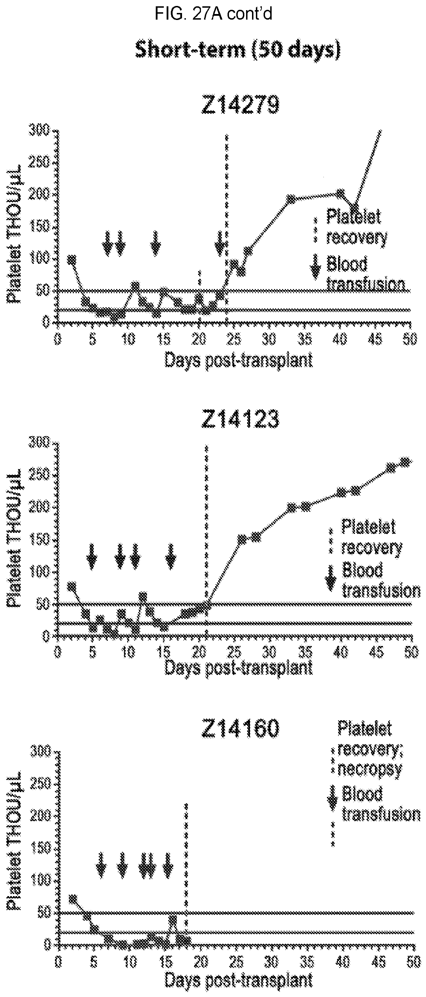

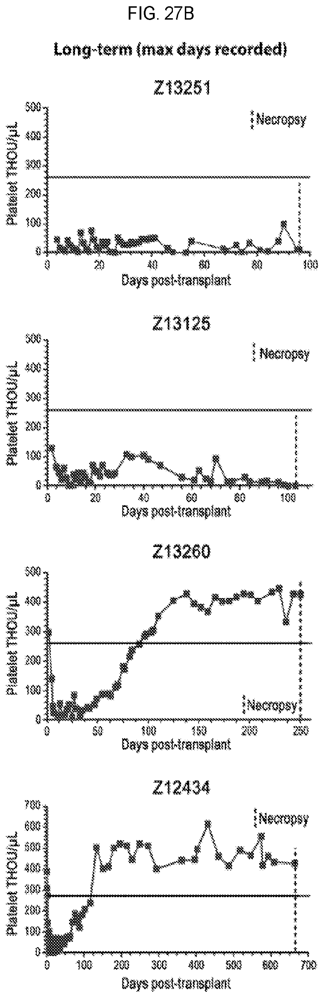

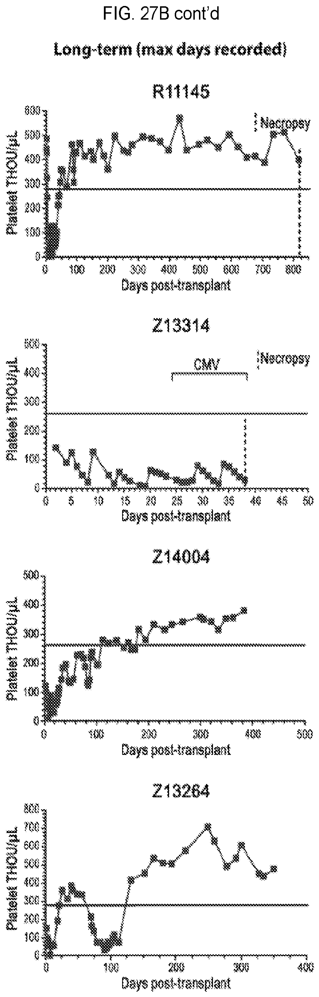

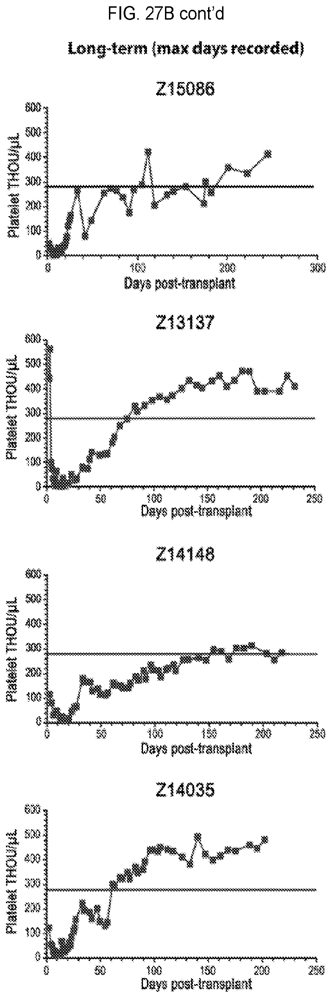

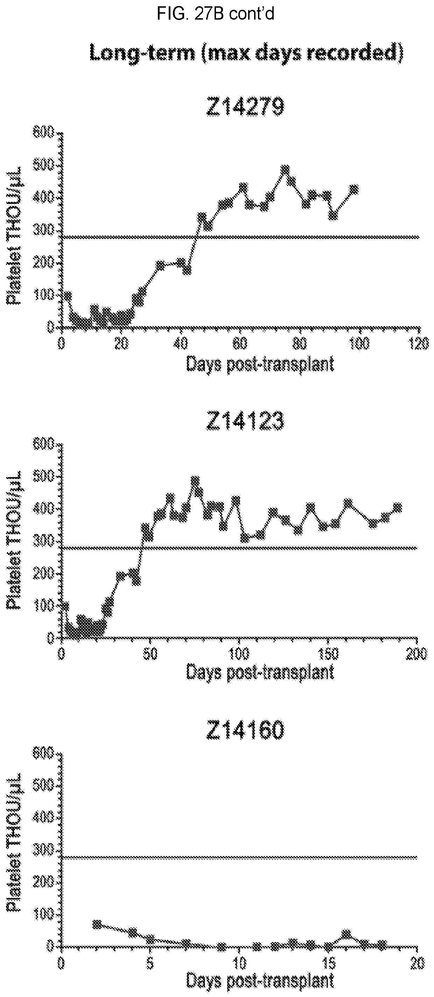

FIGs. 27A and 27B (Related to FIGs. 29A-29D). Short-term and long-term follow-up of platelet counts after autologous stem cell transplant in the nonhuman primate. The platelet counts of 15 individual nonhuman primates were tracked over time following myeloablative total body irradiation and lentivirus gene modified autologous HSC transplant. (27A) The day of platelet engraftment (dashed vertical line) was indicated when the criteria defined in FIG 22A, 22B were met. Blood transfusion is indicated by arrows. (27B) Long-term follow-up (maximum number of days recorded) of platelet counts in the PB of transplanted nonhuman primates. The horizontal black bar indicates the average lower limit of platelet counts in healthy and historically, successfully engrafted primates (260,000/µl).
Long-term follow-up was not available for the reasons stated as described in relation to FIGs. 26A, 26B. The day of necropsy is indicated by the dashed line in FIG. 27B.

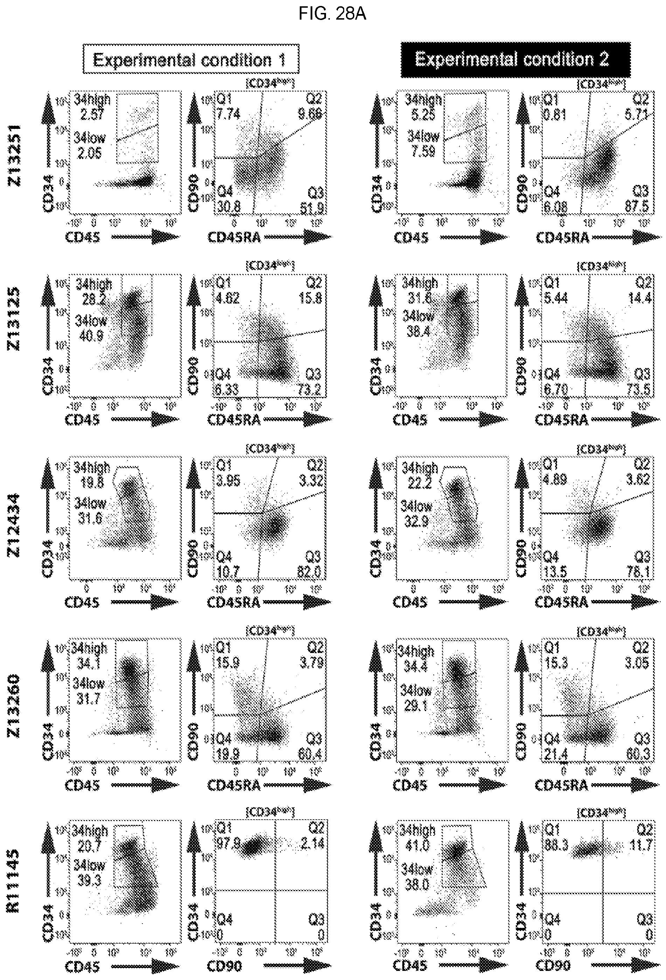

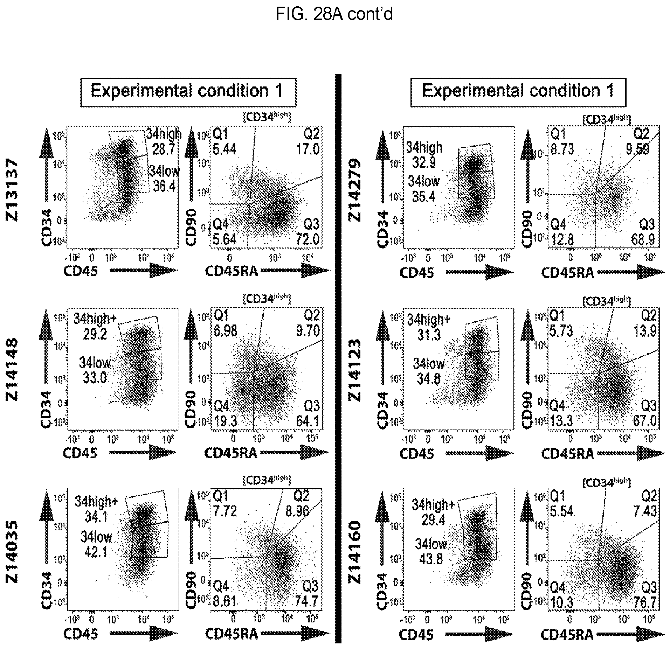

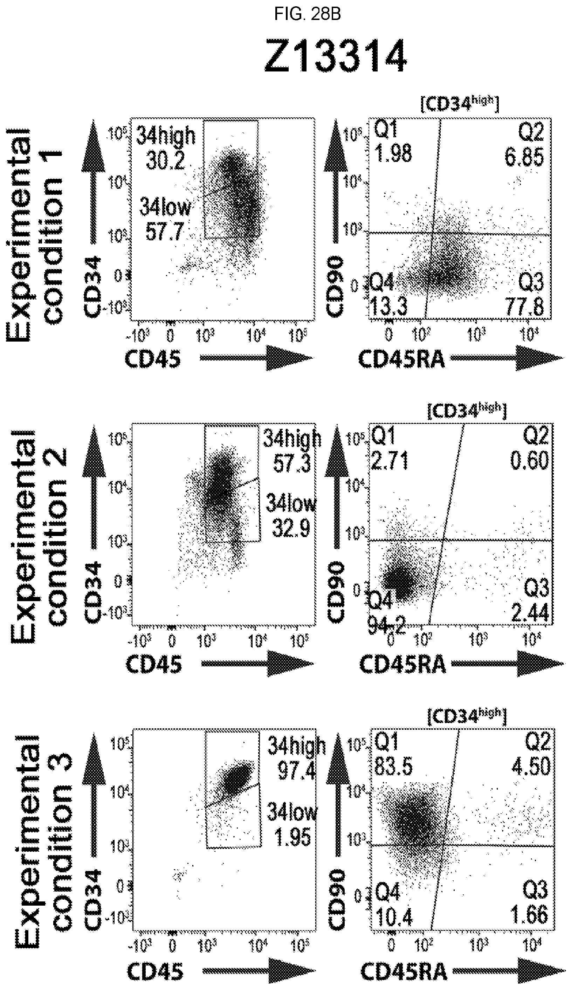

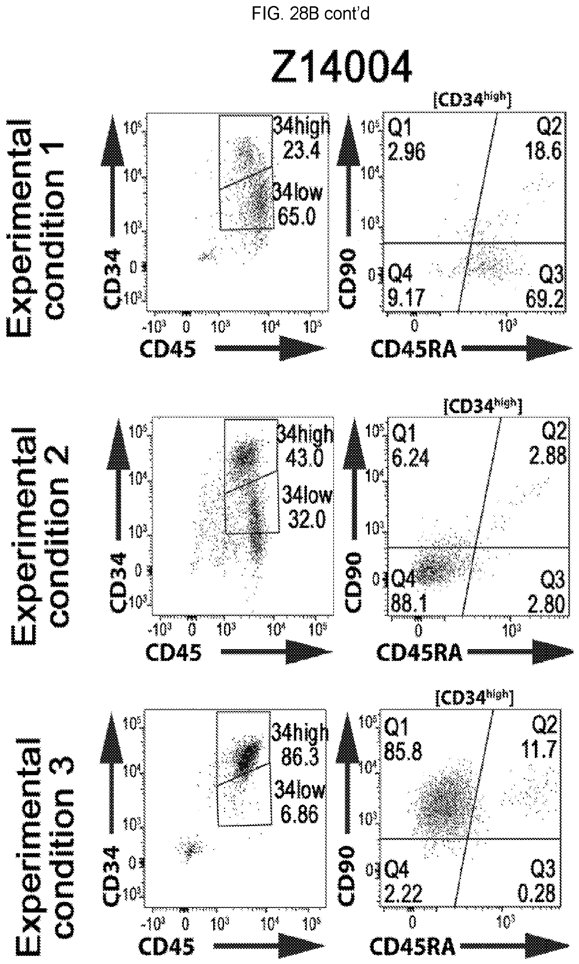

FIGs. 28A and 28B (Related to FIGs. 29A-29D). Flow cytometric quantification of HSPC subpopulations in autologous NHP stem cell transplants. (28A, 28B) Flow cytometric assessment of cell surface marker expression including: CD34, CD45, CD45RA and CD90. These markers were used to discriminate CD34⁺ cells (CD45^{int}CD34⁺), HSC-enriched fractions (hematopoietic stem cells: CD45^{int}CD34⁺CD45RA⁻CD90⁺), MPP-enriched fractions (MPP: CD45^{int}CD34⁺CD45RA⁻CD90⁻) and LMP-enriched fractions (lympho-myeloid progenitors: CD45^{int}CD34⁺CD45RA⁺CD90⁻). The number of phenotypically distinct HSPCs in each infused cell product was quantified individually for each transplant schema (Experimental condition 1-3, Table 1).

**Table 1.**

| **Monkey** | **Stem cell source** | **Condition 1** | **Condition 2** | **Condition 3** | **Days of culture** |
|---|---|---|---|---|---|
| Z13251 | GCSF/SCF Primed BM | CD45RA⁺ cells (GFP) | CD45RA⁻ cells (mCh) | - | 2 |
| Z13125 | GCSF/SCF Primed BM | VSVG envelop test (GFP) | Cocal envelop test (mCh) | - | 2 |
| Z13260 | Steady state BM | VSVG envelop test (GFP), 7 days of culture (SP-STF) | Cocal envelop test (mCh), 7 days of culture (SP-STF) | - | 9 |
| Z12434 | Steady state BM | Cocal envelop test (GFP), 7 days of culture (SP-STF) | VSVG envelop test (mCh), 7 days of culture (SP-STF) | - | 9 |
| R11145 | Steady state BM | 7 days of co-culture with HUVEC (GFP) | 7 days of culture (SP-STF) (mCh) | - | 9 |
| Z13314 | GCSF/SCF Primed BM | HSC (GFP) | MPP (mCh) | LMP (mCer) | 2 |
| Z14004 | GCSF/SCF Primed BM | HSC (GFP) | MPP (mCh) | LMP (mCer) | 2 |
| Z13264 | GCSF/SCF Primed BM | HSC (mCh) | MPP (mCer) | LMP (GFP) | 2 |
| Z15086 | GCSF/SCF Primed BM | HSC (mCer) | MPP (GFP) | LMP (mCh) | 2 |
| Z13137 | GCSF/SCF Primed BM | Lentivirus - Transduction | - | - | 2 |
| Z14148 | GCSF/SCF Primed BM | Lentivirus - Transduction | - | - | 2 |
| Z14035 | GCSF/SCF Primed BM | Lentivirus - Transduction | - | - | 2 |
| Z14279 | GCSF/SCF Primed BM | Lentivirus - Transduction | - | - | 2 |
| Z14123 | GCSF/SCF Primed BM | Lentivirus - Transduction | - | - | 2 |
| Z14160 | GCSF/SCF Primed BM | Lentivirus - Transduction | - | - | 2 |

The total number (sum of condition 1 + 2 + 3) as well as the cell count/kg body weight of CD34⁺ cells, HSC-, MPP- and LMP-enriched fractions for each NHP is summarized in Table 3 and 4.
Lack of anti-CD45RA staining did not allow for a discrimination and quantification of MPP- and LMP-enriched fractions for animal R11145.

FIGs. 29A-29D. Positive linear and logarithmic correlations between new phenotype HSCs / kg body weight administered and the day of neutrophil and platelet engraftment. (29A) Representative tracking of neutrophil and platelet counts following myeloablative total body irradiation and lentiviral genetically modified autologous HSC transplant from two individual nonhuman primates showing engraftment failure (upper row, animal Z13251, middle row Z13125) or successful long-term engraftment (lower row, animal R11145). Long dashed line: day of neutrophil/platelet engraftment; short dashed line for Z13251 and Z13125 (only upper two rows): day of necropsy; lower horizontal black bar - LLE (lower limit for engraftment): defined minimum number of neutrophils [500/µl] and platelets [20,000-50,000/µl] for the onset of recovery; upper horizontal black bar - LLN (lower limit of normal) gray bar: duration of GCSF administration; arrows: platelet transfusions. (29B) Statistical comparison of transplanted HSC-enriched fractions (CD34⁺CD45RA⁻CD90⁺), MPP-enriched fractions (CD34⁺CD45RA⁻CD90⁻) and LMP-enriched fractions (CD34⁺CD45RA⁺CD90⁻) per kg body weight in animals with engraftment failure (left) and long-term engraftment (right). (29C, 29D) Nonhuman primates demonstrating long-term engraftment (circles) were included for correlation analysis. Engraftment data of animals showing engraftment failure (boxes) were excluded for calculation of correlation. Correlation of CD34⁺CD45RA⁻CD90⁺fractions / kg body weight (from Table 4) with the day of (29C) neutrophil and (29D) platelet engraftment (dashed lines from FIGs. 26A and FIG. 27A, listed in Table 5) was calculated using Spearman's rank correlation coefficient (correlation coefficient R²: 0.0-0.19 = very weak, 0.20-0.39 = weak, 0.4-0.59 = moderate, 0.6-0.79 = strong, 0.8-1.0 = very strong). The linear regression and the 95% confidence interval for each correlation are indicated with the solid and the dotted line, respectively.
FIGs. 30A and 30B (Related to FIGs. 29A-29D). No linear correlation between classically defined HSPC counts / kg body weight and the day of neutrophil and platelet engraftment. Nonhuman primates demonstrating long-term engraftment (circles) were included in this analysis. Engraftment data of animals showing engraftment failure (boxes) were excluded for calculation of correlation. Correlation of CD34⁺ cells, MPP-enriched and LMP-enriched fractions / kg body weight (from Table 4) with the day of (30A) neutrophil and (30B) platelet engraftment (dashed lines from FIG. 26A and FIG. 27A listed in Table 2) was calculated using Spearman's rank correlation coefficient (correlation coefficient R²: 0.0-0.19 = very weak, 0.20-0.39 = weak, 0.4-0.59 = moderate, 0.6-0.79 = strong, 0.8-1.0 = very strong). The linear regression and the 95% confidence interval for each correlation are indicated with the solid and the dotted line, respectively.
FIGs. 31A and 31B (Related to FIGs. 29A-29D). No linear correlation between total CFC counts and CFCs / kg body weight and the day of neutrophil and platelet engraftment. Nonhuman primates demonstrating long-term engraftment (circles) were included in this analysis. Engraftment data of animals showing engraftment failure (boxes) were excluded for calculation of correlation. Correlation of total CFCs and CFCs / kg body weight (from Table 2) with the day of (31A) neutrophil and (31B) platelet engraftment (dashed lines from FIG. 26A and FIG. 27A listed in Table 2) was calculated using Spearman's rank correlation coefficient (correlation coefficient R²: 0.0-0.19 = very weak, 0.20-0.39 = weak, 0.4-0.59 = moderate, 0.6-0.79 = strong, 0.8-1.0 = very strong). The linear regression and the 95% confidence interval for each correlation are indicated with the solid and the dotted line, respectively.

**Table 2: Total CFCs and CFCs / kg body weight of transplanted CD34⁺ cells.**

| **Monkey** | **Total CFCs** | **CFCs/kg** |
|---|---|---|
| Z13251 | 236,690 | 72,604 |
| Z13125 | 1,287,678 | 297,385 |
| Z13260 | 495,228 | 115,707 |
| Z12434 | - | - |
| R11145 | 2,220,642 | 541,620 |
| Z13314 | 491,174 | 137,970 |
| Z14004 | 941,483 | 219,459 |
| Z13264 | 558,574 | 145,841 |
| Z15086 | 492,031 | 175,725 |
| Z13137 | 7,956,600 | 1,610,648 |
| Z14148 | 6,285,714 | 1,667,298 |
| Z14035 | 7,095,652 | 1,971,014 |
| Z14279 | 4,310,806 | 1,152,622 |
| Z14123 | 2,684,525 | 856,866 |
| Z14160 | 1,122,418 | 249,426 |

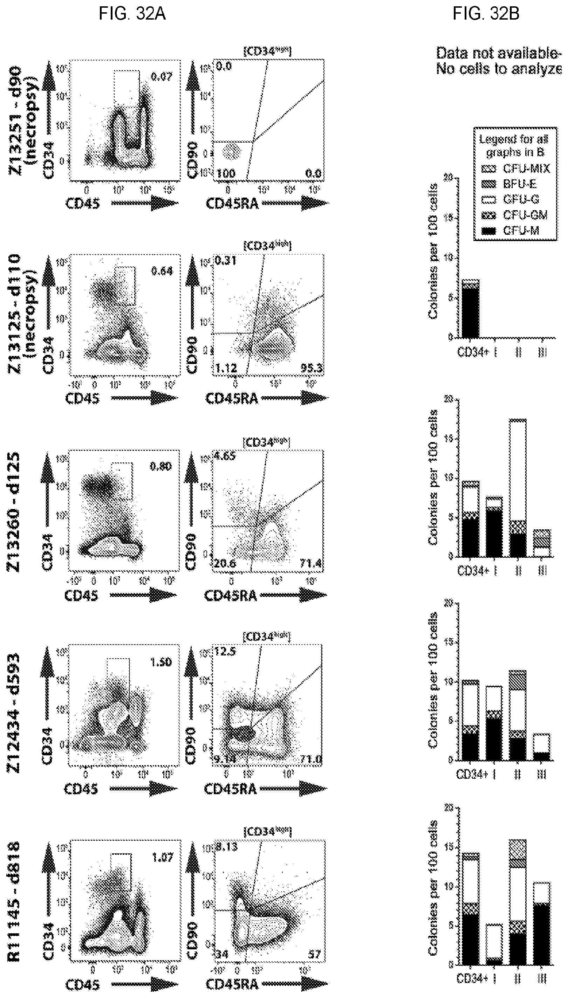

FIGs. 32A and 32B (Related to FIGs. 29A-29D). Composition of the bone marrow compartment in NHP with engraftment failure and successful long-term engraftment. (32A) Flow cytometric assessment and quantification of CD34⁺ cells, HSC-, MPP- and LMP-enriched fraction in the bone marrow of NHP. The day of collection and analysis post-transplant is indicated for each monkey individually (Y-axis). Bone marrow samples from Z13251 and Z13125 (engraftment failure) and Z13314 (CMV) were collected and analyzed on the day of necropsy. (32B) BM-derived CD34⁺ and HSPCs of fraction i + ii together, and separately fraction iii from all animals were sort-purified and introduced into CFC assays. After 14 days, the frequency of primary CFC was determined by counting arising colonies identified as CFU-G, CFU-M, CFU-GM, and BFU-E. Colonies including erythroid and myeloid cells were scored as CFU-MIX. The different colony-subtypes are coded as indicated in the figure legend. No HSPC fractions could be identified for animal Z13251.

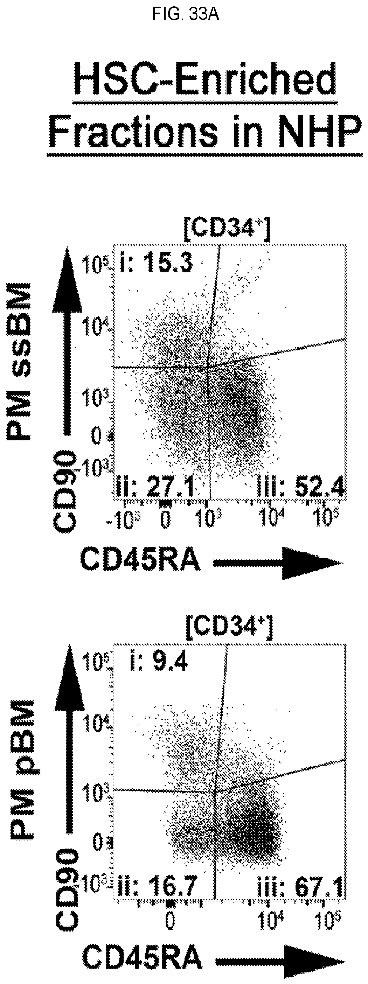

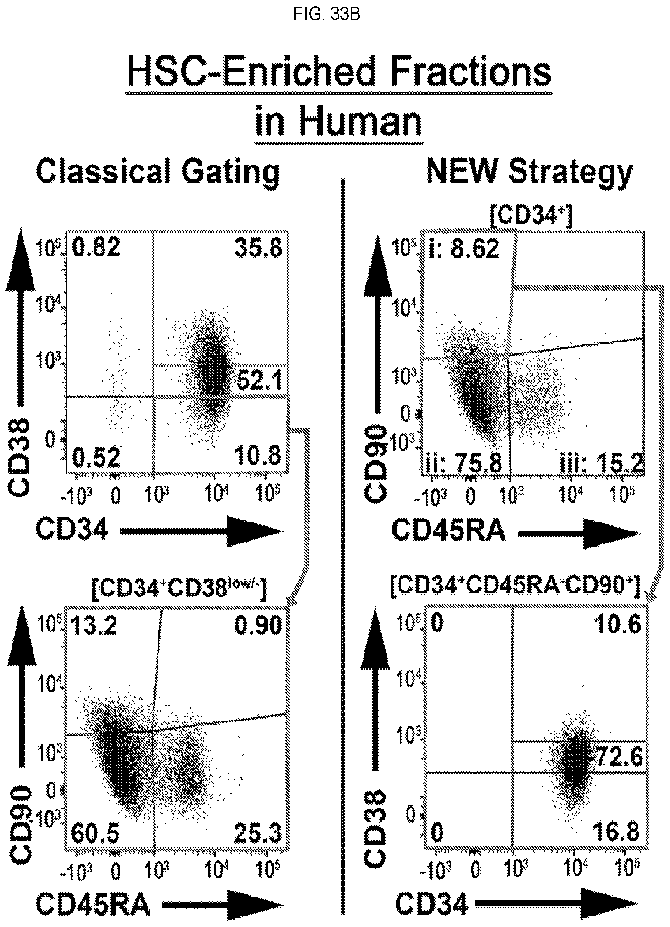

FIGs. 33A and 33B. Phenotype similarities observed between human and NHP HSC-enriched cell fractions. (33A) Gating of the HSC-enriched CD34⁺CD45RA⁻CD90⁺ cell fraction in NHP ssBM and pBM. (33B) Gating of an HSC-enriched cell fraction in human GCSF-mobilized peripheral blood stem cells (PBSCs) using the classical (left) and the disclosed (right) gating strategy for a CD34⁺CD45RA⁻CD90⁺ subfraction.

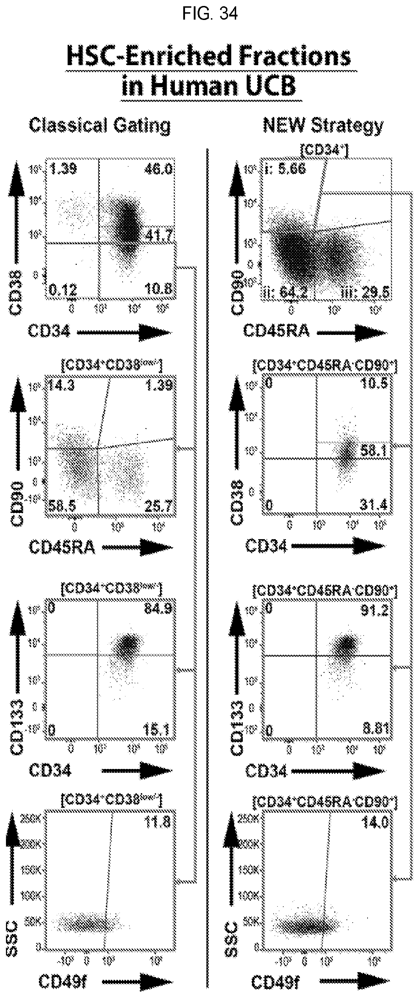

FIG. 34. (Related to FIGs. 33A, 33B) Gating of human HSC-enriched cell fractions in umbilical cord blood. Gating of an HSC-enriched cell fraction in human umbilical cord blood (UCB) stem cells using the classical (left) and the disclosed (right) gating strategy for a CD34⁺CD45RA⁻ CD90⁺ subfraction. Additional gating of CD34⁺CD38^{low/-} and CD34⁺CD45RA⁻CD90⁺ cell fraction for expression of CD133 and CD49f (bottom two plots).

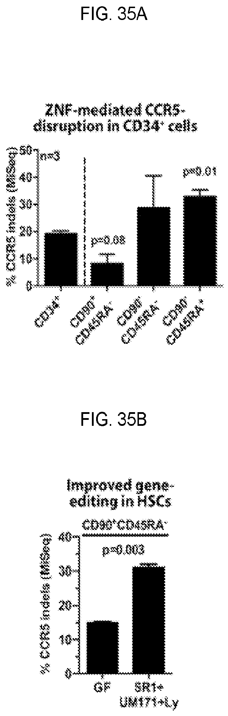

FIGs. 35A, 35B. CCR5-disruption in CD34⁺ subpopulations. CCR5-disruption efficiency in CD34⁺ subpopulation. ZFN (Zinc finger nuclease)-mediated CCR-5 disruption was performed in NHP CD34⁺ cells for both experiments. Analysis of CCR5-disruption was then carried out by MiSeq on sort-purified subpopulations using the cell surface marker CD45RA and CD90. (35A) Comparison of disruption efficiency in NHP bulk and sort-purified CD34⁺ subpopulations discriminating HSC- (CD45RA⁻CD90⁺), MPP- (CD45RA⁻CD90⁻) and LMPP (CD45RA⁺CD90⁻)-enriched fractions. (35B) Improved culture-conditions for CCR5 gene editing of our HSC-enriched CD34⁺CD45RA⁻CD90⁺ cell fraction using a small molecule mix containing SR1, UM171 and Ly2228820 compared to standard culture medium supplemented with growth factors (GF) only.

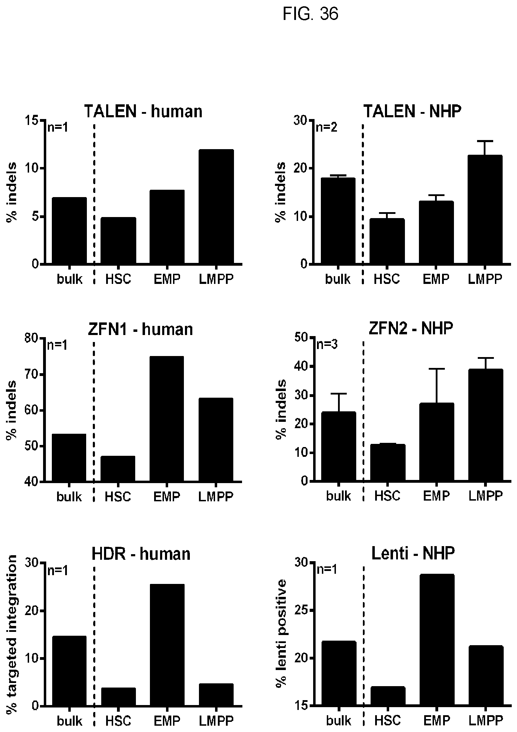

FIG. 36. Gene-editing/transfer efficiency in human and NHP CD34 subpopulation. Evaluation of gene-editing/transfer efficiency in human and NHP CD34-subpopulations using the cell surface marker CD45RA and CD90 to discriminate HSC- (CD34⁺CD45RA⁻CD90⁺), EMP-(CD34⁺CD45RA⁻CD90⁻) and LMPP- (CD34⁺CD45RA⁺CD90⁻) -enriched cell. Analysis of gene-disruption with TALEN and ZFNs was carried out by MiSeq on sort-purified subpopulations. Efficiency of HDR and lentiviral gene-transfer was evaluated by flow-cytometry and quantification of ectopic GFP expression.

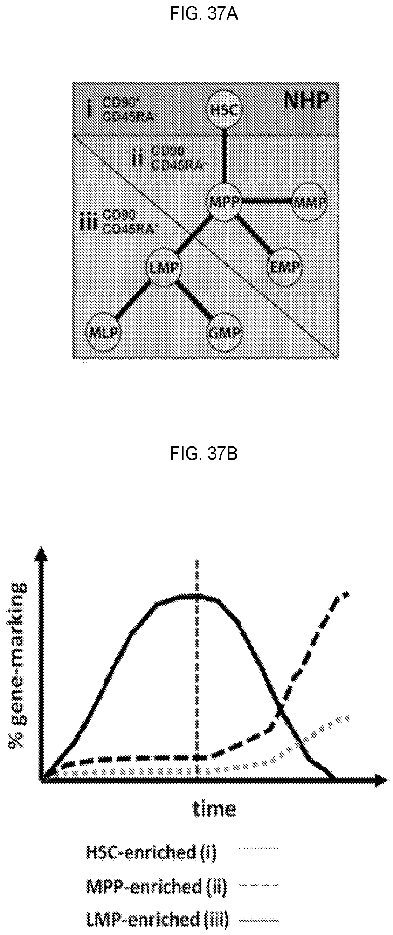

FIGs. 37A, 37B. Expected Contribution and Phases of Sorted Cell Populations in competitive reconstitution experiments. (37A) Schematic of the sorted CD34 subpopulation based on CD90 and CD45RA expression. (37B) Anticipated contribution of sort-purified CD34 subpopulation in the reconstitution of hematopoiesis similar to this schema, with an early phase of neutrophil recovery (short-term engraftment) expected to be mostly driven by the more committed, highly proliferative LMP (CD45RA⁺CD90⁻)-enriched fraction, followed by appearance of MPP (CD45RA⁻CD90⁻)-derived cells once LMPs are exhausted and a very late contribution of cells by HSCs (CD45RA⁻CD90⁺) once MPPs are exhausted.

### DETAILED DESCRIPTION

For more than three decades, hematopoietic stem cell (HSC) research has been performed based on the classical model of human hematopoiesis. This widely accepted model suggests an early segregation of lymphoid and erythro-myeloid potentials into a common lymphoid progenitor (CLP) and a common myeloid progenitor (CMP), respectively (Galy *et al.,* 1995; Manz *et al.,* 2002). However, this model has been challenged by proposals of a great variety of alternative lineage relationships and read-outs for multipotent HSCs, including but not limited to the cell surface markers of CD38 and CD49f (Adolfsson *et al.,* 2005; Doulatov *et al.,* 2010; Görgens *et al.,* 2013; Notta *et al.,* 2015; Masiuk *et al* 2017; Zonari *et al* 2017, US 2012/0252060).The hypothesis based on the current model of hematopoiesis (see FIG 37) would teach against the ability to identify a single enriched HSC-population that would result in both short and long term engraftment and reconstitution of all hematopoietic lineages.

Using the cell surface marker CD133, one revised model of human hematopoiesis proposes that HSCs and multipotent progenitors (HSCs/MPPs: CD34⁺CD45RA⁻CD133⁺) give rise to lympho-myeloid progenitors (LMPs: CD34⁺CD45RA⁺CD133⁺) and erythro-myeloid progenitors (EMPs: CD34⁺CD45RA⁻CD133^{low}) (Görgens *et al.,* 2014; Görgens *et al.,* 2013; Radtke *et al.,* 2015). This model suggests that multipotent HSCs/MPPs can easily be identified as CD34⁺CD45RA⁻CD133⁺ HSPCs with erythroid differentiation potential.

Clonal analysis and comparison of RNA expression profiles of human HSPCs from fetal liver, umbilical cord blood (UCB), and steady state bone marrow (ssBM) further revealed a shift in hematopoietic lineage relationships during development and aging (Notta *et al.,* 2015). Primarily focusing on the lineage relationships of primitive HSCs/MPPs and erythrocyte/megakaryocyte progenitors, Notta *et al.* have redefined the hematopoietic model, proposing an early separation of megakaryocyte potentials in adult stem cell sources (Notta *et* al., 2015).

While hematopoietic lineage relationships are still under investigation, consequences of these findings on the development of treatment strategies for hematological diseases and malignancies are rarely discussed. A critical factor for the development of allogeneic and autologous stem cell therapies is the availability of a read-out for multipotent HSCs/MPPs supporting the development of all blood cell lineages. Unfortunately, the current gold standard, the NOD/SCID mouse repopulation assay, does not support erythrocyte and megakaryocyte development and, thus, does not accurately read out multipotent human HSCs/MPPs (Mazurier *et al.,* 2003). In addition, development of therapeutic approaches with human HSPCs in the mouse model is not possible due to differences in marker expression, physiology, life span, and the demand on stem cell self-renewal and differentiation compared to humans (Larochelle *et al.,* 2011).

Development of clinical treatment strategies is currently performed in large animal models such as the dog and nonhuman primate (NHP). The pigtail macaque (PM; *Macaca nemestrina*) and the rhesus macaque (RM: *Macaca mulatta*) share a close evolutionary relationship with humans and have been used as a pre-clinical model system to study basic HSC biology (Shepherd *et al.,* 2007) or to develop specific HSC gene therapy approaches (Peterson *et al.,* 2016). Surprisingly, however, a comparison of hematopoietic subpopulations and the hierarchical organization of defined lineages has not been performed between NHPs and humans. This is a critical factor for a better understanding of the newly defined blood lineage associations and hierarchies, as well as the development of treatment approaches based on these lineages.

Stringent development and confirmation of a true HSC phenotype cell population can only be achieved after robust multilineage reconstitution of an irradiated recipient. Of the various models developed, retrovirus transduction and subsequent transplantation of CD34⁺ cells into human or animal recipients has permitted tracking of tens of thousands of individual cells *in vivo* over time and lineage development (Bystrykh, *et al.,* 2012).

The current disclosure shows that in the NHP, megakaryocyte and erythrocyte lineages branch off independently from a multipotent progenitor (MPP: CD34⁺CD45RA⁻CD90⁻CD117⁺, FIG. 1B). Upon differentiation, megakaryocyte-macrophage/monocyte progenitors (MMP: CD34⁺CD45RA⁻CD90⁻CD117⁺ CD123⁺) gained CD123 expression, whereas erythro-myeloid progenitors (EMP: CD34⁺CD45RA⁻CD90⁻CD117⁻) showed downregulation of CD117 and CD34 expression (FIG. 1B). Interestingly, expression of the IL-3 receptor (CD123) on human HSPCs has been reported for hematological malignancies (Liu *et al.,* 2015; Testa *et al.,* 2014) as well as for a great variety of primitive and more committed oligo-potent progenitors such as the CMP, GMP (granulocyte-macrophage progenitor) and monocyte/DC progenitors, but is low or entirely absent on erythrocyte and megakaryocyte progenitors (Manz *et al.,* 2002; Taussig *et al.,* 2005). Previous studies including CD123 expression are mostly based on lineage segregations proposed by the classical model of human hematopoiesis and do not include other cell surface markers such as CD90, CD117 and CD133, which allow for a separation of recently identified progenitor populations. These data provide evidence that erythrocyte and megakaryocyte potentials segregate into independent subpopulations.

Understanding hematopoietic lineage relationships is not only important for the basic understanding of stem cell biology but also critically important for rationally designing new therapeutic applications and strategies. Within the last decades, a great variety of cell surface markers have been reported for the identification and enrichment of human HSCs (Masiuk *et al.,* in press; Zonari *et al.,* 2017; Doulatov *et al.,* 2010; Görgens *et al.,* 2013; Kikushige *et al.,* 2008; Lansdorp *et al.,* 1990; Majeti *et al.,* 2007; Notta *et al.,* 2011; Terstappen *et al.,* 1991). The conclusion from these studies has been that human HSCs are found to be enriched in CD34⁺CD38^{low}CD45RA⁻CD49f⁺CD90⁺CD117⁺CD133⁺ fractions (FIG. 1A). The current disclosure describes development and processing of a population in NHPs based on available cross-species reactive antibodies and novel gating strategies and shows that primitive NHP HSPCs with multi-lineage potential are enriched in a CD34⁺CD45RA⁻CD90⁺CD117⁺ fractions containing lymphoid, myeloid, erythroid as well as megakaryocytic differentiation potential (FIG. 1B).
The methods of the invention do not utilize CD38 or CD49f and do not utilize markers other than CD34, CD45RA, CD90, CD117, CD123, and/or CD133 to isolate the stem cell population as claimed. Thus, in particular embodiments, the current disclosure excludes selection based on any cell surface marker except for CD34, CD45RA, and CD90. In particular embodiments, the current disclosure excludes selection based on CD38 and CD49f, and includes selection based on CD34, CD45RA, CD90, CD133 (human) or CD117 (NHP). In particular embodiments, the profile excludes consideration of markers other than CD34 (human and NHP), CD45RA (human and NHP), CD90 (human and NHP), and optionally CD133 (human), CD117 (NHP) and/or CD123 (NHP).

Described herein is development and processing a CD45RA⁻CD90⁺ subset of enriched CD34⁺ cells for rapid multilineage hematopoiesis in a clinically relevant NHP autologous HSC transplantation model. Evidence for early-engrafting HSC-like clones which are stably maintained long-term are demonstrated. The disclosure is supported by tracking hundreds of thousands of unique clone signatures in animals over years of follow-up. Most importantly, we demonstrate the phenotype and transcriptomes of these cells are highly similar between NHP and humans. Biologically robust competitive repopulation experiments in four animals demonstrate the capacity of CD34⁺CD45RA⁻CD90⁺ cells to completely reconstitute hematopoiesis in the transplant setting. The current disclosure also provides that many fewer cells are needed for successful hematopoietic reconstitution in this setting (122,000 cells / kg) as compared to the 5-10 million cells / kg typically required for CD34⁺ cells, the current clinical gold standard for HSC enrichment. The number of nonhuman primates described in this disclosure, validation of *in vitro* biology *in vivo* both retrospectively and prospectively, and the tracking of individual clones to confirm the disclosure supports early HSC engraftment and clonal stability after transplantation. Most importantly for the fields of HSC biology/transplantation, gene therapy and gene editing, this disclosure develops and processes a highly refined and markedly reduced cell population capable of completely functional hematopoietic reconstitution.

The current disclosure provides that the disclosed highly HSC-enriched CD34⁺CD45RA⁻ CD90⁺ cell population is accountable for all gene-modified cells observed in PB and BM following transplant. This shows that everything required for short- and long-term, multilineage hematopoietic reconstitution is contained within this engineered cell population. The limitations of methods in the prior art is that the enriched-HSC populations were not sufficient to re-populate all of the hematopoietic lineages, meaning, practitioners would need to add additional cells during their preparations. See, e.g., Kohn *et al.,* 2010. By having a clearly defined cell population as disclosed in this patent application, the manufacturing of cell and gene-modified therapeutics could be streamlined leading to faster processing of stem cell sources and reduced reagent needs for cytokines, consumables and modifying agents (such as vector, nucleases or other reagents). These are critical requirements if stem cell and gene therapy will translate outside of specialized academic medical centers and be utilized in a broader setting.

The disclosed cell populations have a major impact in the development of novel HSC-based therapies. The NHP model offers a read out of hematopoiesis which is not possible in small animal models such as the mouse, process and enrich multipotent HSC, and determine their multi-lineage engraftment potential in a clinically relevant transplant model. Furthermore, it allows scale-up of cell isolation and manipulation protocols, testing of safety and off-target effects, evaluation of the development of all hematopoietic lineages, as well as long-term engraftment follow-up in a pre-clinical transplant setting with high homology to humans.

*In vivo* observations in competitively transplanted animals show that the selection of the CD34⁺CD45RA⁻CD90⁺ cell population is a significant refinement in the target cell population for transplantation, as well as in approaches such as combining HSC-enrichment with genetic modification for the purposes of gene therapy. Currently, the gold-standard for clinical gene therapy is lentiviral (LV) transduction of enriched CD34⁺ cells. Focusing on creation of CD34⁺CD45RA⁻CD90⁺ cell populations results in a 20-fold reduction in overall cell numbers and thus dramatically reduces materials required for vector-based or nuclease-mediated gene therapy and editing protocols (FIG 35 and 36). It is well known in the art, that genetic modification of stem cells is more difficult than more differentiated cell types. To ensure the
HSC-enriched CD34⁺CD45RA⁻CD90⁺ population was amenable to genetic manipulation we conducted experiments to provide a variety of genetic modifications, zinc-finger nuclease (ZFN) disruption (FIG 35), TALENs, and homology directed repair (HDR) (FIG 36). When comparing human and NHP CD34⁺CD45RA⁻CD90⁺ HSPCs, significant overlap in gene expression of key regulators was noted. The cross-species conservation of the HSC-enriched CD34⁺CD45RA⁻ CD90⁺ phenotype indicates significant implications for human transplantation and gene therapy studies.

A strong correlation between CD34⁺CD45RA⁻CD90⁺ HSCs/kg body weight and the success of multi-lineage engraftment was achieved, as well as the time to neutrophil and platelet recovery, further supporting the clinical translation of the described, phenotypically defined, HSC-enriched cell populations. This is the first disclosure of a statistically significant and direct correlation of a phenotypically and/or functionally defined HSPC subpopulation with short- and long-term engraftment markers in the transplantation setting. The correlation between CD34⁺CD45RA⁻CD90⁺ cell dose and engraftment was not affected by different stem cell sources, gene modifications, expansion protocols, or cryopreservation, making this phenotype a valuable tool for the *in vitro* analysis of various novel HSC modifications, expansion strategies and quality control of stem cell infusion products.

In summary, the development of phenotypically and transcriptionally unique cell populations between human and NHP HSPCs and blood lineages is a major advance for the development of novel HSPC therapies. While each of the markers in the profiles described herein is individually known, the processing methods disclosed herein provide a novel strategy that defines HSC capable of providing for both short- and long-term engraftment. Furthermore, the development and processing of the disclosed cell populations results in predictable correlation with the time to hematopoietic recovery of neutrophils and platelets in the myeloablative transplant setting, providing a measure of potency. The ability to predict engraftment failure or success is a critical unmet need in the transplant setting where even with large doses of CD34⁺ cells as evidenced by our experiments (FIG. 29B and FIGs. 30A and 30B) result in engraftment failure by clinically defined parameters of neutrophils, platelets, and CFCs.

The detection of persistent and HSC-like clonal signatures early after transplant, before all lineages have reconstituted, shows our CD34⁺CD45RA⁻CD90⁺ cell population immediately contributes to long-term, multi-lineage hematopoietic reconstitution lasting up to 7+ years in the disclosed clinically relevant model.

These advances also allow for the development and testing of gene modification approaches to correct HSC in non-malignant diseases with monogenetic mutations, such as Fanconi anemia, hemoglobinopathies, as well as pathogen-mediated diseases, such as HIV/AIDS through the disruption of CCR5. Considerations relevant to these aspects of the current disclosure are now described in more detail.

In use, the engineered cell population may be administered with or without genetic modification in transplantation. Transplantation is a medical procedure in which a blood or bone marrow donor stem cell source is administered to patients. In order to allow for successful transplantation, conditioning reagents are administered to make room for the new HSC graft. A transplant may replace abnormal blood-forming stem cells with healthy cells in both non-malignant and malignant settings. An essential component to HSC transplantation with or without genetic modification is conditioning regimens administered prior to the infusion of the HSC-enriched cell population. Gyurkocza & Sandmaier, 2014. For malignant conditions, such as cancers or tumors, myeloablative conditioning regimens with total body irradiation (TBI) and chemotherapeutic agents with nonoverlapping toxicities are used to provide sufficient immunoablation to prevent HSC graft rejection and reduce tumor.
TBI may be conducted with 4 doses of 255cGy. In this setting, graft versus host diseases (GVHD) and graft versus tumor (GVT) or graft versus leukemia (GVL) effects contribute to the effectiveness of the HCT transplant. In patient populations that will not benefit from GVHD or GVL/GVT there is added benefit of removing CD45RA+ cells from the infusion product. Removal of CD45RA+ cells from the graft due resulting in T-cell depleted grafts had lower relapse rates in the transplant setting. Bleakley *et al.,* 2015. However, there are significant patient populations, such as older and medically infirm patients, or patients in which the GVT or GVL response is not desired, such as in non-malignant conditions such as monogenetic diseases and cure regiments for pathogen-mediated illnesses such as HIV/AIDS in which non-genotoxic conditioning regimens are being utilized and developed. The field has moved towards reduced intensity and non-genotoxic conditioning regimens for patients, in particular for younger patients in which myeloablative conditioning would cause irreversible developmental harm (Palchaudhuri *et al.,* 2016). Non-genotoxic conditioning regimens may include biologic agents such as anti-c-Kit monoclonal antibodies that allow for depletion of HSCs in bone marrow niches in the immunodeficient setting, or in combination with blockade of CD47 using engineered fragments of human SIRPalpha or antibodies that block CD47 activity (Chhabra *et al.,* 2016). Non-genotoxic conditioning may be accomplished utilizing immunotoxin targeting the hematopoietic-cell-restricted CD45 receptor, in particular CD45-saporin (SAP) (Palchaudhuri *et al.,* 2016)

Stem cell sources include umbilical cord blood, placental blood, bone marrow and peripheral blood (see U.S. Patent Nos. 5,004,681; 7,399,633; and 7,147,626; Craddock *et al.,* 1997; Jin *et al.,* 2008; Pelus, 2008; Papayannopoulou *et al.,* 1998; Tricot *et al.,* 2008; and Weaver *et al.,* 2001), as well as fetal liver, and embryonic stem cells (ESC) and induced pluripotent stem cells (iPSCs) that can be differentiated into HSCs. Methods regarding collection, anti-coagulation and processing, etc. of blood and tissue samples are well known in the art. See, for example, Alsever *et al.,* 1941; De Gowin, *et al.,* 1940; Smith, *et al.,* 1959; Rous and Turner, 1916; and Hum, 1968. Stem cell sources of HSC also include aortal-gonadal-mesonephros derived cells, lymph, liver, thymus, and spleen from age-appropriate donors. All collected stem cell sources of HSC can be screened for undesirable components and discarded, treated, or used according to accepted current standards at the time. These stem cell sources can be steady state/naïve or primed with mobilizing or growth factor agents. Stem cell sources can also include hematopoietic stem cell and progenitor cells (HSPCs) that can be further processed to refine HSCs from other progenitor cells.

In order to avoid surgical procedures to perform a bone marrow harvest to isolate HSCs, approaches are emerging to harvest stem cells from the peripheral blood. Mobilization is a process whereby stem cells are stimulated out of the bone marrow (BM) niche into the peripheral blood (PB), and likely proliferate in the PB. Mobilization allows for a larger frequency of stem cells within the PB minimizing the number of days of apheresis, reaching target number collection of stem cells, and minimizing discomfort to the donor source. Agents that enhance mobilization can either enhance proliferation in the PB, or enhance migration from the BM to PB, or both. Mobilizing agents include cytotoxic drugs, cytokines, and/or small molecules. A historically used regimen is a combination of cyclophosphamide (Cy) plus granulocyte-colony stimulating factor (G-CSF). Bonig *et al.,* 2009. Additional mobilizing agents include alpha4-integrin blockade with anti-functional antibodies and CXCR4 blockade with the small-molecule inhibitor AMD3100. AMD3100 is a bicyclam molecule that specifically and reversibly blocks SDF-1 binding to CXCR4. Another embodiment is the combined regiment of GM-CSF or GCSF with AMD3100. The mobilizing agent may be C4, a CXC chemokine ligand for the CXCR2 receptor. GRObeta rapidly mobilizes short- and long-term repopulating cells in mice and/or monkeys and synergistically enhances mobilization responses with G-CSF (Pelus *et al.,* 2006). Furthermore, GRObeta can be combined with antagonists of VLA4 to synergistically increase circulating HSPC numbers (Karpova *et al.,* 2016). The CXC4 inhibitor,
plerixafor, may be used as a single agent for mobilization of HSPCs. Plerixafor is also known commercially under the trade names Mozobil, REvixil, UMK121, AMD 3000, AMD 3100, AMD3000, AMD3100, GZ 316455, GZ316455, JM 3100, JM3100, SDZ SID 791, SDZSID791.

HSC can be collected and isolated from a sample using any appropriate technique. Appropriate collection and isolation procedures include magnetic separation; fluorescence activated cell sorting (FACS; Williams *et al.,* 1985; Lu *et al.,* 1986); nanosorting based on fluorophore expression; affinity chromatography; cytotoxic agents joined to a monoclonal antibody or used in conjunction with a monoclonal antibody, e.g., complement and cytotoxins; "panning" with antibody attached to a solid matrix (Broxmeyer *et al.,* 1984); selective agglutination using a lectin such as soybean (Reisner *et al.,* 1980); immunomagnetic bead-based sorting or combinations of these techniques, etc.

In particular embodiments, it is important to remove contaminating cell populations that would interfere with isolation of the CD34⁺/CD45RA⁻/CD90⁺cell population, in particular red blood cells. Removing includes both biochemical and mechanical methods to remove the undesired cell populations. Examples include lysis of red blood cells using detergents, hetastarch, hetastarch with centrifugation, cell washing, cell washing with density gradient, Ficoll-hypaque, Sepx, Optipress, Filters, and other protocols that have been used both in the manufacture of HSC cell and/or gene therapies for research and therapeutic purposes.

In particular embodiments, a HSC sample (for example, a fresh cord blood unit) can be processed to select/enrich for CD34⁺/CD45RA⁻/CD90⁺ (both human and NHP), CD34⁺/CD45RA⁻ /CD90⁺/CD133⁺ (human), or CD34⁺/CD45RA⁻/CD90⁺/CD117⁺ cells (both human and NHP) using appropriate antibodies directly or indirectly conjugated to magnetic particles in connection with a magnetic cell separator, for example, the CliniMACS^{®} Cell Separation System (Miltenyi Biotec, Bergisch Gladbach, Germany). U.S. Pat. No. 5,877,299 describes exemplary hematopoietic antigens that can be used to isolate, collect, and enrich HSC cells from samples.

In particular embodiments, cell populations can be isolated and/or analyzed based on light scattering properties of the cells based on side scatter channel (SSC) brightness and forward scatter channel (FSC) brightness. Side scatter refers to the amount of light scattered orthogonally (90° from the direction of the laser source), as measured by flow cytometry. Forward scatter refers to the amount of light scattered generally less than 90° from the direction of the light source. Generally, as cell granularity increases, the side scatter increases and as cell diameter increases, the forward scatter increases.

Side scatter and forward scatter are measured as intensity of light. Those skilled in the art recognize that the amount of side scatter can be differentiated with user-defined settings. In particular embodiments, low (lo) side scatter refers to less than 50% intensity, less than 40% intensity, less than 30% intensity, or even less intensity, in the side scatter channel of the flow cytometer. Conversely high (hi) side scatter cells are the reciprocal population of cells that are not low side scatter. Forward scatter is defined in the same manner as side scatter but the light is collected in forward scatter channel. Thus, particular embodiments include selection of cell populations based on precise combinations of cell surface markers (CD markers) and the associated light scattering properties of the cells.

The cell populations disclosed herein rely on positive expression of particular CD markers and the negative expression of other CD markers. As is understood by one of ordinary skill in the art of flow cytometry, "hi", "int", "lo", "+" and "-" refer to the intensity of a signal relative to negative or other populations. See, for example, FIGs. 3 and 4. In particular embodiments, positive expression (+) means that the marker is detectable on a cell using flow cytometry. In particular embodiments, negative expression (-) means that the marker is not detectable using flow cytometry. In particular embodiments, "hi" means that the positive expression of a marker of interest is brighter as measured by fluorescence (using for example FACS) than other cells also positive for expression. In these embodiments, those of ordinary skill in the art recognize that brightness is based on a threshold of detection. Generally, one of skill in the art will analyze a negative control tube first, and set a gate (bitmap) around the population of interest by FSC and SSC and adjust the photomultiplier tube voltages and gains for fluorescence in the desired emission wavelengths, such that 97% of the cells appear unstained for the fluorescence marker with the negative control. Once these parameters are established, stained cells are analyzed and fluorescence recorded as relative to the unstained fluorescent cell population. In particular embodiments, and representative of a typical FACS plot, hi implies to the farthest right (x line) or highest top line (upper right or left) while lo implies within the left lower quadrant or in the middle between the right and left quadrant (but shifted relative to the negative population). In particular embodiments, "hi" refers to greater than 20-fold of +, greater than 30-fold of +, greater than 40-fold of +, greater than 50-fold of +, greater than 60-fold of +, greater than 70-fold of +, greater than 80-fold of +, greater than 90-fold of +, greater than 100-fold of +, or more of an increase in detectable fluorescence relative to + cells. Conversely, "lo" can refer to a reciprocal population of those defined as "hi".

Novel gating strategies disclosed herein are shown in, for example, FIG. 33B.

Once HSCs have been collected and isolated, HSC expansion can be performed. Expansion can occur in the presence of one or more growth factors, such as: angiopoietin-like proteins (Angptls, e.g., Angptl2, Angptl3, Angptl7, Angpt15, and Mfap4); erythropoietin; fibroblast growth factor-1 (FGF-1); FLT3-ligand (FLT3-L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF; also known as the c-kit ligand or mast cell growth factor); thrombopoietin (TPO); and analogs thereof (wherein the analogs include any structural variants of the growth factors having the biological activity of the naturally occurring growth factor; see, e.g., WO 2007/1145227 and U.S. Patent Publication No. 2010/0183564). For clarity, growth factor agents can also be used as mobilizing agents. Particular embodiments utilize expansion in stem cell supportive media (e.g. StemSpan) supplemented with either SCF, TPO, and FLT3-L or SCF and IL-3, or other combinations of growth factors.

In particular embodiments, the type, amount and/or concentration of growth factors suitable for expanding HSC is the amount or concentration effective to promote proliferation. HSC populations are preferably expanded until a sufficient number of cells are obtained to provide for at least one infusion into a human subject, typically around 10⁴ cells/kg to 10⁹ cells/kg.

The amount or concentration of growth factors suitable for expanding HSC depends on the activity of the growth factor preparation, and the species correspondence between the growth factors and HSC, etc. Generally, when the growth factor(s) and HSC are of the same species, the total amount of growth factor in the culture medium ranges from 1 ng/ml to 5 µg/ml, from 5 ng/ml to 1 µg/ml, or from 5 ng/ml to 250 ng/ml. In additional embodiments, the amount of growth factors can be in the range of 5-1000 or 50-100 ng/ml.

In particular embodiments, growth factors are present in an expansion culture condition at the following concentrations: 25-300 ng/ml SCF, 25-300 ng/ml FLT3-L, 25-100 ng/ml TPO, 25-100 ng/ml IL-6 and 10 ng/ml IL-3. In more specific embodiments, 50, 100, or 200 ng/ml SCF; 50, 100, or 200 ng/ml of FLT3-L; 50 or 100 ng/ml TPO; 50 or 100 ng/ml IL-6; and 10 ng/ml IL-3 can be used.

HSC can be expanded in a tissue culture dish onto which an extracellular matrix protein such as fibronectin (FN), or a fragment thereof (e.g., CH-296 (Dao et. al., 1998, Blood 92(12):4612-21)) or RetroNectin^{®} (a recombinant human fibronectin fragment; (Clontech Laboratories, Inc., Madison, WI) is bound.

Notch agonists can be particularly useful for expanding HSC. In particular embodiments, HSC can be expanded by exposing the HSC to an immobilized Notch agonist, and 50 ng/ml or 100 ng/ml SCF; to an immobilized Notch agonist, and 50 ng/ml or 100 ng/ml of each of FLT3-L, IL-6, TPO, and SCF; or an immobilized Notch agonist, and 50 ng/ml or 100 ng/ml of each of FLT3-L, IL-6, TPO, and SCF, and 10 ng/ml of IL-11 or IL-3.

Additional methods to expand and/or maintain HSC in culture can utilize one or more of a commercially available base media such as StemSpan SFEM or ACF media (both from Stem Cell Technologies) or XVivo media types (Lonza) supplemented with one or more of: Cyto/chemokines (e.g., G-CSF, SCF, TPO, FLT3-L, IL-3, IL-6); small molecules such as aryl-hydrocarbon receptor antagonists (e.g., StemRegenin1 (e.g., Phenol, 4-[2-[[2-benzo[b]thien-3-yl-9-(1-methylethyl)-9H-purin-6-yl]amino]ethyl]); GNF351 (e.g., N-(2-(3H-Indol-3-yl)ethyl)-9-isopropyl-2-(5-methyl-3-pyridyl)-7H-purin-6-amine,N-(2-(1H-Indol-3-yl)ethyl)-9-isopropyl- 2-(5-methylpyridin-3-yl)-9H-purin-6-amine); CH223191 (e.g., 1-Methyl-N-[2-methyl-4-[2-(2-methylphenyl)diazenyl]phenyl-1H-pyrazole-5-carboxamide), pyrimidoindole derivatives (e.g., UM171 (e.g., (1r,4r)-N1-(2-benzyl-7-(2-methyl-2H-tetrazol-5-yl)-9H-pyrimido[4,5-b]indol-4-yl)cyclohexane-1,4-diamine); UM729 (Methyl 4-((3-(piperidin-1-yl)propyl)amino)-9H-pyrimido[4,5-b] indole-7-carboxylate); UM118428 (e.g., Tranylcypromine HCl, (trans-2-Phenylcyclopropylamine hydrochloride)), and glucocorticoid receptor antagonists (e.g., mifepristone (e.g., RU-486), RU-43044, Miconazole, 11-oxa cortisol, 11-oxa prednisolone, Dexamethasone mesylate). Additional agents which could be utilized include protamine sulfate, rapamycin, polybrene, fibronectin fragment, prostaglandins or nonsteroidal anti-inflammatory drugs (e.g., celecoxib, diclofenac, diflunisal, etodolac, ibuprofen, indomethacin, ketoprofen, nabumetone, naproxen, oxaprozin, piroxicam, salsalate, sulindac, tolmetin). Endothelial cell co-culture may also be used.

Reference to CD34, CD45RA, CD90, CD117, CD123, and CD133 are understood by those of ordinary skill in the art (see, for example, FIG. 6). For other readers, CD (clusters of differentiation) antigens are proteins expressed on the surface of a cell that are detectable via specific antibodies. CD34 is a highly glycosylated type I transmembrane protein expressed on 1-4% of bone marrow cells. CD45RA is related to fibronectin type III, has a molecular weight of 205-220 kDa and is expressed on B cells, naïve T cells, and monocytes. CD90 is a GPI-cell anchored molecule found on prothymocyte cells in humans. CD117 is the c-kit ligand receptor found on 1-4% of bone marrow stem cells. CD123A is related to the cytokine receptor superfamily and the fibronectin type III superfamily, has a molecular weight of 70 kDa and is expressed on bone marrow stem cells granulocytes, monocytes and megakaryocytes. CD133 is a pentaspan transmembrane glycoprotein expressed on primitive hematopoietic progenitor cells.

In particular embodiments, disclosed cell populations can provide short- and long-term engraftment for a therapeutic purpose, for example, in the context of cord blood transplants, HSC transplants typically are used after radio-ablation of a patient, and other uses such as those described in Gratwohl *et al.,* 2010.

In particular embodiments, disclosed cell populations can undergo genetic modification for the desired research or therapeutic purpose. "Genetic modification or gene-modifying" means gene disruption, gene editing, gene transfer and any combination thereof In particular embodiments, gene modification or gene-modifying by gene transfer can be accomplished using any number of DNA or RNA viral vector based or non-viral vector based gene transfer technologies. Examples of viral-mediated gene transfer include lentiviral vectors, foamy viral vectors, adenoviral vectors, adeno-associated viral vectors, alpharetroviral vectors or gammaretroviral vectors; non-viral methods include transposon-mediated, plasmid DNA, nanoparticle delivery, or mRNA delivery using transfection, electroporation or nucleofection. Examples of gene editing technologies include specific nucleases, or non-nuclease-based methods. Examples of nuclease-based methods include Zinc fingers (ZFNs), tal effector nucleases (TALENs), meganucleases or meganuclease-TALEN fusions (MegaTALEs), or clustered regularly interspaced short palindromic repeats (CRISPR) delivered with a Cas9 nuclease. Examples of non-nuclease-mediated gene editing include chimeric transcription factors, chimeric chromatin modifiers, forced DNA looping or biodegradable nanoparticles. The phenotype of interest could be used to qualitatively and quantitatively assess the success of manipulations such as gene transfer or genetic editing, expansion or maintenance. For example, there are a number of molecules which are proposed to expand or maintain populations of hematopoietic cells such as StemRegennin1 (SR1), UM171, UM729, LY2228820, Notch Ligands, homeobox proteins, cytokines and chemokines. Measurement of this cell population before and after manipulation could be used as a measure of manipulation success. Aspects of particular of these options are now described in more detail.

Lentiviral vectors. "Lentivirus" refers to a genus of retroviruses that are capable of infecting dividing and non-dividing cells and typically produce high viral titers. Lentiviral vectors have been employed in gene therapy for a number of diseases. For example, hematopoietic gene therapies using lentiviral vectors or gamma retroviral vectors have been used for x-linked adrenoleukodystrophy and beta thalassaemia. See, e.g., Kohn *et al.,* 2010; Cartier *et al.,* 2012; and Cavazzana-Calvo *et al.,* 2010. Several examples of lentiviruses include HIV (human immunodeficiency virus: including HIV type 1, and HIV type 2); equine infectious anemia virus; feline immunodeficiency virus (FIV); bovine immune deficiency virus (BIV); and simian immunodeficiency virus (SIV).

Foamy viruses (FVes) are the largest retroviruses known today and are widespread among different mammals, including all non-human primate species, however are absent in humans. This complete apathogenicity qualifies FV vectors as ideal gene transfer vehicles for genetic therapies in humans and clearly distinguishes FV vectors as gene delivery system from HIV -derived and also gammaretrovirus-derived vectors.

FV vectors are suitable for gene therapy applications because they can (1) accommodate large transgenes (> 9kb), (2) transduce slowly dividing cells efficiently, and (3) integrate as a provirus into the genome of target cells, thus enabling stable long-term expression of the transgene(s). FV vectors do need cell division for the pre-integration complex to enter the nucleus, however the complex is stable for at least 30 days and still infective. The intracellular half-life of the FV pre-integration complex is comparable to the one of lentiviruses and significantly higher than for gammaretroviruses, therefore FV are also - similar to LV vectors - able to transduce rarely dividing cells. FV vectors are natural self-inactivating vectors and characterized by the fact that they seem to have hardly any potential to activate neighboring genes. In addition, FV vectors can enter any cells known (although the receptor is not identified yet) and infectious vector particles can be concentrated 100-fold without loss of infectivity due to a stable envelope protein. FV vectors achieve high transduction efficiency in pluripotent HSC and have been used in animal models to correct monogenetic diseases such as leukocyte adhesion deficiency (LAD) in dogs and Fanconi anemia in mice. FV vectors are also used in preclinical studies of β-thalassemia.

Additional examples of viral vectors include those derived from adenoviruses (e.g., adenovirus 5 (Ad5), adenovirus 35 (Ad35), adenovirus 11 (Ad11), adenovirus 26 (Ad26), adenovirus 48 (Ad48) or adenovirus 50 (Ad50)), adeno-associated virus (AAV; see, e.g., U.S. Pat. No. 5,604,090; Kay *et al.,* 2000; Nakai *et al.,* z1998), alphaviruses, cytomegaloviruses (CMV), flaviviruses, herpes viruses (e.g., herpes simplex), influenza viruses, papilloma viruses (e.g., human and bovine papilloma virus; see, e.g., U.S. Pat. No. 5,719,054), poxviruses, vaccinia viruses, etc. See Kozarsky and Wilson, 1993; Rosenfeld, *et al.,* 1991; Rosenfeld, *et al.,* 1992; Mastrangeli, *et al.,* 1993; Walsh, *et al.,* 1993; and Lundstrom, 1999. Examples include modified vaccinia Ankara (MVA) and NYVAC, or strains derived therefrom. Other examples include avipox vectors, such as a fowlpox vectors (e.g., FP9) or canarypox vectors (e.g., ALVAC and strains derived therefrom).

Other methods of gene delivery include use of artificial chromosome vectors such as mammalian artificial chromosomes (Vos, 1998) and yeast artificial chromosomes (YAC). YAC are typically used when the inserted nucleic acids are too large for more conventional vectors (e.g., greater than 12 kb).

Vectors and other methods to deliver nucleic acids can include regulatory sequences to control the expression of the nucleic acid molecules. These regulatory sequences can be eukaryotic or prokaryotic in nature. In particular embodiments, the regulatory sequence can be a tissue specific promoter such that the expression of the one or more therapeutic proteins will be substantially greater in the target tissue type compared to other types of tissue. In particular embodiments, the regulatory sequence can result in the constitutive expression of the one or more therapeutic proteins upon entry of the vector into the cell. Alternatively, the regulatory sequences can include inducible sequences. Inducible regulatory sequences are well known to those skilled in the art and are those sequences that require the presence of an additional inducing factor to result in expression of the one or more therapeutic proteins. Examples of suitable regulatory sequences include binding sites corresponding to tissue-specific transcription factors based on endogenous nuclear proteins, sequences that direct expression in a specific cell type, the lac operator, the tetracycline operator and the steroid hormone operator. Any inducible regulatory sequence known to those of skill in the art may be used.

In particular embodiments, the nucleic acid is stably integrated into the genome of a cell. In particular embodiments, the nucleic acid is stably maintained in a cell as a separate, episomal segment.

In particular embodiments, the efficiency of integration, the size of the DNA sequence that can be integrated, and the number of copies of a DNA sequence that can be integrated into a genome can be improved by using transposons. Transposons or transposable elements include a short nucleic acid sequence with terminal repeat sequences upstream and downstream. Active transposons can encode enzymes that facilitate the excision and insertion of nucleic acid into a target DNA sequence.

A number of transposable elements have been described in the art that facilitate insertion of nucleic acids into the genome of vertebrates, including humans. Examples include sleeping beauty (e.g., derived from the genome of salmonid fish); piggyback (e.g., derived from lepidopteran cells and/or the Myotis lucifugus); mariner (e.g., derived from Drosophila); frog prince (e.g., derived from Rana pipiens); Tol2 (e.g., derived from medaka fish); TcBuster (e.g., derived from the red flour beetle Tribolium castaneum) and spinON.

Additional Gene Editing Agents. Gene editing agents can modify or affect particular sequences of a cell's endogenous genome. In particular embodiments, the modification includes removal or disruption of an endogenous gene such that the endogenous gene's encoded protein is no longer expressed, expressed to a reduced degree, expressed as an incomplete protein, an unstable protein, an incorrectly folded protein and/or a nonfunctional protein. In particular embodiments, the effect is reduced expression of a protein through an interfering RNA-type mechanism. Thus, gene editing agents are useful for genome editing, for example gene disruption, gene editing by homologous recombination, and gene therapy to insert therapeutic genes at the appropriate chromosomal target sites with a human genome.

Particular gene editing agents include transcription activator-like effector nucleases (TALENs). TALENs refer to fusion proteins including a transcription activator-like effector (TALE) DNA binding protein and a DNA cleavage domain. TALENs are used to edit genes and genomes by inducing double strand breaks (DSBs) in the DNA, which induce repair mechanisms in cells. Generally, two TALENs must bind and flank each side of the target DNA site for the DNA cleavage domain to dimerize and induce a DSB. The DSB is repaired in the cell by non-homologous end-joining (NHEJ) or by homologous recombination (HR) with an exogenous double-stranded donor DNA fragment.

As indicated, TALENs have been engineered to bind a target sequence of, for example, an endogenous genome, and cut DNA at the location of the target sequence. The TALEs of TALENs are DNA binding proteins secreted by *Xanthomonas* bacteria. The DNA binding domain of TALEs include a highly conserved 33 or 34 amino acid repeat, with divergent residues at the 12^{th} and 13^{th} positions of each repeat. These two positions, referred to as the Repeat Variable Diresidue (RVD), show a strong correlation with specific nucleotide recognition. Accordingly, targeting specificity can be improved by changing the amino acids in the RVD and incorporating nonconventional RVD amino acids.

Examples of DNA cleavage domains that can be used in TALEN fusions are wild-type and variant Fokl endonucleases. The Fokl domain functions as a dimer requiring two constructs with unique DNA binding domains for sites on the target sequence. The Fokl cleavage domain cleaves within a five or six base pair spacer sequence separating the two inverted half-sites.

Particular embodiments utilize MegaTALs as gene editing agents. MegaTALs have a single chain rare-cleaving nuclease structure in which a TALE is fused with the DNA cleavage domain of a meganuclease. Meganucleases, also known as homing endonucleases, are single peptide chains that have both DNA recognition and nuclease function in the same domain. In contrast to the TALEN, the megaTAL only requires the delivery of a single peptide chain for functional activity.

Particular embodiments utilize zinc finger nucleases (ZFNs) as gene editing agents. ZFNs are a class of site-specific nucleases engineered to bind and cleave DNA at specific positions. ZFNs are used to introduce DSBs at a specific site in a DNA sequence which enables the ZFNs to target unique sequences within a genome in a variety of different cells. Moreover, subsequent to double-stranded breakage, homologous recombination or non-homologous end joining takes place to repair the DSB, thus enabling genome editing.

ZFNs are synthesized by fusing a zinc finger DNA-binding domain to a DNA cleavage domain. The DNA-binding domain includes three to six zinc finger proteins which are transcription factors. The DNA cleavage domain includes the catalytic domain of, for example, Fokl endonuclease.

Particular embodiments utilize CRISPR-Cas systems as gene editing agents. Guide RNA can be used, for example, with gene-editing agents such as CRISPR-Cas systems. CRISPR-Cas systems include CRISPR repeats and a set of CRISPR-associated genes (Cas).

The CRISPR repeats (clustered regularly interspaced short palindromic repeats) include a cluster of short direct repeats separated by spacers of short variable sequences of similar size as the repeats. The repeats range in size from 24 to 48 base pairs and have some dyad symmetry which implies the formation of a secondary structure, such as a hairpin, although the repeats are not truly palindromic. The spacers, separating the repeats, match exactly the sequences from prokaryotic viruses, plasmids, and transposons. The Cas genes encode nucleases, helicases, RNA-binding proteins, and a polymerase that unwind and cut DNA. Cas1, Cas2, and Cas9 are examples of Cas genes.

The source of CRISPR spacers indicate that CRISPR-Cas systems play a role in adaptive immunity in bacteria. There are at least three types of CRISPR-Cas immune system reactions, and Cas1 and Cas2 genes are involved in spacer acquisition in all three. Spacer acquisition, involving the capture and insertion of invading viral DNA into a CRISPR locus occurs in the first stage of adaptive immunity. More particularly, spacer acquisition begins with Cas1 and Cas2 recognizing invading DNA and cleaving a protospacer, which is ligated to the direct repeat adjacent to a leader sequence. Subsequently, single strand extension repairs take place and the direct repeat is duplicated.

The next stage of CRISPR-related adaptive immunity involves CRISPR RNA (crRNA) biogenesis, which occurs differently in each type of CRISPR-Cas system. In general, during this stage, the CRISPR transcript is cleaved by Cas genes to produce crRNAs. In the type I system, Cas6e/Cas6f cleaves the transcript. The type II system employs a transactivating (tracr) RNA to form a dsRNA, which is cleaved by Cas9 and RNase III. The type III system uses a Cas6 homolog for cleavage.

In the final stage of CRISPR-related adaptive immunity, processed crRNAs associate with Cas proteins to form interference complexes. In type I and type II systems, the Cas proteins interact with protospacer adjacent motifs (PAMs), which are short 3-5 bp DNA sequences, for degradation of invading DNA, while the type III systems do not require interaction with a PAM for degradation. In the type III-B system, the crRNA basepairs with the mRNA, instead of the targeted DNA, for degradation.

CRISPR-Cas systems thus function as an RNAi-like immune system in prokaryotes. The CRISPR-Cas technology has been exploited to inactivate genes in human cell lines and cells. As an example, the CRISPR-Cas9 system, which is based on the type II system, has been used as an agent for genome editing.

The type II system requires three components: Cas9, crRNA, and tracrRNA. The system can be simplified by combining tracrRNA and crRNA into a single synthetic single guide RNA (sgRNA).

At least three different Cas9 nucleases have been developed for genome editing. The first is the wild type Cas9 which introduces DSBs at a specific DNA site, resulting in the activation of DSB repair machinery. DSBs can be repaired by the NHEJ pathway or by homology-directed repair (HDR) pathway. The second is a mutant Cas9, known as the Cas9D10A, with only nickase activity, which means that it only cleaves one DNA strand and does not activate NHEJ. Thus, the DNA repairs proceed via the HDR pathway only. The third is a nuclease-deficient Cas9 (dCas9) which does not have cleavage activity but is able to bind DNA. Therefore, dCas9 is able to target specific sequences of a genome without cleavage. By fusing dCas9 with various effector domains, dCas9 can be used either as a gene silencing or activation tool. In certain embodiments, the method comprises contacting the HSC with a nucleic acid encoding a donor template for homology directed repair. For example, the donor template for HDR comprises homology arms to the target sequence, and a transgene to be inserted into the genome of the cell. In some embodiments, the transgene or trasngenes to be introduced into the genome, wherein the transgene restores native function of a monogenetic disease, provides insertion of an *in vivo* selection cassette, render cells resistant to pathogen infections, such as CCR5, and/or contain safety switch technology to destroy the cell in the event of an adverse clinical event. In particular embodiments, the *in vivo* selection cassettes include knockdown of HPRT with 6TG resistance (Choudhary *et al.,* 2013). In particular embodiments, the *in vivo* selection cassettes relies on the use of the methylguanine methyltransferase (MGMT) P140K mutation that is resistance to low dose chemotherapy of O6-benzylguanine and telozolomide (Gori *et al.,* 2012).

Therapeutic Uses of Genetically Modified Cell Populations. As one example, a gene can be selected to provide a therapeutically effective response against a condition that may be
inherited. The condition may be Grave's Disease, rheumatoid arthritis, pernicious anemia, Multiple Sclerosis (MS), inflammatory bowel disease, systemic lupus erythematosus (SLE), adenosine deaminase deficiency (ADA-SCID) or severe combined immunodeficiency disease (SCID) stemming from deficiency in gamma chain (X-linked SCID), JAK 3 kinase deficiency, purine nucleoside phsosphorylase deficiency, adenosine deaminase (ADA) defieciency, MHC Class II deficiency or recombinase activating gene (RAG) deficiency, Wiskott-Aldrich syndrome (WAS), chronic granulomatous disease (CGD), Fanconi anemia (FA), Battens disease, adrenoleukodystrophy (ALD) or metachromatic leukodystrophy (MLD), muscular dystrophy, pulmonary aveolar proteinosis (PAP), pyruvate kinase deficiency, Shwachmann-Diamond-Blackfan anemia, dyskeratosis congenita, xeroderma pigmentosa, cystic fibrosis, Parkinson's disease, Alzheimer's disease, or amyotrophic lateral sclerosis (Lou Gehrig's disease). Depending on the condition, the therapeutic gene may be a gene that encodes a protein and/or a gene whose function has been interrupted. Exemplary therapeutic gene and gene products include: soluble CD40; CTLA; Fas L; antibodies to CD4, CD5, CD7, CD52, etc.; antibodies to IL1, IL2, IL6; an antibody to TCR specifically present on autoreactive T cells; IL4; IL10; IL12; IL13; IL1Ra, sIL1R1, sIL1RII; sTNFRI; sTNFRII; antibodies to TNF; P53, PTPN22, and DRB1*1501/DQB1*0602; globin family genes; WAS; phox; FANC family genes; dystrophin; pyruvate kinase; CLN3; ABCD1; arylsulfatase A; SFTPB; SFTPC; NLX2.1; ABCA3; GATA1; ribosomal protein genes; TERT; TERC; DKC1; TINF2; CFTR; LRRK2; PARK2; PARK7; PINK1; SNCA; PSEN1; PSEN2; APP; SOD1; TDP43; FUS; ubiquilin 2; and/or C9ORF72. Therapeutically effective amounts may provide function to immune and other blood cells and/or microglial cells or may alternatively - depending on the treated condition - inhibit lymphocyte activation, induce apoptosis in lymphocytes, eliminate various subsets of lymphocytes, inhibit T cell activation, eliminate or inhibit autoreactive T cells, inhibit Th-2 or Th-1 lymphocyte activity, antagonize IL1 or TNF, reduce inflammation, induce selective tolerance to an inciting agent, reduce or eliminate an immune-mediated condition; and/or reduce or eliminate a symptom of the immune-mediated condition. Therapeutic effective amounts may also provide functional DNA repair mechanisms; surface protein expression; telomere maintenance; lysosomal function; breakdown of lipids or other proteins such as amyloids; permit ribosomal function; and/or permit development of mature blood cell lineages which would otherwise not develop such as macrophages other white blood cell types.

As another example, a gene can be selected to provide a therapeutically effective response against diseases related to red blood cells and clotting. The disease may be a hemoglobinopathy like thalassemia, or a sickle cell disease/trait. The therapeutic gene may be, for example, a gene that induces or increases production of hemoglobin; induces or increases production of beta-globin, or alpha-globin; or increases the availability of oxygen to cells in the body. The therapeutic gene may be, for example, HBB or CYB5R3. Exemplary effective treatments may, for example, increase blood cell counts, improve blood cell function, or increase oxygenation of cells in patients. The disease may be hemophilia. The therapeutic gene may be, for example, a gene that increases the production of coagulation/clotting factor VIII or coagulation/clotting factor IX, causes the production of normal versions of coagulation factor VIII or coagulation factor IX, a gene that reduces the production of antibodies to coagulation/clotting factor VIII or coagulation/clotting factor IX, or a gene that causes the proper formation of blood clots. Exemplary therapeutic genes include F8 and F9. Exemplary effective treatments may, for example, increase or induce the production of coagulation/clotting factors VIII and IX; improve the functioning of coagulation/clotting factors VIII and IX, or reduce clotting time in subjects.

Other uses with supporting detail are found in PCT/US2016/014378.

In these embodiments, cell populations (e.g., HSC populations, MPP populations) can be formulated into cell-based compositions for administration to a subject. A cell-based composition refers to expanded cells prepared with a pharmaceutically acceptable carrier for administration to ^{a} subject. Cell-based compositions may be administered to a subject in need thereof as soon as is reasonably possible following the completion of expansion and formulation for administration.

Exemplary carriers include saline, buffered saline, physiological saline, water, Hanks' solution, Ringer's solution, Nonnosol-R (Abbott Labs), Plasma-Lyte A^{®} (Baxter Laboratories, Inc., Morton Grove, IL), glycerol, ethanol, and combinations thereof.

In particular embodiments, carriers can be supplemented with human serum albumin (HSA) or other human serum components or fetal bovine serum or other species serum components. In particular embodiments, a carrier for infusion includes buffered saline with 5% HSA or dextrose. Additional isotonic agents include polyhydric sugar alcohols including trihydric or higher sugar alcohols, such as glycerin, erythritol, arabitol, xylitol, sorbitol, or mannitol.

Carriers can include buffering agents, such as citrate buffers, succinate buffers, tartrate buffers, fumarate buffers, gluconate buffers, oxalate buffers, lactate buffers, acetate buffers, phosphate buffers, histidine buffers, and/or trimethylamine salts.

Stabilizers refer to a broad category of excipients which can range in function from a bulking agent to an additive which helps to prevent cell adherence to container walls. Typical stabilizers can include polyhydric sugar alcohols; amino acids, such as arginine, lysine, glycine, glutamine, asparagine, histidine, alanine, ornithine, L-leucine, 2-phenylalanine, glutamic acid, and threonine; organic sugars or sugar alcohols, such as lactose, trehalose, stachyose, mannitol, sorbitol, xylitol, ribitol, myoinisitol, galactitol, glycerol, and cyclitols, such as inositol; PEG; amino acid polymers; sulfur-containing reducing agents, such as urea, glutathione, thioctic acid, sodium thioglycolate, thioglycerol, alpha-monothioglycerol, and sodium thiosulfate; low molecular weight polypeptides (i.e., <10 residues); proteins such as HSA, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran.

Where necessary or beneficial, cell-based compositions can include a local anesthetic such as lidocaine to ease pain at a site of injection.

Therapeutically effective amounts of cells within cell-based compositions can be greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹.

In cell-based compositions disclosed herein, cells are generally in a volume of a liter or less, 500 mls or less, 250 mls or less or 100 mls or less. Hence the density of administered cells is typically greater than 10⁴ cells/ml, 10⁷ cells/ml or 10⁸ cells/ml.

The cell-based compositions disclosed herein can be prepared for administration by, for example, injection, infusion, perfusion, or lavage.

In particular embodiments, it can be necessary or beneficial to cryopreserve a cell and/or cell-based composition. The terms "frozen/freezing" and "cryopreserved/cryopreserving" can be used interchangeably. Freezing includes freeze drying. As is understood by one of ordinary skill in the art, the freezing of cells can be destructive (see Mazur, P., 1977, Cryobiology 14:251-272) but there are numerous procedures available to prevent such damage. For example, damage can be avoided by (a) use of a cryoprotective agent, (b) control of the freezing rate, and/or (c) storage at a temperature sufficiently low to minimize degradative reactions. Exemplary cryoprotective agents include dimethyl sulfoxide (DMSO) (Lovelock and Bishop, 1959, Nature 183:1394-1395; Ashwood-Smith, 1961, Nature 190:1204-1205), glycerol, polyvinylpyrrolidine (Rinfret, 1960, Ann. N.Y. Acad. Sci. 85:576), polyethylene glycol (Sloviter and Ravdin, 1962, Nature 196:548), albumin, dextran, sucrose, ethylene glycol, i-erythritol, D-ribitol, D-mannitol (Rowe *et al.,* 1962), D-sorbitol, i-inositol, D-lactose, choline chloride (Bender *et al..,* 1960), amino acids (Phan The Tran and Bender, 1960), methanol, acetamide, glycerol monoacetate (Lovelock, 1954), and inorganic salts (Phan The Tran and Bender, 1960; Phan The Tran and Bender, 1961). In particular embodiments, DMSO can be used. Addition of plasma (e.g., to a concentration of 20-25%) can augment the protective effects of DMSO. After addition of DMSO, cells can be kept at 0° C until freezing, because DMSO concentrations of 1% can be toxic at temperatures above 4°C.

In the cryopreservation of cells, slow controlled cooling rates can be critical and different cryoprotective agents (Rapatz *et al.,* 1968) and different cell types have different optimal cooling rates (see e.g., Rowe and Rinfret, 1962; Rowe, 1968; Lewis, *et al.,* 1967; and Mazur, 1970 for effects of cooling velocity on survival of stem cells and on their transplantation potential). The heat of fusion phase where water turns to ice should be minimal. The cooling procedure can be carried out by use of, e.g., a programmable freezing device or a methanol bath procedure. Programmable freezing apparatuses allow determination of optimal cooling rates and facilitate standard reproducible cooling.

In particular embodiments, DMSO-treated cells can be pre-cooled on ice and transferred to a tray containing chilled methanol which is placed, in turn, in a mechanical refrigerator (e.g., Harris or Revco) at -80° C. Thermocouple measurements of the methanol bath and the samples indicate a cooling rate of 1° to 3° C/minute can be preferred. After at least two hours, the specimens can have reached a temperature of -80° C and can be placed directly into liquid nitrogen (-196° C).

After thorough freezing, the cells can be rapidly transferred to a long-term cryogenic storage vessel. In a preferred embodiment, samples can be cryogenically stored in liquid nitrogen (-196° C) or vapor (-1° C). Such storage is facilitated by the availability of highly efficient liquid nitrogen refrigerators.

Further considerations and procedures for the manipulation, cryopreservation, and long term storage of cells, can be found in the following exemplary references: U.S. Patent Nos. 4,199,022; 3,753,357; and 4,559,298; Gorin, 1986; Bone-Marrow Conservation, Culture and Transplantation, Proceedings of a Panel, Moscow, July 22-26, 1968, International Atomic Energy Agency, Vienna, pp. 107-186; Livesey and Linner, 1987; Linner *et al.,* 1986; Simione, 1992).

Following cryopreservation, frozen cells can be thawed for use in accordance with methods known to those of ordinary skill in the art. Frozen cells are preferably thawed quickly and chilled immediately upon thawing. In particular embodiments, the vial containing the frozen cells can be immersed up to its neck in a warm water bath; gentle rotation will ensure mixing of the cell suspension as it thaws and increase heat transfer from the warm water to the internal ice mass. As soon as the ice has completely melted, the vial can be immediately placed on ice.

**In** particular embodiments, methods can be used to prevent cellular clumping during thawing. Exemplary methods include: the addition before and/or after freezing of DNase (Spitzer *et al.,* 1980), low molecular weight dextran and citrate, hydroxyethyl starch (Stiff *et al.,* 1983), etc.

As is understood by one of ordinary skill in the art, if a cryoprotective agent that is toxic to humans is used, it should be removed prior to therapeutic use. DMSO has no serious toxicity.

### Example.1. Experimental Methods.

NHP Animal Care. Healthy juvenile pigtail macaques (Macaca nemestrina) as well as juvenile rhesus macaques (Macaca mulatta), which served as donors for BM CD34⁺ cells in these studies under conditions approved by the American Association for the Accreditation of Laboratory Animal Care.

Cell Sources, CD34⁺ Enrichment and in Vitro Culture. Human GCSF-primed peripheral blood stem cells (PBSCs) CD34⁺ cells were collected after informed consent according to the Declaration of Helsinki and enriched as previously described (Radtke *et al.,* 2015). Pigtail macaque steady state bone marrow, GCSF/SCF-primed bone marrow and umbilical cord blood, as well as Rhesus macaque steady state bone marrow CD34⁺ cells were harvested and enriched as previously described (Trobridge *et al.,* 2008). Briefly for enrichment of NHP CD34⁺ cells, red cells were lysed in ammonium chloride lysis buffer, while white blood cells were incubated for 20 minutes with the 12.8 immunoglobulin M anti-CD34 antibody, then washed and incubated for another 20 minutes with magnetic activated cell sorting anti-immunoglobulin M microbeads (Miltenyi Biotech, Bergisch Gladbach, Germany). For enrichment of human CD34⁺ cells, microbead conjugated anti-CD34 antibody (Miltenyi Biotech) was used. The cell suspension was run through magnetic columns enriching for CD34⁺ cell fractions with a purity of 60-80% confirmed by flow cytometry. Enriched CD34⁺ cells were cultured in StemSpan (Stemcell Technologies, Vancouver, British Columbia, Canada) supplemented with 100 U/ml penicillin streptomycin (Gibco by Life Technologies, Waltham, MA) and either SCF (Peprotech, Rocky Hill, NJ), TPO (Peprotech) and FLT3-L (Miltenyi Biotec), or SCF and IL-3 (Peprotech) (100 ng/ml each).

Flow Cytometry Analysis and FACS. Antibodies used for FACS analysis and sorting of pigtail macaque and rhesus macaque cells are listed in FIG. 2. Dead cells and debris were excluded via FSC/SSC gating. 123count eBeads (eBioscience, San Diego, CA) were used for quantification purposes. Flow cytometric analyses were performed on an LSR Ilu (BD, Franklin Lakes, NJ) and FACSAria Ilu (BD). Cells were sorted using a FACSAria Ilu cell sorter (BD) and sort purity assessed by recovery of sorted cells.

Colony-forming Cell (CFC) Assay. For CFC assays 1000-1200 sorted cells were seeded into 3.5 ml ColonyGEL 1402 (ReachBio, Seattle, WA). Hematopoietic colonies were scored after 12-14 days. Arising colonies were identified as colony forming unit- (CFU-) granulocyte (CFU-G), macrophage (CFU-M), granulocyte-macrophage (CFU-GM) and burst forming unit-erythrocyte (BFU-E). Colonies including erythroid and myeloid cells were scored as CFU-MIX. For secondary CFC assays, all cells or individual colonies were harvested, dissociated, washed, and seeded into 3.5 ml ColonyGel 1402. Hematopoietic colonies were analyzed/quantified after 12-14 days without discrimination of colony-subtypes.

Megakaryocyte Assay. For megakaryocyte differentiation 3,000-5,000 sorted cells were seeded into 2 ml MegaCult (StemCell Technologies) and differentiation performed according to the manufacturer's instructions. Myeloid (granulocytes and/or macrophages/monocytes) as well as megakaryocyte colonies were quantified microscopically after 10-12 days.

T Cell Assay. T cell differentiation potential was tested as previously described (La Motte-Mohs *et al.,* 2005). Sort-purified CD34-subpopulations were co-cultured with murine stromal cells OP9-DL1 expressing the mouse Delta-like 1 gene (Schmitt and Zuniga-Pflucker, 2002) in α-minimal essential medium (α MEM) (Gibco by Life Technologies) supplemented with 20% FBS, 100 U/ml penicillin, 100 U/ml streptomycin, 5 ng/ml FLT3-L, and 5 ng/ml IL-7 (R&D System, Minneapolis, MN). Half-media changes were performed on a bi-weekly basis. Obtained cells were stained with antibodies against CD2, CD3, CD4, CD5, CD8, and CD45 and analyzed/quantified by flow cytometry 5 weeks post-seeding.

MS-5 Assay. Lympho-myeloid differentiation potentials were tested in the clonal MS-5 assay as previously described (Doulatov *et al.,* 2010). Single-sorted hematopoietic progenitors were deposited onto stromal MS-5 cells (Itoh *et al.,* 1989) in 96-well plates. Co-cultures were carried out in H5100 medium (StemCell Technologies) supplemented with 100 U/ml penicillin, 100 U/ml streptomycin, 100 ng/ml SCF, 50 ng/ml TPO, 20 ng/ml IL-2 (R&D System) and 10 ng/ml IL-7 for 5 weeks with weekly half-media changes. Cells were harvested by physical dissociation, filtered through a 70-µm filter, and stained with antibodies against CD11b, CD14, CD20, CD34, CD45, and CD56 for analysis by flow cytometry.

RNA Isolation and RNA-Seq. Total RNA was extracted from sort-purified cell populations with the Arcturus PicoPure RNA Isolation Kit (Thermo Fisher Scientific, Waltham, MA) according to the manufacturer's protocol. All original RNA-seq data were uploaded to the NCBI database (BioProject Accession codes PRJNA320857 [NHP] and PRJNA320858 [human]). Detailed information on the RNA QC, sequencing, and analysis of RNA-seq data is below in the Supplemental Experimental Procedures sections.

Statistics. Statistical analysis was performed using GraphPad Prism Version 5. All data are given as mean ± standard error of the mean (SEM). Significance analyses was performed with the paired Student t test (*: p < 0.05; **: p < 0.01; ***: p <0.001).

Supplemental Experimental Procedures. *RNA quality control:* Total RNA integrity was analyzed using an Agilent 2200 TapeStation (Agilent Technologies, Inc., Santa Clara, CA) and quantified using a Trinean DropSense96 spectrophotometer (Caliper Life Sciences, Hopkinton, MA).

*RNA-seq expression analysis:* RNA-seq libraries were prepared from total RNA using the TruSeq RNA Sample Prep Kit (Illumina, Inc., San Diego, CA, USA) and a Sciclone NGSx Workstation (PerkinElmer, Waltham, MA, USA). Library size distribution was validated using an Agilent 2200 TapeStation (Agilent Technologies, Santa Clara, CA, USA). Additional library QC, blending of pooled indexed libraries, and cluster optimization was performed using Life Technologies Invitrogen Qubit^{®} 2.0 Fluorometer (Life Technologies-Invitrogen, Carlsbad, CA, USA). RNA-seq libraries were pooled and clustered onto a flow cell lane. Sequencing was performed using an Illumina HiSeq 2500 in rapid mode employing a paired-end, 50 base read length (PE50) sequencing strategy.

Image analysis and base calling was performed using Illumina's Real Time Analysis v1.18 software, followed by 'demultiplexing' of indexed reads and generation of FASTQ files, using Illumina's bcl2fastq Conversion Software v1.8.4.

*RNA-seq data analysis:* Paired reads were aligned to the human genome assembly (hg38) (Lander *et al.,* 2001) using bwa aln (Li and Durbin, 2009). SAM files were converted to BAM files and subsequently merged using the samtools view and merge options, respectively (Li *et al.,* 2009). The RNA-sequence analysis relies on the *Bioconductor* packages for R and follows the workflow outlined by Love *et al.* (2015) (Huber *et al.,* 2015; Love *et al.,* 2015). The gene transfer format (GTF) file (Homo_sapiens.GRCh38.83.gtf) used to define genomic features was obtained from Ensembl (Zerbino *et al.,* 2015) and read into R as a transcription database object using the *GenomicFeatures* package (Lawrence *et al.,* 2013). The *seqlevels, mapSeqlevels, newstyle,* and *renameSeqlevels* functions from the *GenomelnfoDb* package were used to convert from Ensembl chromosome labels to UCSC labels. Using the *GenomicFeatures* package, transcripts identified in the transcription database object were organized by genes as a *GRangesList* object (Lawrence *et al.,* 2013). Reads for each gene were counted using the *summarizeOverlaps* function from the *GenomicAlignments* package in "IntersectionNotEmpty" mode (Lawrence *et al.,* 2013). After replacing the column data of the generated *SummarizedExperiment* object with a custom, descriptive data frame was converted to a *DESeqDataSet* object with experimental groups corresponding to cell type and individual using the identically named function from the *DESeq2* package (Love *et al.,* 2014). Human samples were put through the same analysis separately to avoid forcing similarities in expression.

The non-human primate (NHP) data set was filtered to only include transcripts with more than one read across all NHP samples and subsequently analyzed for differential expression using the *DESeq* function of the *DESeq2* package (Love *et al.,* 2014). This creates a *DESeqDataSet* object that has estimated size factors and dispersions. Plots comparing mean expression of one condition to the relative log₂ fold change of another were generated for each comparison using the *plotMA* function from the *DESeq2* package and genes with an adjusted p-value < 0.05 were colored red. The data frames containing the relative expression values, p-values, and adjusted p-values of each gene for each comparison were saved at this point.

Returning to the filtered NHP *DESeqDataSet* with estimated size factors and dispersions, the *rlog* function from the *DESeq2* package was used in order to reduce the range of variances across mean expression values and allow for more accurate clustering of samples. The count data (*assay*) of the *DESeqDataSet* was transposed and supplied to the built-in *dist* R function to calculate the distance between samples. The resulting distances were converted to a matrix and supplied to the *pheatmap* function of the identically named package while also specifying that the original distances be used as the clustering distances for the samples. The *rlog* modified dataset was supplied to the *plotPCA* function from the *DESeq* package (Li and Durbin, 2009) to calculate the variation between samples and grouped by cell type and individual. Visualization was done using *ggplot2* as described in Love *et al.* 2015.

Returning to the data frames containing adjusted p-values for each pairwise comparison, genes were identified that had an adjusted p-value < 0.05, making them significantly differentially expressed. The complete (unfiltered) *DESeqDataSet* for both human and NHP samples had size factors and dispersion estimates calculated and were variance stabilized using the *DESeq* and *rlog* functions from the *DESeq2* package (Love *et al.,* 2014). For each species, the average rlog expression value was calculated for each gene. Before continuing the analysis, the set of genes was subset to those previously identified as differentially expressed in at least one of the NHP pairwise comparisons. For each remaining gene, the average rlog expression for each cell type in each species was calculated and compared to the mean rlog expression of that gene in order to calculate the deviation from the mean for each cell type. At this point, the ensembl gene games were converted to external gene names (when available) using the *biomaRt* package. Finally, scatterplots were generated using *ggplot2* for each set of cell types comparable across species. The average deviation from the rlog mean expression value in the NHP specific cell type samples was plotted against the average deviation from the rlog mean expression value in the corresponding human cell type samples, so each dot represents a single gene. Genes of interest were colored red and labeled. The number of genes falling within each quadrant was also calculated.

Autologous NHP transplants and *ex vivo* engineering of HSPCs. Autologous NHP transplants, priming (mobilization), collection of cells and genetic engineering were conducted consistent with previously published protocols (Trobridge et al., 2008). Briefly, animals were pretreated with G-CSF and SCF for 4 days to prime the BM. On day 4 after priming BM aspirates were performed, CD34⁺ fractions isolated, and cells plated into StemSpan SFEM containing 1% penicillin/streptomycin (P/S, Life Technologies) supplemented with SCF, TPO, and FLT3-L 100 ng/ml each. Cells were prestimulated overnight and re-plated in culture medium containing 4 µg/ml protamine sulfate, and 1 µg/ml Cyclosporine A onto flasks coated with CH296 (retronectin) for transduction with lentiviral vectors (MOI 10). A second dose of lentivirus (MOI 10 IU/cell) was added after 6-8 hours for overnight culture. Next day, cells were collected, residual virus removed, and washed cells re-plated in culture medium supplemented with prostaglandin E2 (PGE2). After 2 hours, cells were collected and prepared for infusion.

### Example 1 Definitions of Neutrophil/Platelet Engraftment and Engraftment Failure.

*Neutrophils:* The day of neutrophil engraftment is defined as a minimum of 500 neutrophils per µl peripheral blood (lower limit for engraftment: LLE) for a duration of at least 3 consecutive days. The duration of GCSF administration was not considered in this definition and is indicated by the yellow bar in each graph. Lower limit of normal (LLN) neutrophil counts in non-transplanted animals is 1,800 per µl.

*Platelets:* The day of platelet engraftment is defined as a minimum of 20,000-50,000 platelets per µl peripheral blood for a duration of at least 7 consecutive days, and trending toward a self-sustained increase in platelet counts without transfusion reaching greater than 50,000 platelets per µl. Lower limit of normal for platelet counts in non-transplanted animals is 260,000 per µl.

*Engraftment failure means no* sustained platelet counts above 50,000/mcL without transfusion support over the first 100 days after transplant. Additionally, drop of ANC to <500/mcL requiring G-CSF support, which became less effective over the first 100 days after transplant.

NHP Animal Housing and Care / Ethics statement. Healthy juvenile pigtail macaques (*Macaca nemestrina*) as well as juvenile rhesus macaques (*Macaca mulattaunder* conditions approved by the American Association for the Accreditation of Laboratory Animal Care. All experimental procedures performed were reviewed and approved by the Institutional Animal Care and Use Committee. This study was carried out in strict accordance with the recommendations in the Guide for the Care and Use of Laboratory Animals of the National Institutes of Health ("The Guide") and monkeys were randomly assigned to the study. This included at least twice-daily observation by animal technicians for basic husbandry parameters (e.g. food intake, activity, stool consistency, and overall appearance) as well as daily observation by a veterinary technician and/or veterinarian. Animals were housed in cages approved by "The Guide" and in accordance with Animal Welfare Act regulations. Animals were fed twice daily, and were fasted for up to 14 hours prior to sedation. Environmental enrichment included grouping in compound, large activity, or run-through connected cages, perches, toys, food treats, and foraging activities. If a clinical abnormality was noted by WaNPRC personnel, standard WaNPRC procedures were followed to notify the veterinary staff for evaluation and determination for admission as a clinical case. Animals were sedated by administration of ketamine HCl and/or telazol and supportive agents prior to all procedures. Following sedation, animals were monitored according to WaNPRC standard protocols. WaNPRC surgical support staff are trained and experienced in the administration of anesthetics and have monitoring equipment available to assist: electronic monitoring of heart rate, respiration, and blood oxygenation; audible alarms and LCD readouts; monitoring of blood pressure, temperature, etc. For minor procedures, the presence or absence of deep pain was tested by the toe-pinch reflex. The absence of response (leg flexion) to this test indicates adequate anesthesia for this procedure. Similar parameters were used in cases of general anesthesia, including the loss of palpebral reflexes (eye blink). Analgesics were provided as prescribed by the Clinical Veterinary staff for at least 48 hours after the procedures, and could be extended at the discretion of the clinical veterinarian, based on clinical signs. Decisions to euthanize animals were made in close consultation with veterinary staff and were performed in accordance with guidelines as established by the American Veterinary Medical Association Panel on Euthanasia (2013). Prior to euthanasia, animals were first rendered unconscious by administration of ketamine HCl.

Preconditioning and supportive care for autologous NHP transplants. In parallel to cell processing, macaques were conditioned with fractionated, myeloablative total body irradiation of 1020 cGy from a 6 MV x-ray beam of a single-source linear accelerator located at the Fred Hutchinson Cancer Research Center South Lake Union Facility (Seattle, Washington, USA); irradiation was administered as a fractionated dose over the 2 days before cell infusion. During irradiation, animals were housed in a specially modified cage that provided unrestricted access for the irradiation while simultaneously minimizing excess movement. The dose was administered at a rate of 7 cGy/min delivered as a midline tissue dose. Granulocyte colony-stimulating factor was administered daily from the day of cell infusion until the animals began to show onset of neutrophil recovery. Supportive care, including antibiotics, electrolytes, fluids, and transfusions, was given as necessary, and blood counts were analyzed daily to monitor hematopoietic recovery.

Integration Site Analysis (ISA). Processing of gDNA to amplify integration loci included either LAM-PCR (Beard, *et al.,* 2007) (animal T04228) or MGS-PCR (Beard, *et al.,* 2014) methods (animals M05189, J02370, Z08103 and Z09132). A variety of next generation sequencing platforms were used depending on date of transplant and sample collection. Platforms included single end 454 GS FLX Titanium (Roche), single end Ion Torrent PGM, and paired end Illumina Miseq. A detailed list of samples is included in a dataset available for download (*BioProject PRJNA357116).* Integration sites were identified using a method similar to that described by Hocum *et al.* 2015. For Illumina data, the forward and reverse reads were stitched using PEAR with the *-q 30* option to trim sequence reads after two bases with a quality score below 30 were observed (Zhang *et al.,* 2014). Stitched FASTQ files and raw FASTA files were filtered using custom python scripts. *Pairwise2* from the *Bio* module was used to confirm the presence of the LTR primer at the start of the sequence read using a gap open penalty of -2, a gap extension penalty of -1, and requiring a total mapping score of 24 or greater, equivalent to two mismatches or one insertion or deletion (indel). Presence or absence of the long terminal repeat (LTR) region was determined using *Pairwise2* to align a known 22 base pair sequence from the LTR region to the sequence read using the same gap penalties described previously and requiring a total mapping score of 20 or greater, corresponding to two mismatches or one indel. To remove reads representing vector sequences (as opposed to genomic sequences) a known 24 base pair sequence from the LV backbone was aligned to the sequence read using *Pairwise2* and the same settings as described for primer alignment. Reads containing the primer sequence and the LTR sequence but not the vector sequence were then trimmed at the end of the aligned LTR sequence and output in FASTQ or FASTA format depending on the input format. The *Macaca mulatta* reference genome (rheMac3, GCA_000230795.1, Oct. 2010) provided by the Beijing Genomics Institute, Shenzhen was downloaded from the UCSC genome browser (http://aenome.ucsc.edu/) (Kent, *et al.,,* (2002). Genome research). Filtered and trimmed sequence reads were aligned to the reference genome using BLAT with options *-out=blast8, -tileSize=11, -stepSize=5,* and - *ooc=rh11-2253.ooc* (Kent, *et al.,* (2002). BLAT--the BLAST-like alignment tool). The rh11-2253.occ file includes a list of 11-mers occurring at least 2,253 times in the genome to be masked by BLAT and was generated using the following command: $blat rheMac3.2bit /dev/null /dev/null -tileSize=11 -stepSize=5 -makeOoc=rh11-2253.ooc -repMatch=2253 as recommended by UCSC [http://genome.ucsc.edu/goldenpath/help/blatSpec.html and http://genome.ucsc.edu/FAQ/FAQblat.html#blat6]. Resulting blast8 files were parsed using a custom python script. The blast8 files contained multiple possible alignments for each sequence read, so any sequence read with a secondary alignment with percent identity up to 95% of the best alignment was discarded. Sequence reads were then grouped based on their genomic alignment positions and orientation (sense (+) vs. antisense (-)). Any alignments within 5 base pairs of one another with identical orientations were considered to originate from the same IS; the genomic position with the greatest number of contributing sequence reads is reported as the IS. Multiple sequencing runs of the same sample were combined using a custom python script by combining the number of sequence reads for each genomic position.

Persistence Analysis. For each sample, clone contributions were normalized by converting the number of sequence reads to frequencies for each sample. A filter was applied to only retain clones with a contribution of three or more sequence reads, then only those clones detected <3 months after transplant. For each subsequent sample, the sum of early engrafting clone contributions was calculated. Finally, all samples collected within the time frame were grouped for each animal. An example analysis is as follows: In animal J02370 there are three samples available from the first 3 months after transplant. 3,946, 1,633, and 93,885 integration site (IS)-associated sequence reads in samples from 29, 60, and 64 days after transplant respectively were identified. These IS-associated reads correspond to 1,615, 620, and 5,055 unique IS respectively. Some IS were identified in multiple samples, thus a total of 6,422 unique IS were identified between 0 and 3 months after transplant. These 6,422 unique IS account for 100% of the IS-associated sequence reads observed in samples from the first 3 months after transplant. However, only 527, 209, and 2,201 IS are represented by three or more sequence reads in each sample respectively. Again, some IS were identified in multiple samples, thus a total of 2,634 unique IS are represented by three or more sequence reads in samples from the first 3 months after transplant. These 2,634 clones are deemed early repopulating clones, and account for 63.7%, 65.8%, and 96.4% of IS associated sequence reads observed in the three samples from the first 3 months after transplant, making the average frequency of early repopulating clones in this time frame 75.3%. For the second time frame of interest (>3 months after transplant, but <12 months after transplant), there is one sample available (109 days after transplant). In this sample, 101,926 IS-associated reads representing 5,681 unique IS were found. Of the 5,681 IS-associated reads, 885 were also early repopulating clones, however only 776 of those were represented by three or more sequence reads. The 776 clones that are early repopulating clones and represented by three or more sequence reads account for 76.3% of IS-associated reads from the sample 109 days after transplant, giving the frequency of early repopulating clones observed in the second time frame. This process is repeated for the third and fourth time frames of interest (12-24 months after transplant and >24 months after transplant) to obtain an average frequency of early repopulating clones of 87.2% and 94.6%, respectively.

Identifying HSC Signatures. The HSC signature was defined as a clone signature present in more than one PB or BM cell lineage at a single time point more than 6 months after transplant and detected in any other sample at any other time point. A custom R script was used to identify HSC clone signatures, and to generate contribution graphs. All files for a given animal were read into R and sample clone contributions were normalized by converting to frequencies. Clones shared between subsets were identified as HSC clones and remaining samples were parsed to identify HSC clones present.

HSC Clone Contribution Graphs. HSC clone contributions were determined by dividing the number of sequence reads for each clone by the total number of HSC clone sequence reads for the sample. Unallocated clones were noted. The mean maximum frequency of HSC clones was calculated. One animal, Z09132, had 676 clones contributing above the mean maximum frequency of HSC clones, thus only the top 100 most abundant HSC clones were displayed due to color limitations. A data frame was constructed including three columns: (1), days after transplant; (2), clone frequency in the sample; (3), clone identifier (genomic locus or barcode). The data frame was visualized using the R package *ggplot2.*

Results. Persistence of Early Clones Over Years after Transplant. The time to full, multilineage reconstitution in the PM model was first established by discreet analysis of hematopoietic subsets in five transplanted animals (FIG. 3). The day of engraftment for each lineage was defined as the first day ≥1% fluorophore⁺ cells were observed with consecutive increases over the next three measurements. Based on these data, the following time frames were established: "initial engraftment" 0-3 months, "stabilization" 3 months to 1 year, "homeostasis" >1 year, and "long-term" >2 years after transplant.

To study the kinetics of individual blood cell clones in autologous CD34⁺ hematopoietic stem and progenitor cell (HSPC) grafts, engraftment of individual LV gene-modified cell clones were retrospectively analyzed by high-throughput retrovirus ISA (FIG. 2A). 134,528 clones (range 6,830 to 43,558 unique clones per animal, n=5) were followed for 2 to 7.5 years after transplant (FIG. 4). In all animals, clones engrafting in the first 0-3 months contributed more than half of the detected, gene-modified blood cells stably for >2 years post-transplant (FIG. 2A).

Early Engraftment of Persistent HSC Clones. To deduce whether any of these early engrafting and persistent clones displayed HSC biology *in vivo,* the specific clone signature (i.e. genomic locus of LV integration) had to be detected in multiple mature cell lineages late after transplant and detected at additional time points. Clone signatures shared between short-lived granulocytes or monocytes and longer-lived B cells or T cells were analyzed at time points ≥6 months after transplant in three of five animals (FIG. 4). 175, 291 and 740 shared (i.e. HSC) clone signatures were observed, representing 0.4%, 0.9% and 2.1% of all clones detected in these animals, respectively (FIG. 4). The most abundant of these HSC clone signatures were then tracked over time (FIG. 2B). Clones were characterized as "high abundance" HSC clones if their maximum contribution over time was greater than the mean maximum contribution for all HSC clones identified in the animal. Abundant HSC clones were observed within the first 6 months after transplant which were stably maintained throughout follow-up. These data demonstrate a subpopulation of CD34⁺ cells with multilineage capacity which engrafts very early after autologous transplantation and is stably maintained over years of recipient NHP lifetime.

Phenotypical analysis of NHP HSPC subpopulations. A great variety of cell surface markers have been described for the quantification and isolation of functionally distinct human HSPC populations (FIG. 6). To test whether antibodies for human HSPCs cross-react and mark phenotypically equivalent hematopoietic populations in the NHP, white blood cells (WBCs) were purified from steady state bone marrow (ssBM) of pigtail macaque (PM; Macaca nemestrina) as well as rhesus macaque (RM; Macaca mulatta) animals (FIG. 5A). Cross-species reactivity of 24 different antigens and 41 clones associated with mature blood cells as well as primitive hematopoiesis were tested by flow cytometry (summarized in FIG. 6).

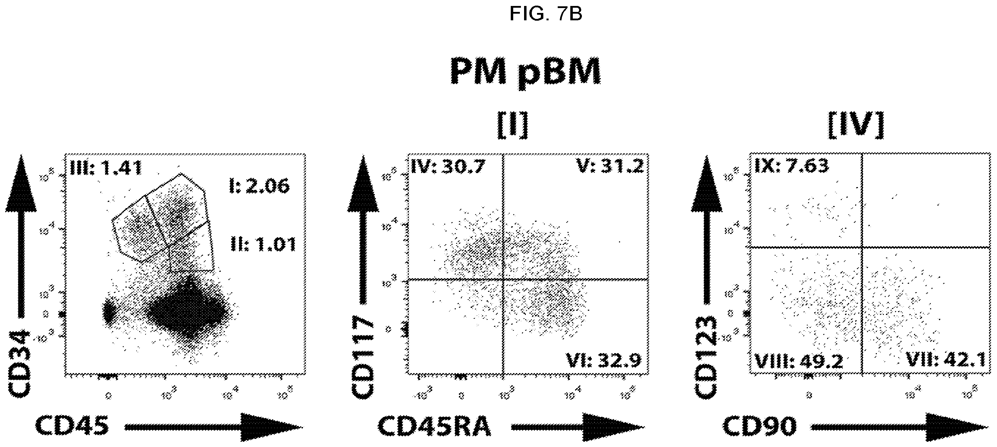

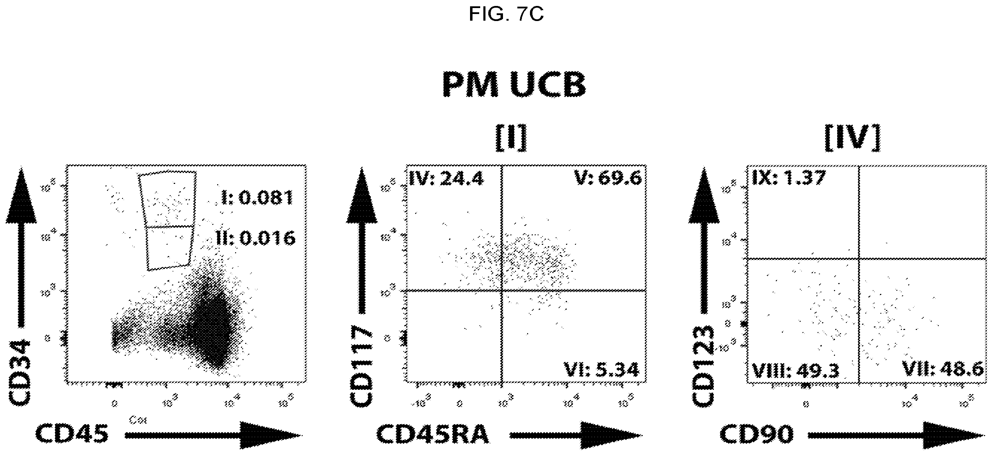

No cross-species reactivity or marking of distinct subpopulations was observed for candidate human HSPC cell surface markers CD38 (Terstappen *et al.,* 1991), CD49f (Notta *et al.,* 2011), CD133 (Yin *et al.,* 1997), and CD135 (Doulatov *et al.,* 2010). Thus, a focus on alternate HSPC markers including CD34 (Civin *et al.,* 1984), CD45RA (Lansdorp *et al.,* 1990), CD90 (Majeti *et al.*, 2007), CD117 (Kikushige *et al.,* 2008), and CD123 (Manz *et al.,* 2002), which demonstrated cross-reactivity in NHP ssBM was chosen. Using these markers nine (I-IX) phenotypically distinct subpopulations within CD34⁺ cells from PM and RM ssBM (FIG. 5B and FIG. 7A) were identified. CD34-expressing cells were first subdivided into three fractions showing low, intermediate (int), or high expression of CD45 and CD34: CD34^{high}CD45^{int} (I), CD34^{low}CD45^{int} (II) and CD34^{int}CD45^{low} (III). None of the other candidate HSPC markers (CD117, CD90, or CD123) were observed in Populations II or III. From Population I three further subdivisions: CD45RA⁻CD117⁺ (IV), CD45RA⁺CD117⁺ (V) and CD45RA⁺CD117⁻ (VI) cells were identified, with only Population IV containing CD90⁺ or CD123⁺ cells. Population IV was further subdivided into CD90⁺CD123⁻ (VII), CD90⁻CD123⁻ (VII) and CD90⁻CD123⁺ (IX) subpopulations based on expression of these markers (FIG. 5B and FIG. 7A). Once phenotyping was established, these analyses were extended to GCSF/SCF-primed BM (pBM) as well as umbilical cord blood (UCB) from PM (FIG. 7B,7C). Phenotypically identical CD34-expressing subpopulations were identified within WBCs from these sources (FIG. 7B,7C). A comprehensive list of identified subpopulations and the nomenclature used throughout the disclosure is presented in FIG. 5C.

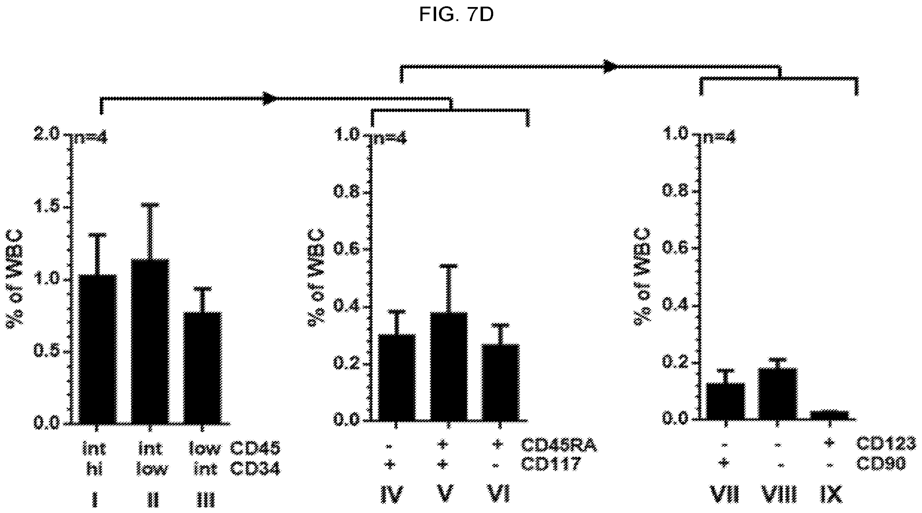

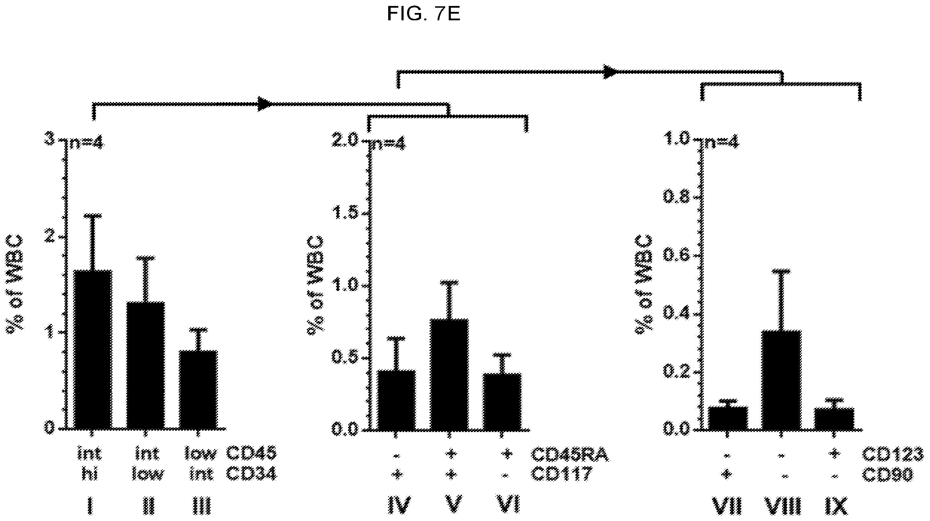

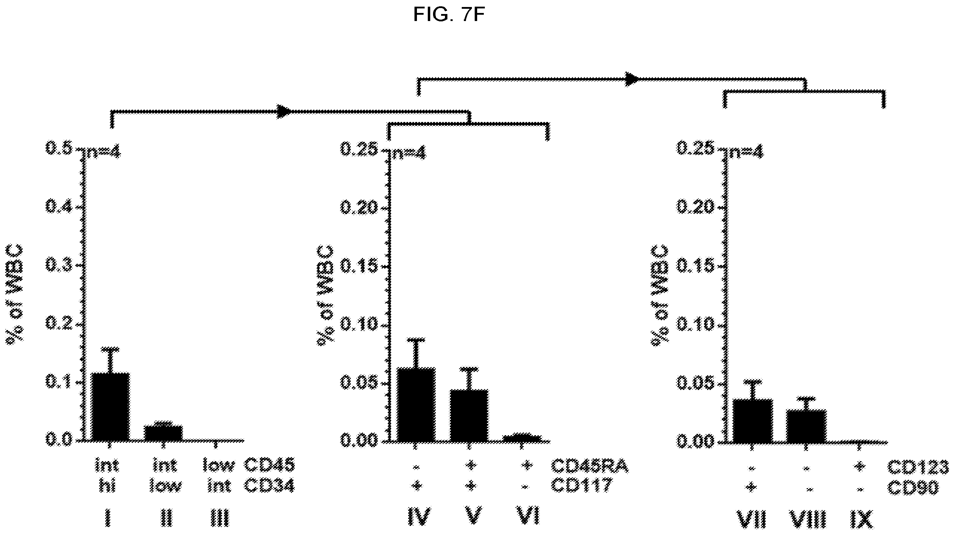

The average frequency of CD34-expressing progenitors in ssBM and pBM, was 5.8 ± 2.9% in PM and 2.2 ± 0.7% in RM (FIG. 5D, FIG. 7D, 7E). Identified subpopulations within CD34⁺ cell fractions showed equal frequencies in PM and RM ssBM as well as PM pBM (FIG. 5D, FIG. 7D,7E). However, compared to ssBM and pBM, only very low numbers of CD34⁺ cells were found in PM UCB (0.13 ± 0.04%), but within these increased frequencies of fraction I, IV and VII (FIG. 7F) were observed.

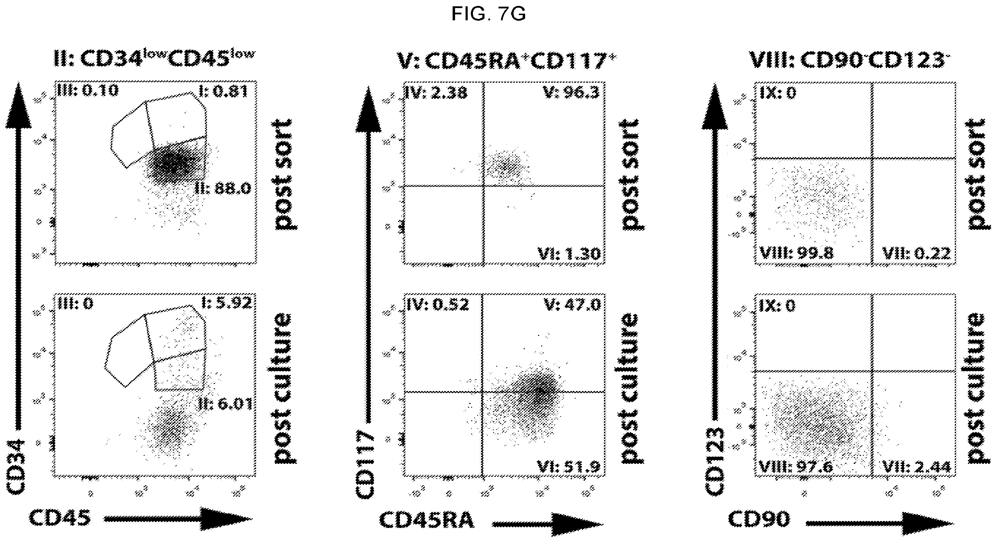

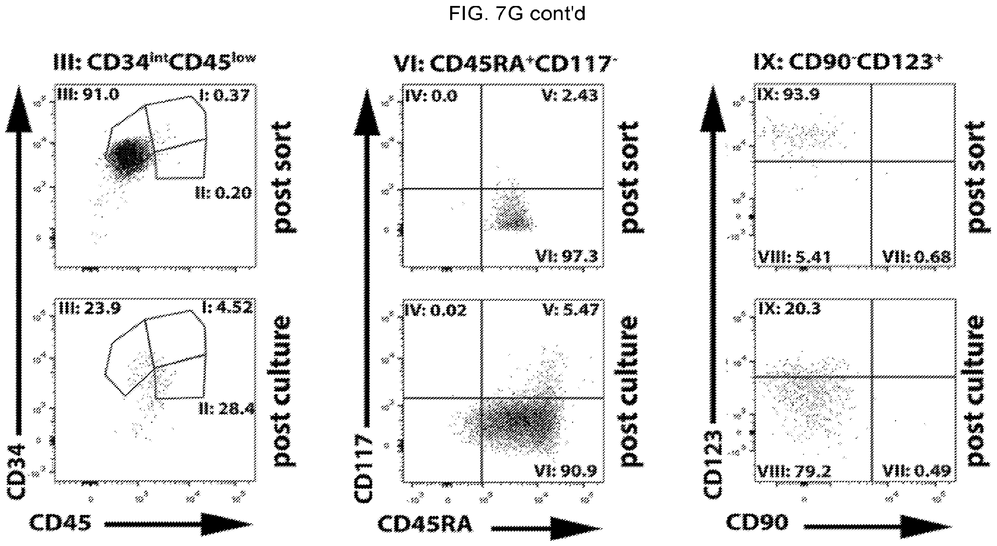

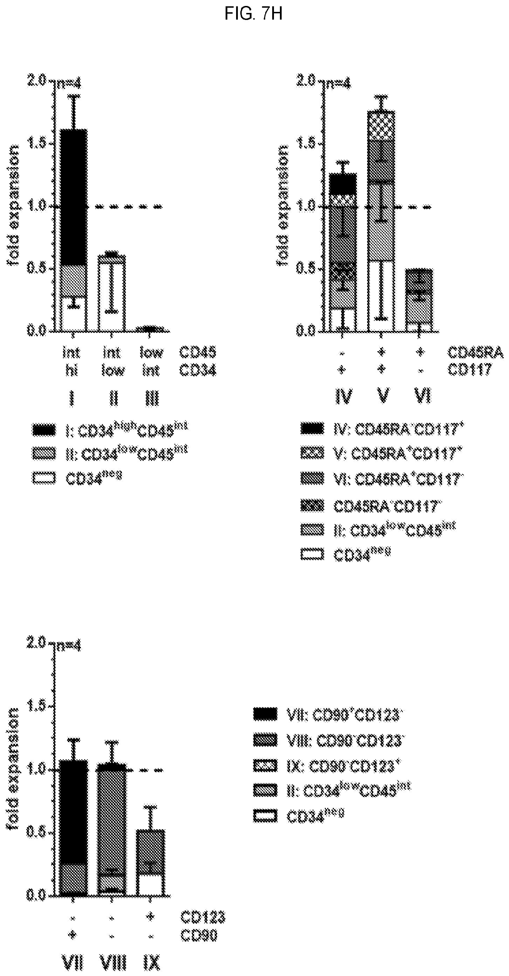

Proliferative potential of defined NHP HSPC subpopulations. The ability to self-renew is a classical definition of a true HSC, thus the proliferation potential of disclosed HSPC subpopulations was first examined. In order to investigate the proliferation potential, PM subpopulations I-IX from ssBM were purified, separately cultured for three days, and the arising progeny were enumerated and analyzed by flow cytometry. All subpopulations lost CD34, CD90 and CD117 expression and gained CD45RA expression upon culture (FIG. 5E). Cells downregulating CD34, CD90 and CD117 or gaining CD45RA never regained or lost marker expression, respectively (FIG. 7G). During culture, Population V demonstrated the greatest proliferation (fold-expansion >> 1), followed by Populations I and IV (fold-expansion > 1), whereas Populations VII and VIII demonstrated no change in cell number (fold-expansion ±1), and Populations II, III, VI and IX demonstrated cell losses (fold-expansion < 1) (FIG. 7H).

Based on these cell surface marker phenotypes, proliferation potential analyses, and reported lineage relationships in human hematopoietic cells, a hierarchical organization of NHP HSPCs, with fraction VII being the most primitive HSPC population generating fraction VIII with a concomitant loss of CD90 expression was predicted (FIG. 1B). CD90⁻ HSPCs (VIII) were proposed to either gain CD45RA-expression giving rise to fraction V and VI or down-regulate CD34-expression as seen for fraction II (FIG. 5F). This proposed hierarchy was next functionally evaluated.

Early segregation of erythroid potentials into CD34^{low} and megakaryocytes into CD123⁺ fractions. To validate the proposed lineage relationships, functional in vitro assays were then applied to the phenotypically defined HSPC subpopulations to analyze and compare erythroid, myeloid and lymphoid lineages in the NHP with recently proposed models for human hematopoiesis.

To determine the myeloid, erythroid and erythro-myeloid differentiation potential of PM and RM ssBM, pBM, and UCB-derived HSPCs, sorted populations were introduced into CFC assays supporting differentiation of CFU-G, CFU-M, CFU-GM, BFU-E as well as CFU-MIX potential. Shapes and sizes of colonies (FIG. 8A and FIG. 9A) as well as morphology and quality of colonies (FIG. 8B) was identical to previously reported colony assays of human HSPCs (Görgens *et al.,* 2013).

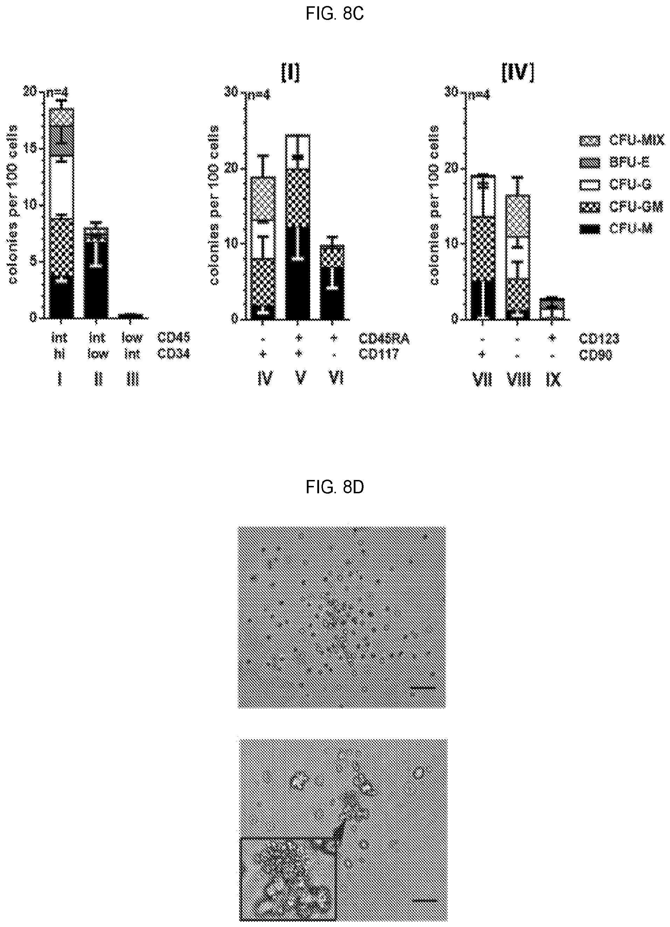

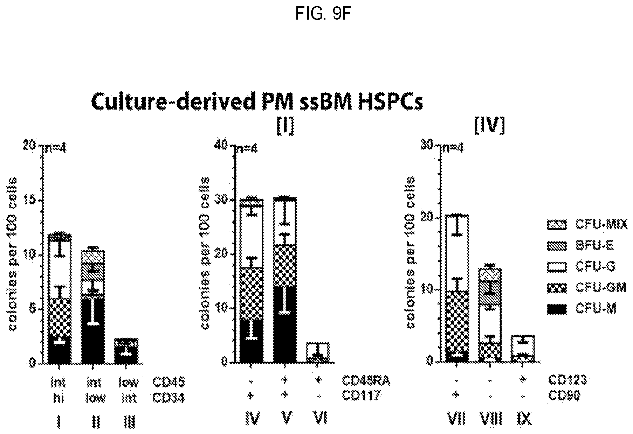

**The** frequency of individual cells with colony-forming potential in NHP HSPC subpopulations ranged from 1% to 40%, with the highest CFC frequencies observed in Populations I, IV, V, VII and VIII (FIG. 8C and FIG. 9B-9D). CFC frequencies were consistent and reproducible within defined subpopulations, independent of the source (ssBM, pBM, UCB) or species tested (PM, RM). Discrimination of colony types revealed that only Populations I, IV and VIII contained progenitors with myeloid, erythroid, and erythro-myeloid differentiation potential (FIG. 8C, FIG. 9B-9E). NHP HSPCs expressing or up-regulating CD45RA (Population V and VI) remained restricted to myeloid lineages, whereas erythroid potentials segregated into Population II and III upon cultivation and down-regulation of CD34-expression (FIG. 9F).

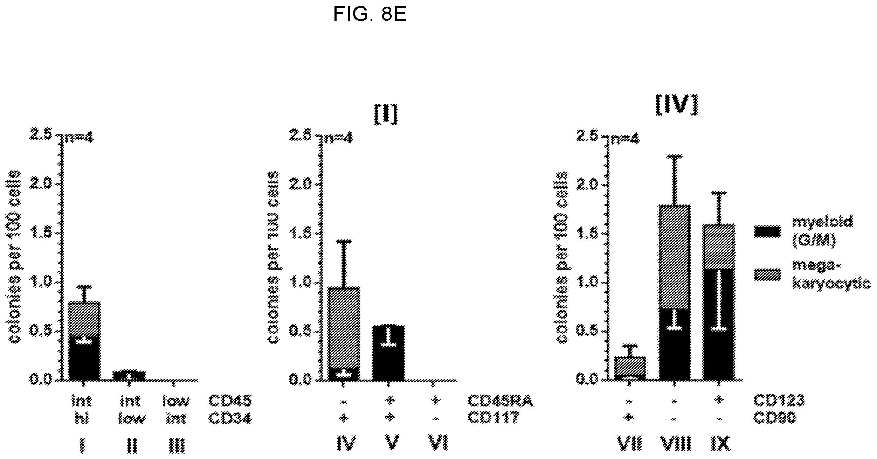

To test the origin of megakaryocyte potential, ssBM-derived PM HSPCs were studied in megakaryocyte differentiation assays. Generation of megakaryocyte colonies (FIG. 8D) was shown for Populations I, IV and VIII, which also displayed erythroid differentiation potential in CFC assays, whereas CD45RA⁺ Populations (V and VI) lacked megakaryocyte potential, giving rise to myeloid (G/M) colonies only (FIG. 8E). Interestingly, Population IX, which entirely lacked erythroid potential, gave rise to megakaryocytes, while erythro-myeloid progenitors from Populations II and III lacked megakaryocyte potential (FIG. 8E).

Together, these data indicate that in CFC and megakaryocyte assays NHP erythroid potential segregates into CD34^{low} cells (Population II) and megakaryocyte potential is restricted to CD123⁺ cells (Population IX), whereas CD45RA⁺ HSPCs (Populations V and VI) are lacking erythroid and megakaryocytic differentiation potentials.

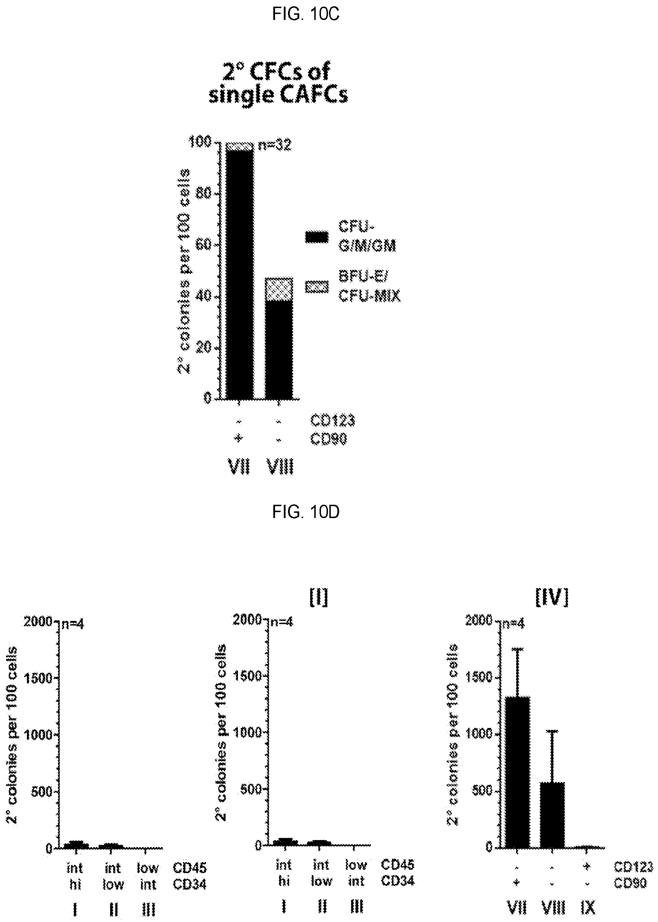

Enrichment of primitive HSPCs in CD90⁺ cell populations. The formation of colonies containing cobblestone area forming cells (CAFCs) is suggested to represent primitive and multipotent HSPCs (de Kruijf *et al.,* 2010; Ploemacher *et al.,* 1989). Closer examination of primary CFC assays reveals that Populations VII and VIII contained clusters of CAFCs (FIG. 10A). CAFCs from Population VIII were mostly associated with CFU-G or CFU-MIX colonies containing no CD34⁺ progenitor cells by flow analysis, whereas autonomous CAFCs contained CD34⁺CD45RA⁺ cells and CD34⁺CD45RA⁻ progenitor cells (FIG. 10B). To test whether CAFCs are primitive cells requiring additional time to develop cell fates in CFC assays, single CAFCs were introduced into secondary CFC assays. All CAFCs from Population VII gave rise to myeloid and erythroid colonies in secondary assays, whereas only 50% of CAFCs from Population VIII showed CFC potential (FIG. 10C). To confirm this, secondary-colony forming potential was tested in freshly isolated as well as culture-derived cells from all Populations I-IX, in both PM-derived and RM-derived ssBM, pBM, and UCB (FIG. 10D and FIG. 11A-11D).

These data show that primitive NHP HSPCs with erythroid, myeloid, megakaryocyte and secondary colony forming-potential are enriched in CD90⁺ subpopulations (Population VII).

CD45RA⁺ HSPC populations are restricted to lympho-myeloid potentials. To analyze the segregation of lymphoid potentials in NHP-hematopoiesis, sort-purified ssBM-derived PM subpopulations were introduced into functional assays that support the differentiation of human HSPCs into T cells, B cells and/or NK cells.

For T cell assays, subpopulations were co-cultured with the murine stromal cell line OP9 stably expressing the Notch-Ligand Delta-like 1 as described previously (La Motte-Mohs *et al.,* 2005). NHP ssBM-derived HSPCs with T cell differentiation potential up-regulated expression of the early lymphoid progenitor markers CD2 and CD5, and CD3 and CD4 were expressed on more mature T cells (FIG. 12A and FIG. 13A). CD4⁺ T helper cells did co-express the T cell receptor (TCR) CD3, whereas differentiation of NHP CD8⁺ cytotoxic T cells was not supported (FIG. 13A). HSPCs with T cell differentiation potential were found in Populations I, IV, V, VII, and VIII, with Population V generating the highest number of mature CD3⁺CD4⁺ T cells (FIG. 12B). Immature and mature T cells were barely detectable in Populations II and IX (FIG. 12B).

To test whether subpopulations containing T cell potentials can also give rise to B and NK cells, single, sort-purified HSPCs were introduced into a previously described clonogenic, multi-lineage readout (MS-5 assay) supporting lymphoid and myeloid differentiation (Doulatov *et al.,* 2010).

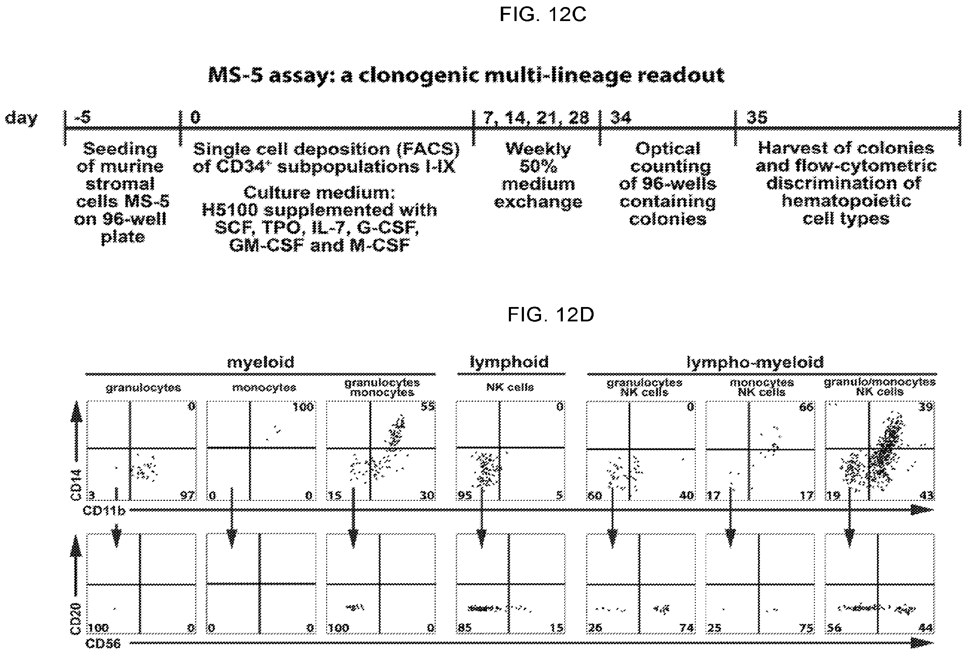

A total of 1,914 flow-sorted single cells out of 4 independent ssBM samples were co-cultured for 5 weeks with the murine stromal cell line MS-5, the colony-forming potential evaluated microscopically, and the phenotype of progeny determined by flow-cytometry (Experimental schema shown in FIG. 12C). Single cells showed lymphoid, myeloid, as well as lympho-myeloid differentiation potential giving rise to granulocytes (CD11b⁺CD14⁻), monocytes (CD11⁺CD14⁺), and NK cells (CD11b⁻CD14⁻CD56⁺) (FIG. 12D). Combinations of granulocytes-monocytes, granulocytes-NK, monocytes-NK, as well as granulocytes-monocytes-NK were found. B cell differentiation (CD20⁺) of NHP HSPCs was not supported within MS-5 assay.

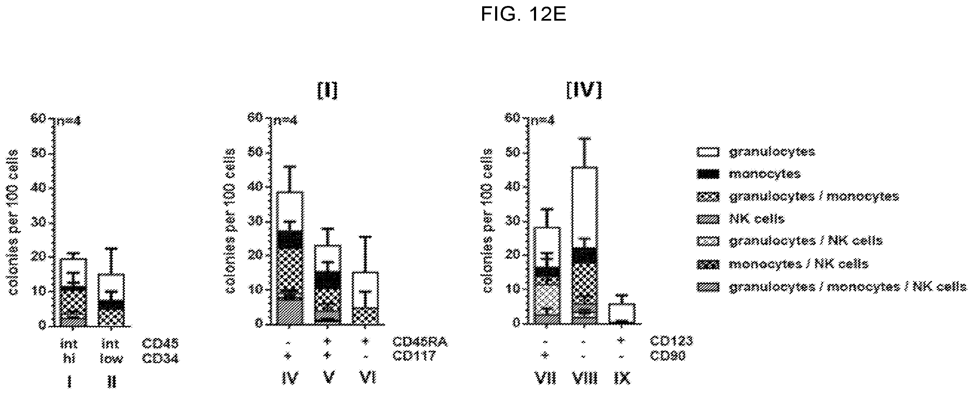

The frequency of HSPCs with clonogenic colony-forming potential following expansion and differentiation in the MS-5 co-culture ranged from 5.7 ± 2.4% in Population IX up to 45.8 ± 7.9% in Population VIII (FIG. 12E, FIG. 14). Populations I, IV, VII and VIII, previously shown to contain more primitive progenitor cells with CFU-MIX potential and substantial secondary CFC potential, showed the highest clonogenic potential in this assay (FIG. 12E, FIG. 14). All tested Populations contained progenitor cells with myeloid differentiation potential, whereas NK cells were exclusively found within Populations showing T cell potentials (I, IV, V, VII and VIII; FIGs. 12B and 12E). Mixed colonies containing granulocytes, monocytes, and NK cells were limited to Populations I, IV, VII and VIII. Uni-lineage progenitor cells containing only NK cell potential were seen in Populations V and VII.

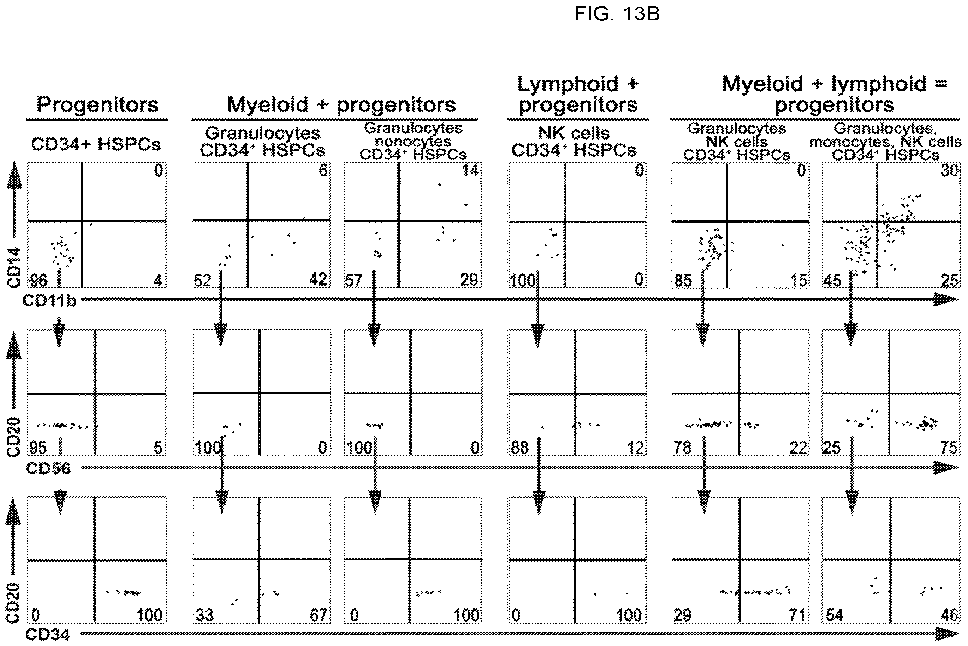

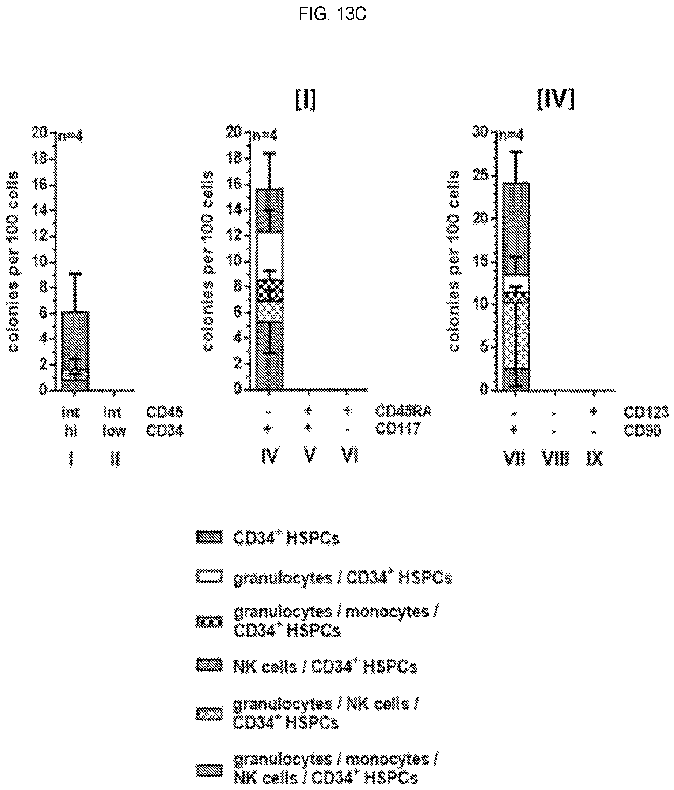

Besides mature granulocytes, monocytes and NK cells, the MS-5 assay supported the maintenance and expansion of CD34⁺ HSPCs forming characteristic cobblestones previously observed in CFC assays (FIG. 10A). Different types of colonies containing granulocytes-HSPCs, granulocytes-monocytes-HSPCs, NK-HSPCs, granulocytes-NK-HSPCs, granulocytes-monocytes-NK-HSPCs, as well as only HSPCs were identified (FIG. 13B). Colonies containing CD34⁺ HSPCs were exclusively found within Populations I, IV and VII (FIG. 13C, FIG. 14). The greatest numbers of colonies containing only CD34⁺ HSPCs were observed in Population VII (FIG. 13C).

These data confirm that lymphoid lineages in the NHP exclusively segregate into CD45RA⁺ cell Populations (VI) also containing granulocyte and macrophage/monocyte capabilities but lacking erythro-megakaryocytic potentials.

Unique transcriptome in NHP stem cells and committed progenitors. Expression of key molecules and activation of distinct biological pathways has been described for more primitive HSCs/MPPs and committed progenitor cells (Notta *et al.,* 2015; Paul *et al.,* 2015). To examine the NHP hematopoietic transcriptome, subpopulations of GCSF/SCF-primed PM-HSPCs either harvested from the bone marrow (pBM) or collected by apheresis (peripheral blood stem cells: PBSCs) were sort-purified for RNA extraction (FIG. 15A). RNA samples with poor integrity as demonstrated by RIN^{e} (RNA integrity number) values lower than 8.0 (Populations II and III, FIG. 15B) were excluded for RNA sequencing (RNA-seq).

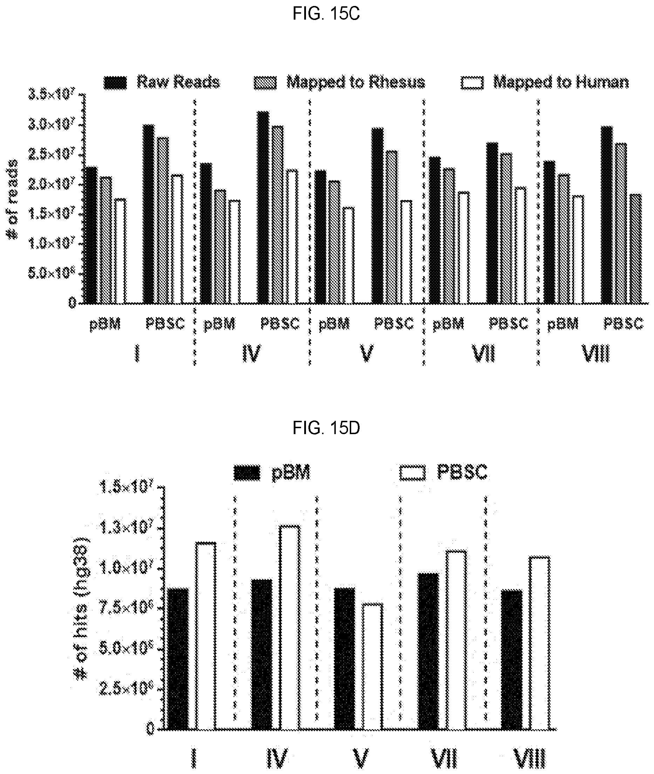

RNA-seq data was aligned to the rhesus (rhemac3) and the human (hg38) genome with 81-93% and 67-91% mapped reads, respectively (FIG. 15C). Due to significantly reduced annotation of the rhesus genome, a comprehensive gene-expression analysis and quantification of expression with the human genome was performed. 47-54% (pBM) and 45-57% (PBSCs) of mapped reads were located in exons of annotated genes and used for downstream analysis (FIG. 15D).

Principal component analysis (FIG. 16A) and unsupervised hierarchical clustering (FIG. 16B) of RNA-seq data showed similar patterns of HSPC subpopulation distributions as a function of source (pBM or PBSCs). Within these transcriptome distributions, Populations VII and V showed the greatest distance, whereas Populations VII and VIII as well as Population IV - a pool of VII and VIII - were more closely related to one other (FIG. 16A, 16B). Interestingly, PBSC-derived circulating progenitor cells lacking CD90 expression (Populations IV, V and VIII; black) appeared more closely related to lympho-myeloid restricted progenitors (V; gray) from pBM (FIG. 16B). Only the HSC-enriched subpopulation in PBSCs (Population VII; black) showed similarities in expression with pBM-derived subpopulations specifically enriched for multipotent HSPCs (IV, VII and VIII; black; FIG. 16B).

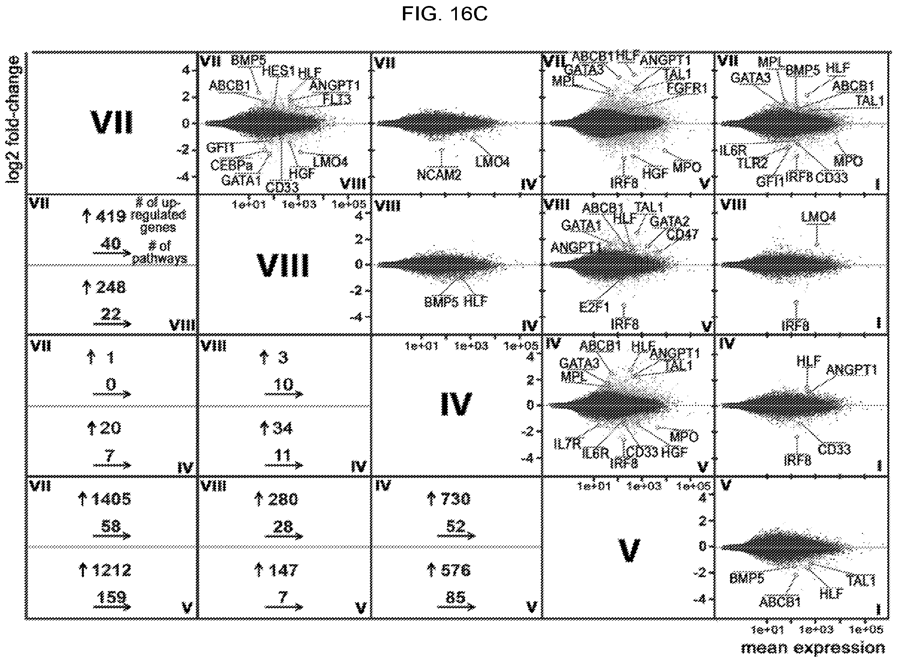

To identify genes differentially/uniquely expressed in functionally distinct NHP HSPC subpopulations, a pair-wise comparison of gene expression levels was next performed. Confirming the greatest distance within the unsupervised hierarchical clustering (FIG. 16B), the most differentially expressed genes were found when comparing Populations VII (1,405 genes) and V (1,212 genes) (FIG. 16C). Comparison of more closely related HSPC populations, e.g. Population IV and both of its subpopulations (Populations VII and VIII), resulted in a low number of differentially expressed genes. There was significant up-regulation of genes reported in human hematopoiesis as associated with primitive HSCs/MPPs (ABCB1, ANGPT1, BMP5, CD47, FLT3, HES1, TAL1) (Notta *et al.,* 2015; Paul *et al.,* 2015) or committed progenitor cells of the lympho-myeloid lineage (CD33, CEBPa, GFI1, HGF, IL6R, IL7R, IRF8, MPO) (Notta *et al.,* 2015; Paul *et* al., 2015) exclusively in Populations VII and V, respectively (highlighted genes in scatter plots, FIG. 16C). Up-regulated genes in Population V were highly enriched for pathways associated with translation, transcription, signaling, immune response, and migration/adhesion, whereas the vast majority of active pathways in Population VII were related to proliferation and proliferation-associated DNA/RNA/protein processing (FIG. 17).

Consistent with the phenotypical and functional analyses, CD34⁺CD45RA⁻CD90⁺CD117⁺ (Population VII) NHP HSPCs showed a transcriptional profile consistent with more primitive HSC, whereas CD34⁺CD45RA⁺CD117⁺ (Population V) progenitor transcriptomes were consistent with lympho-myeloid lineages.

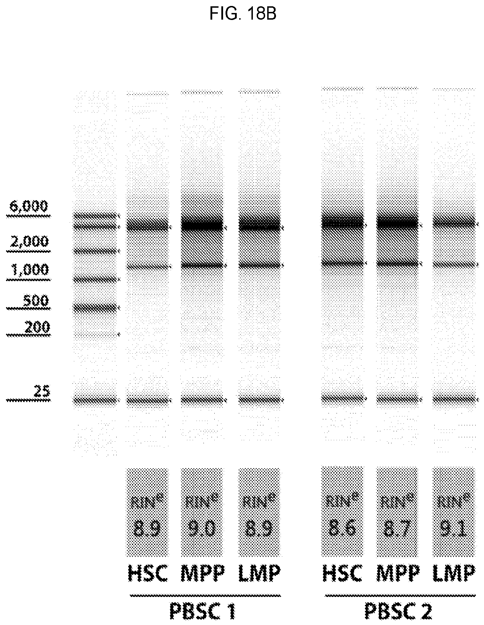

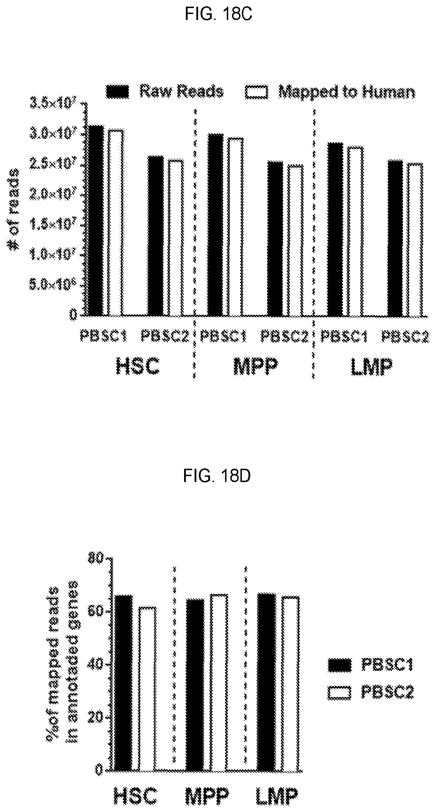

The transcriptome of human and NHP HSPC subpopulations is evolutionarily conserved. Nonhuman primates share a close evolutionary relationship with humans and are considered to be a clinically relevant model system to study HSC biology and treatments for hematopoietic diseases (Donahue *et al.,* 2005). To evaluate whether phenotypically and functionally defined human HSPC fractions correspond to the newly identified NHP HSPC subpopulations, the transcriptome of human and NHP HSPC subpopulations was compared. Representing NHP HSPC Populations VII, VIII, and V, human HSC- (CD34⁺CD45RA⁻CD90⁺CD133⁺), MPP-(CD34⁺CD45RA⁻CD90⁻CD133⁺), and LMP-enriched (CD34⁺CD45RA⁺CD90⁻CD133⁺) fractions were sort-purified from two independent GCSF-primed healthy PBSC donors for RNA extraction and sequencing (FIG. 18A, 18B). RNA-seq data was aligned to the human (hg38) genome with >97% reads mapped (FIG. 18C). 64-67% (hPBSC Donor 1) and 61-67% (hPBSC Donor 2) of mapped reads were located in exons of annotated genes and used for downstream analysis and comparison to RNA-seq data from NHP HSPCs (FIG. 18D).

To determine the overlap of gene expression and identify shared stem cell programs between human and NHP HSPCs, the focus was on differentially expressed genes previously identified in the pair-wise comparison of NHP HSPCs (FIG. 16C, total number of genes: 2,983). For the comparison of gene expression patterns and visualization of differences in expression levels across human and NHP HSPCs, the log₂ fold-change of expression was calculated for each species and gene individually (FIG. 19A-19C).

Comparison of RNA expression in corresponding HSPC subpopulations from human and NHP revealed highly conserved patterns of expression for 72.7% (VII vs. HSC), 58.3% (VIII vs. MPP) and 73.0% (V vs. LMP) of differentially expressed genes (FIG. 19A-19C). Key factors reported to be up-regulated in multipotent human HSCs (ABCB1, ANGPT1, FGFR1, GATA3, HES1, HLF, TAL1, MPL) (Notta *et al.,* 2015; Paul *et al.,* 2015) were also found to be expressed in NHP HSPC Population VII (FIG. 19A). Simultaneously, only low levels of expression for differentiation markers CD33, GFI1, HDC, HGF, IL7R, IRF8, MPO, and NOTCH3 (Notta *et al.,* 2015; Paul *et al.,* 2015) were observed in human HSC-enriched fractions and NHP Population VII (FIG. 19A). On the other hand, genes expressed in the lympho-myeloid restricted human LMP-enriched fraction, associated with more committed progenitor cells (CD33, GFI1, IGFR2, IL7R, IRF8, MPO, NOTCH3, PRTN3, TLR2) (Notta *et al.,* 2015; Paul *et al.,* 2015), were also up-regulated within Population V from NHPs (FIG. 19C). Interestingly, MPP-enriched fractions and Population VIII (FIG. 19B), containing both erythro-myeloid (EMP) and lympho-myeloid (LMP) potentials (Görgens *et al.,* 2013), demonstrated strong up-regulation of GATA2 (erythrocytes-megakaryocytes) and HDC (basophil granulocytes) in both species.

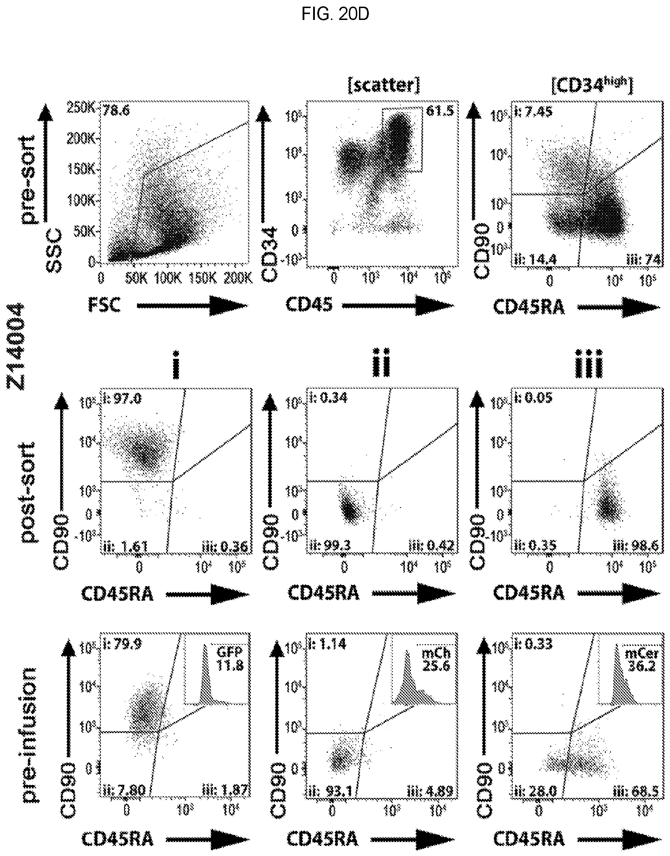

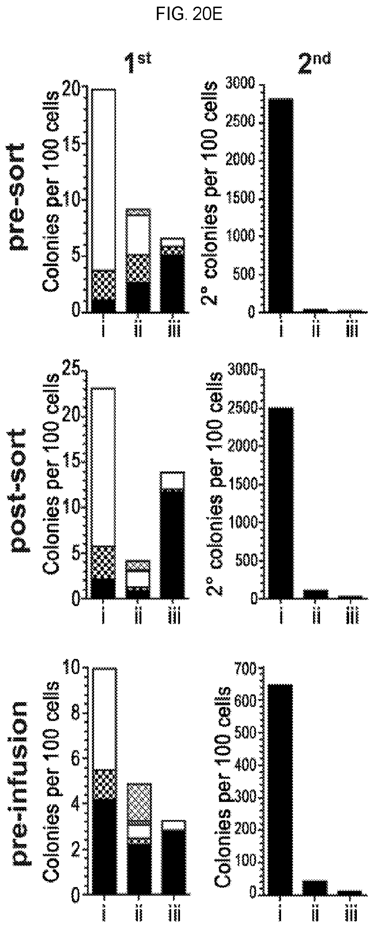

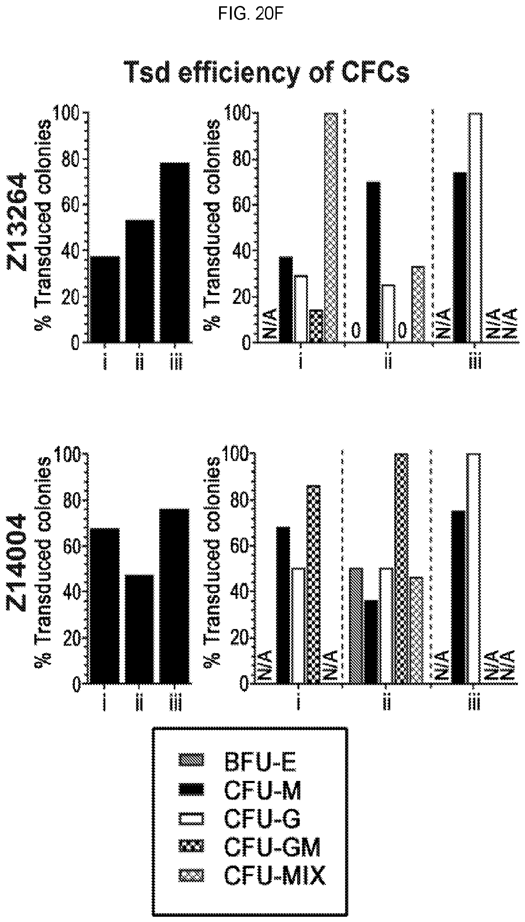

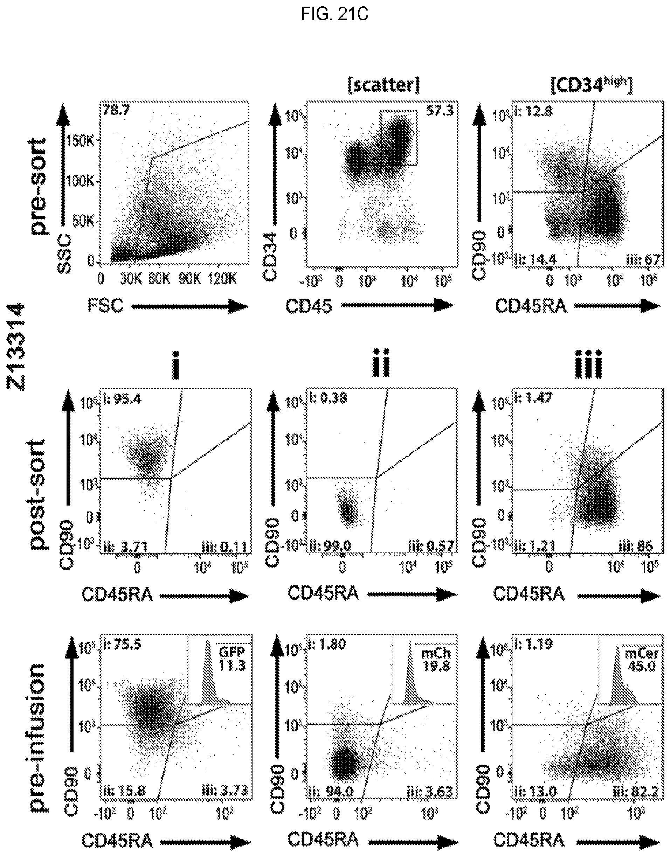

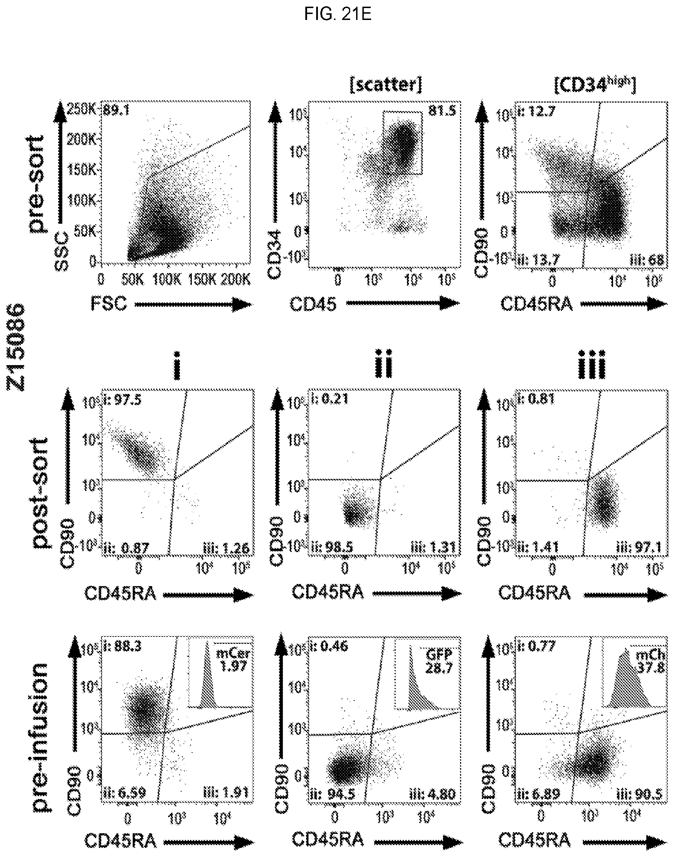

CD34⁺CD45RA⁻CD90⁺ NHP cells fractions display multilineage engraftment potential. To evaluate engraftment, competitive reconstitution experiments were performed in the myeloablative, autologous NHP stem cell transplant model (FIG. 20). Enriched CD34⁺ cells were sort-purified based on CD45RA and CD90 expression into three distinct fractions: a CD45RA⁻ CD90⁺ (fraction *i*), CD45RA⁻CD90⁻ (fraction *ii*) and CD45RA⁺CD90⁻ (fraction *iii*) (FIG. 20A, FIG. 20B, FIG. 20D, FIG. 21A, FIG. 21C, FIG. 21E). Purified fractions were then transduced with LV vectors encoding for GFP (green fluorescent protein), mCherry (mCh) or mCerulean (mCer) fluorescent proteins to independently track performance of each gene-modified cell population *in vivo* (FIG. 20C, FIG. 20D, FIG. 21A, FIG. 21C, FIG. 21E). To ensure that fluorophore protein expression did not influence *in vivo* observations in the first two animals treated, fluorophore transgenes were alternated for fractions *i, ii,* and *iii* purified from the third and fourth animal included in this Example (FIG. 20C).

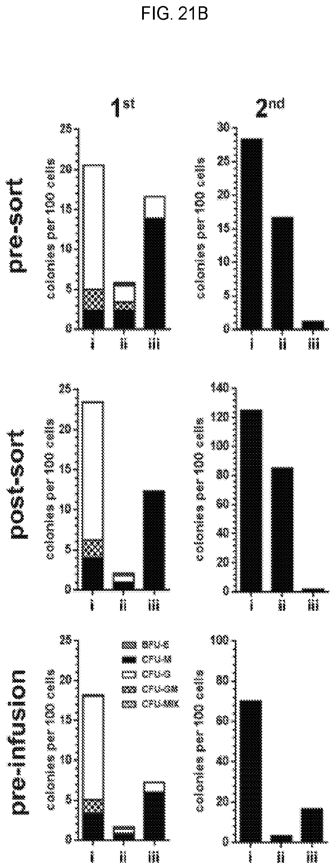

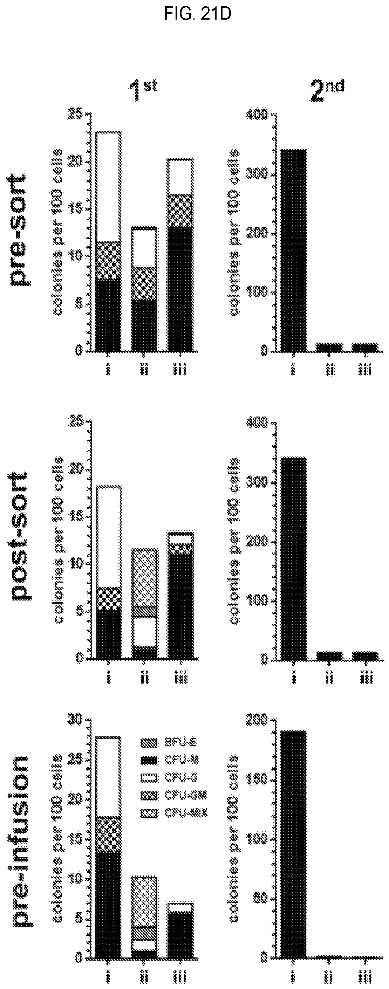

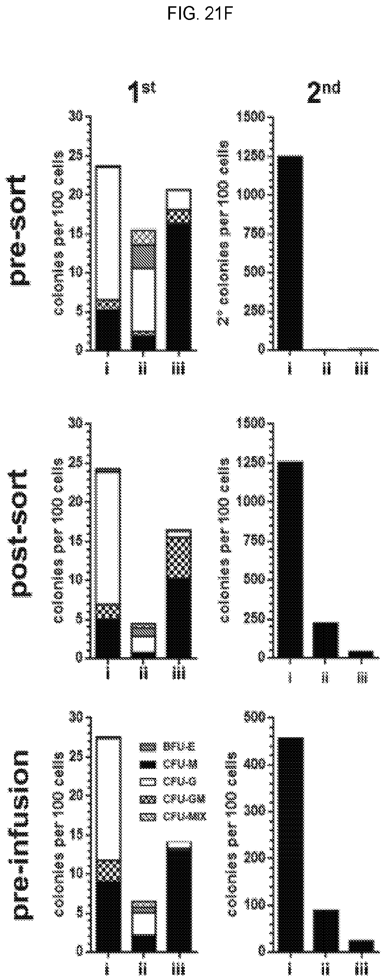

Enriched, purified and transduced cell fractions from all three NHPs maintained marker expression (FIG. 20D, FIG. 21A, FIG. 21C, FIG. 21E) as well as primary and secondary CFC potential (FIG. 20E, FIG. 21B, FIG. 21D, FIG. 21F). Lower fluorochrome expression (FIG. 20D, FIG. 21A, FIG. 21C, FIG. 21E, histogram inlay) as well as decreased transduction efficiency of CFCs (FIG. 20E, FIG. 21B, FIG. 21D, FIG. 21F) was observed for the HSC-enriched fraction (*i*) compared to more committed progenitor cells (fractions *ii* and *iii*)*.*

**All** fractions were pooled and infused into autologous recipient animals after myeloablative total body irradiation (TBI; 1020cGy). Combined fractions (*i+ii+iii*) resulted in total cell doses of 1.68 million (Z13314), 1.73 million (Z14004), 1.12 million (Z13264) and 1.84 million (Z15086) cells per kg body weight (Table 3).

**Table 3: Total number of nucleated cells in each experimental condition. TNC: total nucleated cells. Experimental conditions for each animal are listed in Table 1.**

| **Monkey** | **TNCs condition 1** | **TNCs condition 2** | **TNCs condition 3** | **SUM TNCs** |
|---|---|---|---|---|
| Z13251 | 42,000,000 | 55,000,000 | - | 97,000,000 |
| Z13125 | 15,600,000 | 12,900,000 | - | 28,000,000 |
| Z13260 | 6,500,000 | 5,100,000 | - | 11,600,000 |
| Z12434 | 21,700,000 | 34,000,000 | - | 55,700,000 |
| R11145 | 123,000,000 | 12,000,000 | - | 135,000,000 |
| Z13314 | 1,520,000 | 1,880,000 | 12,400,000 | 15,800,000 |
| Z14004 | 1,900,000 | 8,400,000 | 13,000,000 | 23,300,000 |
| Z13264 | 2,400,000 | 1,200,000 | 7,000,000 | 10,600,000 |
| Z15086 | 1,360,000 | 381,000 | 1,070,000 | 1,848,000 |
| Z13137 | 80,000,000 | - | - | 80,000,000 |
| Z14148 | 66,000,000 | - | - | 66,000,000 |
| Z14035 | 68,000,000 | - | - | 68,000,000 |
| Z14279 | 55,000,000 | - | - | 55,000,000 |
| Z14123 | 97,000,000 | - | - | 97,000,000 |
| Z14160 | 55,000,000 | - | - | 55,000,000 |

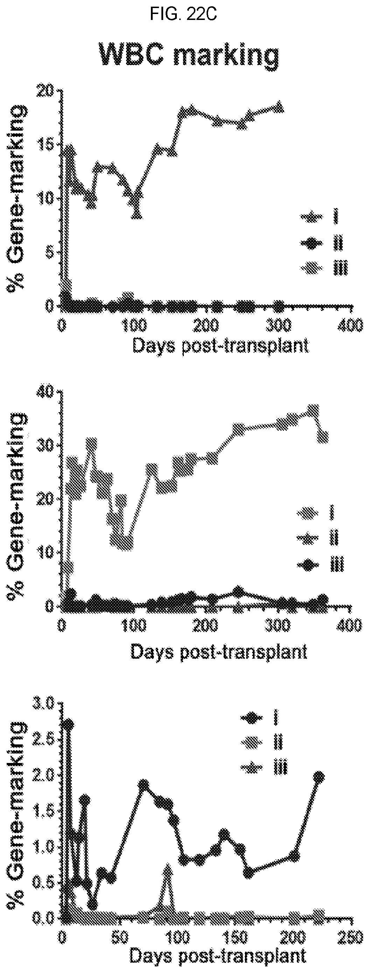

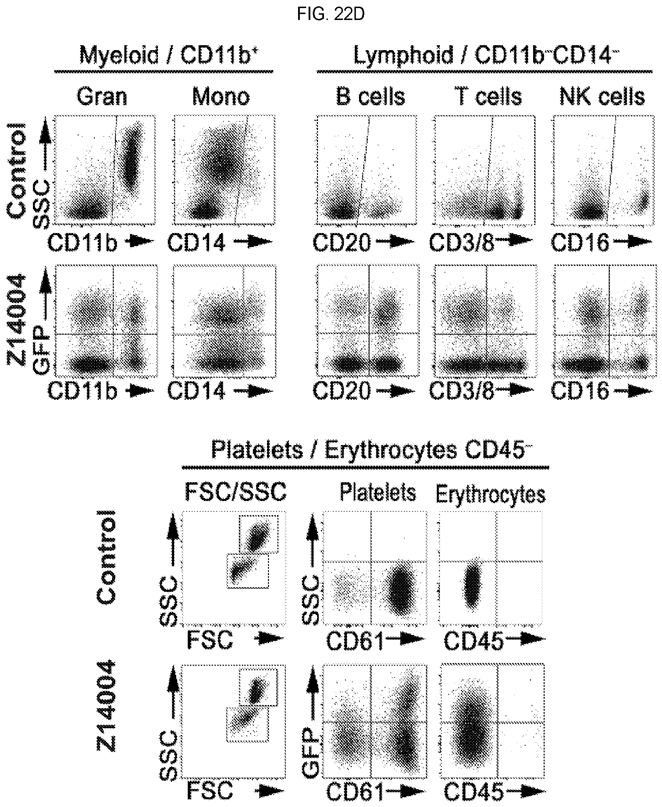

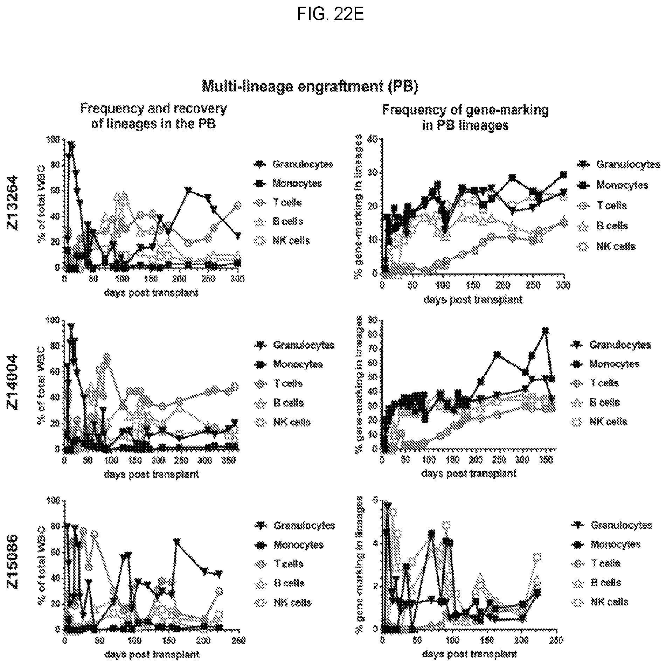

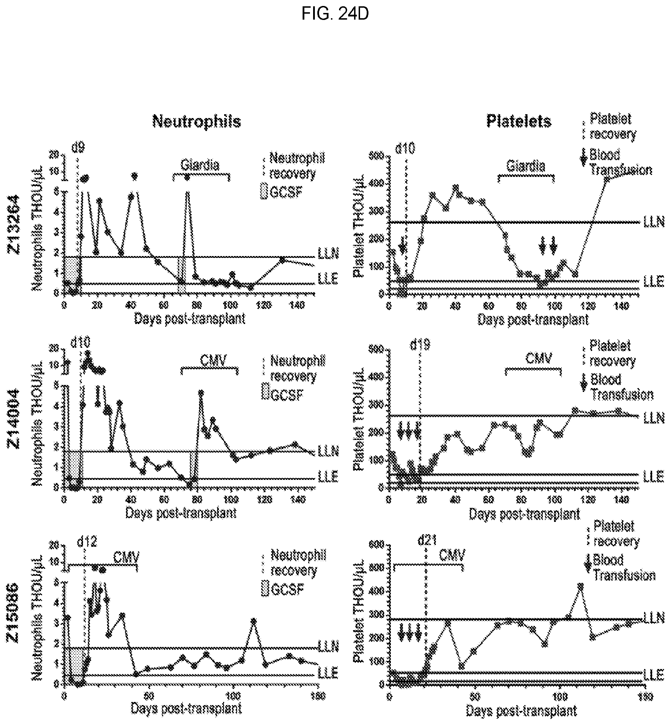

PB absolute neutrophil counts (ANC) and platelet counts rapidly recovered in all animals within 9-10 and 10-20 days, respectively (FIG. 22A, FIG. 22B, FIG. 24A, FIG. 24B, FIG. 24D). Interestingly, PB WBC in all four animals exclusively expressed fluorophore proteins assigned to fraction *i* (CD34⁺CD45RA⁻CD90⁺) cells (FIG. 22C, FIG. 24C). Detailed analysis of PB lineages showed stable gene marking in granulocytes, monocytes, B cells, NK cells, platelets and erythrocytes at similar levels 20-30 days after transplant (FIG. 22D, FIG. 22E). Fluorophore-expressing T cell levels remained low for the first few months after transplant and then started to steadily increase 100 days after infusion (FIG. 22E). Gene-modified T cells exclusively expressed fluorophore proteins assigned to fraction *i* (CD34⁺CD45RA⁻CD90⁺) cells. These observations show that the CD34⁺CD45RA⁻CD90⁺ cell fraction is capable of multilineage engraftment and initial hematopoietic recovery following myeloablative conditioning.

CD34⁺CD45RA⁻CD90⁺ cells rapidly reconstitute the bone marrow stem cell niche. Another hallmark of HSCs is the ability to migrate into and reconstitute the BM niche. Thus, the BM compartment of transplanted animals were comprehensively analyzed (FIG. 23A). BM was obtained at various time points after transplant and subjected to ammonium chloride lysis to remove red blood cells. Resulting WBC populations were assessed by flow cytometry to evaluate fluorophore transgene expression in combination with cell surface marker expression and CFC assays.

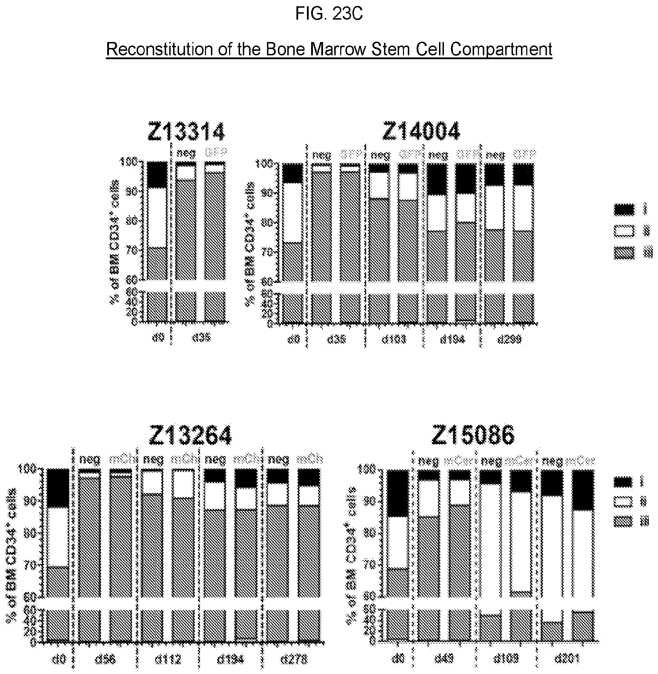

Within 5-8 weeks after transplant, all three animals demonstrated robust engraftment of both unmodified (fluorophore-) and gene-modified (fluorophore⁺) WBCs and HSPCs in the BM (FIG. 23A). Within gene modified BM, reconstitution was exclusively driven by fraction *i* (CD34⁺CD45RA⁻CD90⁺) cells (FIG. 23A). The BM HSPC compartment was initially strongly skewed towards lympho-myeloid progenitors, and then established a more balanced composition over time (FIG. 23C).

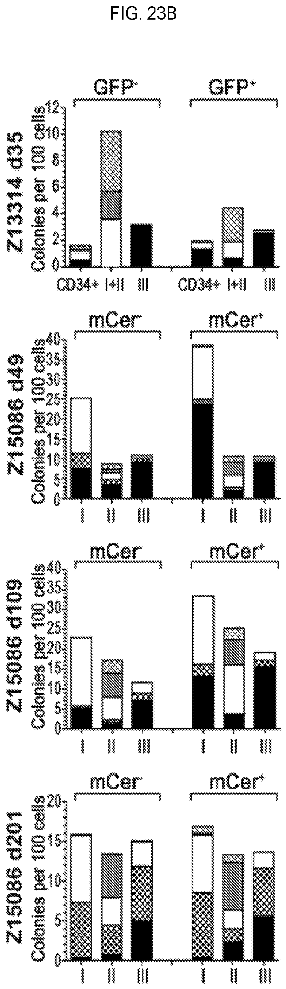

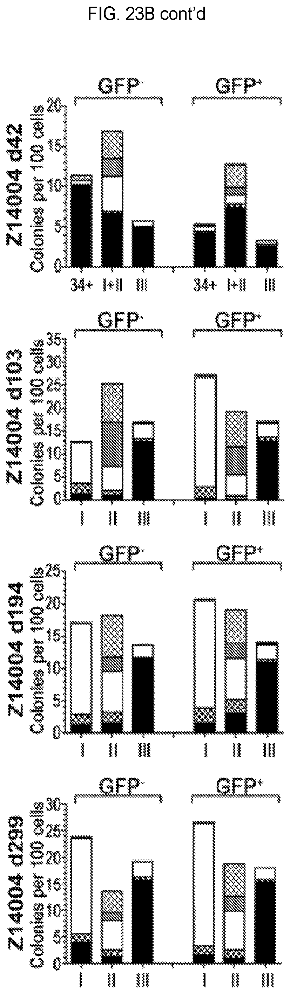

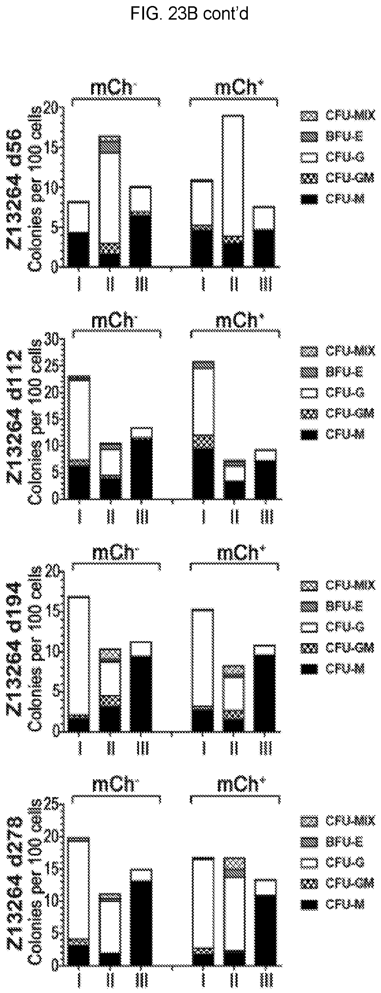

Subsequent purification and functional analysis of gene-modified and unmodified HSPC subpopulations showed similar frequencies of CFCs with an expected enrichment of CFU-Gs in fraction *i*, CFU-MIX and BFU-Es in fraction *ii,* and CFU-Ms in fraction *iii* (FIG. 23B). These data show that reconstitution of the BM niche and the recovery of the HSPC compartment in the NHP following myeloablative TBI is exclusively driven by CD34⁺CD45RA⁻CD90⁺ cells.

True Multilineage Reconstitution Demonstrated by Clonal Analysis. To test whether true multipotent HSC were represented by this early engraftment of fraction *i* cells, fluorophore⁺ PB cell lineages were sort-purified from two animals at 4 months (Z13264, mCh⁺) and 6.5 months (Z14004, GFP⁺) post-transplant for ISA (FIG. 25). Populations sorted included short-lived granulocytes (CD11b⁺CD14⁻) and monocytes (CD11b⁺CD14⁺), and long-lived B cells (CD20⁺) and T cells (CD3⁺).

Clonal analysis demonstrated highly polyclonal engraftment with 6,152 and 18,034 unique clones signatures detected for animals Z13264 and Z14004, respectively (FIG. 25). Of these, 694 (Z13264) and 546 (Z14004) were shared clone signatures between granulocytes and B cells, and 288 (Z13264) and 662 (Z14004) were shared clone signatures between monocytes and T cells, similar to what was analyzed retrospectively in long-term follow-up animals. In total, 1,577 clone signatures (25.6% of total) were shared by two or more lineages in animal Z13264, and 1,502 clone signatures (8.3% of total) were shared by two or more lineages in animal Z14004. These data confirm that fraction *i* cells conferred multilineage reconstitution *in vivo* after transplant.

Given this observation, the range of fraction *i* cell doses required for hematopoietic recovery after myeloablative transplant in this animal model was next determined.

Transplanted number of CD34⁺CD45RA⁻CD90⁺ cells per kg body weight correlates with hematopoietic recovery. To evaluate whether the number of transplanted CD34⁺CD45RA⁻CD90⁺ cells correlates with engraftment, 15 animals for whom HSPC flow cytometry data were available were retrospectively analyzed (Table 4, FIG 28A, FIG 28B). Complete blood cell counts in each animal were evaluated as a function of the transplanted CD34⁺CD45RA⁻CD90⁺ cell dose per kg of animal body weight.

**Table 4: Total number and cell count / kg body weight of transplanted CD34⁺ cells, HSC-enriched, MPP-enriched and LMP-enriched cells. HSC: hematopoietic stem cell; MPP: multipotent progenitor cell; LMP: lympho-myeloid progenitor. The total cell count of administered CD34⁺ cells, HSC-enriched, MPP-enriched and LMP-enriched cells per kg body weight was calculated by multiplying the infused total nucleated cell count (TNC) listed in Table 3 with the frequency of each individual HSPC population of the corresponding experimental condition (FIG. 28A and FIG 28B).**

| **Monkey** | **Total CD34⁺** | **CD34⁺ /kg** | **Total HSC** | **HSC /kg** | **Total MPP** | **MPP /kg** | **Total LMP** | **LMP /kg** |
|---|---|---|---|---|---|---|---|---|
| Z13251 | 4,061, 000 | 1,245, 706 | 106, 000 | 32, 515 | 501, 000 | 153, 681 | 3,159, 100 | 969,049 |
| Z13125 | 8,567, 400 | 1,978, 614 | 444, 780 | 102, 721 | 565, 032 | 130, 492 | 6,285, 816 | 1,451, 690 |
| Z13260 | 4,557, 000 | 1,064, 720 | 523, 400 | 122, 290 | 587, 900 | 137, 360 | 3,210, 900 | 750, 210 |
| Z12434 | 11,549, 400 | 3,875, 638 | 501, 542 | 168, 303 | 1,370,8 09 | 460, 003 | 9,284, 380 | 3,115, 564 |
| R11145 | 30,381, 000 | 7,410, 000 | 1,116,8 40 | 272, 400 | - | - | - | - |
| Z13314 | 6,302, 520 | 1,770, 371 | 1,339,3 00 | 376, 208 | 1,666,8 24 | 468, 209 | 2,963, 272 | 832, 380 |
| Z14004 | 8,483, 700 | 1,977, 552 | 1,723,8 00 | 401, 818 | 3,505,7 00 | 817, 179 | 2,210, 600 | 515 ,291 |
| Z13264 | 4,415, 000 | 1,152, 742 | 2,165,9 40 | 565, 520 | 490, 600 | 128, 094 | 1,649, 820 | 430, 762 |
| Z15086 | 2,198,65 9 | 785,235 | 1,450,6 19 | 518,07 8 | 180,55 4 | 64,484 | 567,48 6 | 202,674 |
| Z13137 | 22,960,0 00 | 4,647,773 | 1,249,0 24 | 252,83 9 | 1,294,9 44 | 262,13 4 | 16,531 ,200 | 3,346,39 7 |
| Z14148 | 19,338,0 00 | 5,129,443 | 1,345,9 24 | 357,00 9 | 3,732,2 34 | 989,98 2 | 12,395 ,658 | 3,287,97 3 |
| Z14035 | 23,188,0 00 | 6,441,111 | 1,790,1 13 | 497,25 4 | 1,996,4 86 | 554,57 9 | 17,321 ,436 | 4,811,51 0 |
| Z14279 | 19,205,0 00 | 5,161,765 | 1,671,8 13 | 447,00 9 | 2,567,5 65 | 686,51 5 | 13,301 ,145 | 3,556,45 6 |
| Z14123 | 28,518,0 00 | 6,632,093 | 1,579,8 97 | 367,41 8 | 2,937,3 54 | 683,10 6 | 21,873 ,306 | 5,086,81 5 |
| Z14160 | 17,215,0 00 | 3,825,556 | 986,42 | 219,20 4 | 2,289,5 95 | 508,79 9 | 11,534 ,050 | 2,563,12 2 |

From hematologic data, it was observed that clinically relevant threshold levels of neutrophils (500/mcL) and platelets (20,000/mcL) could be achieved after transplant in all animals (FIG. 26, FIG. 27), but was not indicative of long-term engraftment. Rather, there appeared to be a clear separation between the ability to maintain stable ANC and platelet counts and the extent of supportive care required over the first 3 months after transplant (FIG. 29A). In particular, two of 15 animals (Z13251 and Z13125) did not sustain platelet counts above 50,000/mcL without transfusion support over the first 100 days after transplant (FIG. 27, FIG. 29A). Additionally, while these two animals initially recovered ANC to within normal ranges for NHP with G-CSF support, following G-CSF discontinuation, ANC dropped to <500/mcL (FIG. 26, FIG. 29A). This prompted additional G-CSF support, which became less effective over the first 100 days after transplant. These two animals were defined as "engraftment failures", and the remaining five animals defined as "engraftment successes."

The transplanted dose of CD34⁺ HSPCs, CD34⁺CD45RA⁻CD90⁺ (HSC-enriched), CD34⁺CD45RA⁻CD90⁻ (MPP-enriched) and CD34⁺CD45RA⁺CD90⁻ (LMP-enriched) cells was evaluated for all animals by determining the frequency of CD34⁺CD45RA⁻CD90⁺, CD34⁺CD45RA⁻ CD90⁻ and CD34⁺CD45RA⁺CD90⁻ cells (FIG. 28A, FIG. 28B) and multiplying this by the CD34⁺ cell dose per kg of body weight at the time of transplant (Table 5 and Table 6).

**Table 5: Weight on the day of transplant (dTx), duration of GCSF treatment and time point of neutrophil/platelet engraftment. No platelet engraftment could be observed for animal Z13251 within 95 days of follow-up after transplant.**

| **Monkey** | **Kg dTx** | **GSCF treatment** | **Days post-transplant*** | **Neutrophil engraftment** | **Platelet engraftment** | **Adverse events** |
|---|---|---|---|---|---|---|
| Z13251 | 3.26 | 0-21 | 95 | 18 | - | Engraftment failure |
| Z13125 | 4.33 | 0-13 | 89 | 11 | 30 | Engraftment failure |
| Z13260 | 4.28 | 0-26 | 250 | 24 | 47 | No |
| Z12434 | 2.98 | 0-16 | 663 | 17 | 40 | No |
| R11145 | 4.10 | 0-14 | 818 | 15 | 30 | No |
| Z13314 | 3.56 | 0-11 | 38 | 10 | 20 | No long-term follow-up due to CMV infection/pre-term euthanasia |
| Z14004 | 4.29 | 0-11 | 245 | 10 | 19 | No |
| Z13264 | 3.83 | 0-11 | 194 | 9 | 10 | No |
| Z15086 | | | 91 | | | |
| Z13137 | 4.94 | 0-17 | 140 | 15 | 30 | CMV |
| Z14148 | 3.77 | 0-13 | 126 | 12 | 24 | - |
| Z14035 | 3.6 | 0-7 | 112 | 7 | 15 | Amobea, CMV |
| Z14279 | 3.74 | 0-9 | 98 | 9 | 20 | - |
| Z14123 | 4.3 | 0-11 | 63 | 10 | 21 | - |
| Z14160 | 4.5 | 0-14 | 18 | 13 | - | Kidney failure, EA |

**Table 6: Frequency of hematopoietic stem and progenitor cell populations in the bone marrow of nonhuman primates with engraftment failure and successful long-term engraftment. Data from FIG. 23A and FIG. 32.**

| **Monkey** | **Days post transplant** | **% CD34⁺ [of CD45⁺]** | **% HSC-enriched fraction [of CD45⁺/ CD34⁺]** | **% MPP-enriched fraction [of CD45⁺/ CD34⁺]** | **% LMP-enriched fraction [of CD45⁺/ CD34⁺]** |
|---|---|---|---|---|---|
| Z13251 | 90 (necropsy) | 0.07 | 0 / 0 | 0.0700 / 100 | 0 / 0 |
| Z13125 | 110 (necropsy) | 0.64 | 0.0019 / 0.29 | 0.0072 / 1.13 | 0.6099 / 95.3 |
| Z13260 | 125 | 0.80 | 0.0372 / 4.65 | 0.1648 / 20.6 | 0.5712 / 71.4 |
| Z12434 | 593 | 1.50 | 0.1875 / 12.5 | 0.1371 / 9.14 | 1.0650 / 71.0 |
| R11145 | 818 | 1.07 | 0.0869 / 8.13 | 0.3638 / 34.0 | 0.6120 / 57.2 |
| Z13314 | 35 (necropsy) | 0.62 | 0.0079 / 1.27 | 0.0244 / 3.94 | 0.5791 / 93.4 |
| Z14004 | 42 | 1.69 | 0.0137 / 0.81 | 0.0514 / 3.04 | 1.6122 / 95.4 |
| Z14004 | 103 | 1.16 | 0.0362 / 3.12 | 0.0999 / 8.62 | 0.9999 / 86.2 |
| Z14004 | 194 | 1.33 | 0.0269 / 2.02 | 0.0299 / 22.5 | 0.9762 / 73.4 |
| Z14004 | 299 | 0.81 | 0.0591 / 7.30 | 0.1238/15.3 | 0.6075 / 75.0 |
| Z13264 | 56 | 1.10 | 0.0475 / 4.32 | 0.0345 / 3.14 | 0.9317 / 84.7 |
| Z13264 | 112 | 2.51 | 0.0291 / 1.16 | 0.2226 /8.87 | 2.1912 / 87.3 |
| Z13264 | 194 | 0.95 | 0.0366 / 3.85 | 0.1862 / 19.6 | 0.6897 / 72.6 |
| Z13264 | 278 | 0.71 | 0.0331 / 4.66 | 0.0452 / 6.37 | 0.6142 / 86.5 |
| Z15086 | 49 | 0.66 | 0.0474 / 7.18 | 0.0752 / 11.4 | 0.4919 / 74.4 |
| Z15086 | 109 | 3.95 | 0.1651 / 4.18 | 1.8012 / 45.6 | 1.9078 / 48.3 |
| Z15086 | 201 | 2.45 | 0.1893 / 7.73 | 1.3573 / 55.4 | 0.8771 / 35.8 |
| Z13137 | - | - | - | - | - |
| Z14148 | - | - | - | - | - |
| Z14035 | - | - | - | - | - |
| Z14279 | - | - | - | - | - |
| Z14123 | - | - | - | - | - |
| Z14160 | - | - | - | - | - |

Animals displaying engraftment success (n = 13) received nearly 5 times the mean cell dose of the HSC-enriched cell fraction compared to animals displaying engraftment failure (n = 2; p value = 0.019), whereas no significant difference were observed for CD34⁺ or the MPP- and LMP-enriched cell fractions (FIG. 29B).

Notably, a strong correlation was observed between the CD34⁺CD45RA⁻CD90⁺ cell dose received and the onset of neutrophil and platelet recovery in animals displaying engraftment success (FIG. 29C, FIG. 29D), whereas TNC (total nucleated cells), numbers of CD34⁺ cells, CD34⁺CD45RA⁻CD90⁻ cells (MPP-enriched), CD34⁺CD45RA⁺CD90⁻ cells (LMP-enriched) (FIG. 30A, FIG. 30B) or CFCs (FIG. 31A, FIG. 31B) did not correlate with the onset or success of engraftment. These data show a minimum dose of 122,000 CD34⁺CD45RA⁻CD90⁺ cells/kg is sufficient to elicit engraftment and that these cells contribute to immediate neutrophil and platelet recovery in this transplant model.

CD34⁺CD45RA⁻CD90⁺ cells are highly enriched for CD34⁺CD38^{-/low} cells in human mobilized PBSCs and UCB. To evaluate whether the assessment of CD34 in combination with CD45RA and CD90 allows for enrichment of a similar cell fraction in humans, the strategy was tested in human GCSF-mobilized PBSCs (FIG. 33) and umbilical cord blood (UCB) (FIG. 34). "Classical gating-strategies" including CD38 (Majeti, *et al.,* 2007; Zonari, *et al.,* (2017). Efficient Ex Vivo Engineering and Expansion of Highly Purified Human Hematopoietic Stem and Progenitor Cell Populations for Gene Therapy. Stem Cell Reports) did not enrich for CD45RA⁻CD90⁺ cells (FIG. 33), whereas CD34⁺CD45RA⁻CD90⁺ fractions were highly enriched for CD38⁻ as well as CD38^{low} cells in PBSCs (FIG. 33). This enrichment of CD38⁻ cells was even greater in UCB (FIG. 34). These data show a phenotypic and transcriptional similarity of human and NHP HSC-enriched cell populations, which can be biologically discriminated by expression of CD34, CD45RA and CD90.

Example 2. Current autologous stem cell transplantation and gene therapy / edting strategies are limited by the inability to identify a true stem cell propulation that reliably predicts engraftment. For gene editing and gene therapy approaches, the ability to target a specific HSC pool and predict enrgaftment levels of the gene modified cells is a major advance, reducing the cost of goods required for manufacture, as well as the off-target effects that compromise safety and efficacy of these treatments. To do this effectively requires a rapid assay to identify and enumerate cells of interest, in this case, the HSC. Additionally, this requires data demonstrating a strong, direct correlation between the number of HSC infused and the level of engraftment after transplant.

In order to determine the quality of a stem cell product and predict the likelihood of long-term engraftment before administration, a great variety of "potency assays" have been proposed. However, identifying, characterizing and quantifying HSC populations for the treatment of hematoloigical diseases and disorders has been difficult to do *ex vivo* since the hallmark functionality of these cells is the ability to produce all blood cell lineages for the lifetime of a recipient (Harper and Rich 2015, Patterson et al. 2015, Rich 2015).

A widely used potency assay, predominantly used for banked/frozen stem cell products such as umbilical cord blood (UCB) or autologous stem cell transplants, is the CFU or CFC assay. Increased numbers of CFUs/CFCs were shown to be a predictor of potency, defined as hematopoietic recovery in myeloablated patients by 42 days after infusion (Page et al. 2011, Page et al. 2012). Unfortunately, the 2 week duration of the CFU/CFC assay does not allow for real-time quality control of fresh or thawed stem cell products immediately before infusion. Furthermore, the CFU/CFC assay is limited in its ability to read-out all heamtopoietic cell lineages. The standard assay is biased towards hematopoietic stem/progenitor cells (HSPCs) with erythroid, myleoid and erythro-myeloid differenentiation potential and does not support lymphoid lineages. To evaluate the lymphoid potential of cell products, a separate assay must be performed. In this assay, cell products are first co-cultured with a cell line and then plated into CFU/CFC assays, which lengthens the entire evaluation period even further and still does not support B cell development.

**To** overcome these major limitations of the CFU/CFC assay (i.e. slow turn around and incomplete lineage representation), many groups have assessed cell surface marker(s) in bone marrow or blood cell populations and tried to correlate these with CFU/CFC potency. Flow cytometry-based evaluation of a cell surface marker phenotype can be performed in real-time, but must be supported by in vitro functional assays, including CFU/CFC formation. However, the most convincing functional data would be in vivo data in a transplant setting. A most recent publication by Shoulars et al. describes a strong correlation (r=0.78) of CD34⁺ HSPCs expressing high levels of the enzyme aldehyde dehydrogenase (ALDH) with CFU/CFC frequencies in cryo-preserved and thawed UCB (Shoulars et al. 2016). Even though the number of ALDH^{high} expressing HSPCs has been shown to correlate with CFU/CFC frequency, correlation of transplant outcome, neutrophila/platelet recovery and multi-lineage long-term engraftment with ALDH-expression has yet to be demonstrated. Moreover, it is unkown if the ALDH^{high} correlation will apply to more common sources of CD34⁺ cells for gene and cell therapy such as bone marrow and growth factor mobilized peripheral blood.

Several groups have used animal models and transplant data in patients in an attempt to correlate potency measures with functional performance (Sartor et al. 2007, Chang et al. 2009, Chang et al. 2009, Trobridge and Kiem 2010, Watts et al. 2012, Tanner et al. 2014). These studies underscore a major problem in defining *in vivo* potency of HSC products: broad variability in the time to engraftment as described by the day of neutrophil and platelet recovery. Furthermore, current potency measures do not predict onset and kinetics of engraftment and do not define a minium number of HSC required to prevent engraftment failure.

Here it is demonstrated for the first time a flow-cytometry based strategy/potency-assay to analyze and predict the onset of neutrophil and platelet recovery in the setting of myeloablative autologous nonhuman primate HSC transplantation. While the examples herein are used in the context of myeloablative conditioning this model can be extended to alternative non-genotoxic conditioning regimens. From this data, the minimum cell number of an HSC-enriched phenotype per kg body weight required for sustained multi-lineage long-term engraftment with recovery of all blood cell lineages in this transplant setting was defined. It is envisioned this flow-cytometry based strategy/potency-assay can also be used as a quality control measure to ensure stem cell aliquots are viable for transplantation. Stem cell aliquots is an aliquot, vial, or other container storing a plurality of cells. These stem cell aliquots can be from fresh, frozen, culture-expanded, gene-modified HSCs. The importance of being able to predict engraftment prior to translantation for humans and NHPs reduces the risk of a failed transplant and has the ability to signficantly reduce medical costs associated with these failed engraftments.

See FIGs. 26-32 and associated descriptions in the Brief Description of the Figures. NHP HSPCs were isolated from either GCSF/SCF primed bone marrow (BM) or steady state BM. HSPCs were primed overnight in StemSpan SFEM media supplemented with SCF, TPO and FLT3-L (SP-STF) and transduced for 16-24 hours with lentivirus encoding for GFP, mCherry (mCh) or mCerulean (mCer). Isolated, primed and transduced HSPCs for Z13260, Z12434 and R11145 were cultured for additional 7 days in culture conditions as indicated.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment. As used herein, a material effect would cause a statistically-significant reduction in an isolated HSC population's ability to demonstrate multi-lineage potential and engraftment.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least,
each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3^{rd} Edition or a dictionary known to those of ordinary skill in the art, such as the Oxford Dictionary of Biochemistry and Molecular Biology (Ed. Anthony Smith, Oxford University Press, Oxford, 2004).

### References:

Adolfsson, et al., (2005). Cell 121, 295-306.
Akashi, et al., (2000). Nature 404, 193-197.
Alsever et al., (1941). N.Y. St. J. Med. 41, 126.
Ashwood-Smith, (1961). Nature 190, 1204-1205.
Aversa, et al., (1987). Transplantation proceedings 19, 277-278.
Beard, et al., (2007). Molecular therapy: the journal of the American Society of Gene Therapy 15, 1356-1365.
Beard, et al., (2010). The Journal of clinical investigation 120, 2345-2354.
Bender et al.., (1960). J. Appl. Physiol. 15, 520.
Bleakley et al., (2015). J Clin Invest. 125(7), 2677-89.
Bleavins, et al., (1993). Veterinary immunology and immunopathology 37, 1-13.
Bonig et al., (2009). Stem Cells 27(4), 836-7.
Brady, et al., (1995).Current biology : CB 5, 909-922.
Broxmeyer et al., (1984). J. Clin. Invest. 73, 939-953.
Bystrykh, et al., (2012). Nat Methods 9, 567-574.
Cartier et al., (2012). Methods Enzymol. 507, 187-198.
Cavazzana-Calvo et al., (2010). Nature 467, 318-322.
Chang, et al., (2009). Pediatr Blood Cancer 53(6), 1100-1106.
Chang, et al., (2009). Biol Blood Marrow Transplant 15(5), 632-638.
Chhabra et al., (2016). Hematopoietic stem cell transplantation in immunocompetent hosts without radiation or chemotherapy. Sci. Transl. Med. 8(351*)*.
Civin, et al., (1984). J Immunol 133, 157-165.
Coxon, et al., (1996). Immunity 5, 653-666.
Craddock et al., (1997). Blood 90(12), 4779-4788.
De Gowin, et al., 1940). J. Am. Med. Ass. 114, 850.
de Kruijf, et al., (2010). Haematologica 95, 1061-1067.
Donahue, et al., (2005). Current protocols in immunology / edited by John E Coligan [et al] Chapter 22, Unit 22A 21.
Doulatov, et al.,(2010). Nat Immunol 11, 585-593.
Doulatov, et al., (2012). Cell stem cell 10, 120-136.
Drize, et al., (1997).Blood 89, 1811-1817.
Drize, et al., (2001). Exp Hematol 29, 786-794.
Fleit, et al., (1982). Proc Natl Acad Sci USA 79, 3275-3279.
Fritsch, et al., (1993). Blood 81, 2301-2309.
Galy, et al., (1995). Immunity 3, 459-473.
Görgens, et al., (2014). Stem cell reports 3, 1058-1072.
Görgens, et al., (2013). Cell reports 3, 1539-1552.
Gori et al., (2012). Cancer Gene Ther. 19(8), 523-9.
Gorin, (1986). Clinics In Haematology 15(1), 19-48.
Gratwohl et al., (2010). JAMA 303(16), 1617-1624.
Gyurkocza & Sandmaier (2014). Blood 124, 344-353.
Harper and Rich (2015). Methods Mol Biol 1235, 33-48.
Harris, et al., (1984). J Immunol 132, 2502-2509.
Hocum, et al., (2015). BMC Bioinformatics 16, 212.
Horn and Kiem (2006). E Blood 108, 771; author reply 771-772.
Huber, et al., (2015). Nature methods 12, 115-121.
Hum, (1968). Storage of Blood, Academic Press, New York, pp. 26-160.
Itoh, et al.,(1989). Experimental hematology 17, 145-153.
Jacobsen, et al., (1993). Veterinary immunology and immunopathology 39, 461-466.
Jin et al., (2008). Journal of Translational Medicine 6, 39.
Jordan, et al., (1990).Genes & Development 4, 220-232.
Karpova et al., ASH 58th Annual Meeting; 659 Combined Targeting of CXCR2 and VLA4 Results in Rapid and Synergistic Mobilization of Hematopoietic Stem and Progenitor Cells in Mice. Session 771; December 5, 2016.
Katz, et al., (1985). Leukemia research 9, 191-198.
Kay et al., (2000). Nat. Genet. 24, 257.
Kent, et al.,, (2002). Genome research 12, 996-1006.
Kent, et al., (2002). BLAT--the BLAST-like alignment tool. Genome research 12, 656-664.
Kikushige, et al., (2008). J Immunol 180, 7358-7367.
Kim, et al., (2014). Cell Stem Cell 14, 473-485.
Kipps, (1989). The CD5 B cell. Adv Immunol 47, 117-185.
Kodo et al., (1984). J. Clin Invest. 73, 1377-1384.
Kohn et al., (2010). Clin. Immunol. 135, 247-54.
Kozarsky and Wilson, (1993). Curr. Opin. in Gen. and Develop. 3, 499-503.
La Motte-Mohs, et al., (1990). The Journal of experimental medicine 172, 363-366.
Lander, et al., (2001). Nature 409, 860-921.
Lansdorp, et al., (1990). The Journal of experimental medicine 172, 363-366.
Larochelle, et al., (2011). Blood 117, 1550-1554.
Lawrence, et al., (2013). Software for computing and annotating genomic ranges. PLoS computational biology 9, e1003118.
Lewis, et al., (1967). Transfusion 7(1), 17-32.
Li and Durbin, (2009). Bioinformatics 25, 1754-1760.
Li, et al., (2009). Bioinformatics 25, 2078-2079.
Linner et al., (1986). J. Histochem. Cytochem. 34(9), 1123-1135.
Liu, et al., (2015). Life Sci 122, 59-64.
Liu, et al., (1987). J Immunol 139, 3521-3526.
Livesey and Linner, (1987). Nature 327, 255.
Love, et al., (2015). RNA-Seq workflow: gene-level exploratory analysis and differential expression. F1000Research 4, 1070.
Love, et al., (2014). Genome biology 15, 550.
Lovelock, (1954). Biochem. J. 56, 265.
Lovelock and Bishop, (1959). Nature 183, 1394-1395.
Lu et al., (1986). Blood 68(1), 126-133.
Lundstrom, (1999). J. Recept. Signal Transduct. Res. 19, 673-686.
Majeti, et al., (2007). Cell stem cell 1, 635-645.
Manz, et al., (2002). Proc Natl Acad Sci U S A 99, 11872-11877.
Masiuk et al., (in press, 2017). Improving Gene Therapy Efficiency Through the Enrichment of Human Hematopoietic Stem Cells, Molecular Therapy.
Mastrangeli, et al., (1993). J. Clin. Invest. 91, 225-234.
Mazur, (1977). Cryobiology 14, 251-272.
Mazur, (1970). Science 168, 939- 949.
Mazurier, et al., (2003). Nature medicine 9, 959-963.
Meeker, et al., (1984). Hybridoma 3, 305-320.
Moingeon, et al., (1989). Immunological reviews 111, 111-144.
Nakahata, and Okumura, (1994). Leukemia & lymphoma 13, 401-409.
Nakai et al., (1998). Blood 91, 4600.
Notta, et al., (2011). Science 333, 218-221.
Notta, et al., (2015). Distinct routes of lineage development reshape the human blood hierarchy across ontogeny. Science.
Ochiai, et al., (1983). J Immunol 131, 864-868.
Page, et al., (2011). Biol Blood Marrow Transplant 17(9), 1362-1374.
Page, et al., (2012). Transfusion 52(2), 272-283.
Palchaudhuri et al., (2016). Nature Biotechnology 34, 738-745
Papayannopoulou et al., (1998). Blood 91(7), 2231-2239.
Patterson, et al., (2015). J Transl Med 13, 94.
Paul, et al., (2015). Cell 163, 1663-1677.
Pelus, (2008). Curr. Opin. Hematol. 15(4), 285-292.
Pelus & Fukuda (2006). Exp. Hematol. 34(8), 1010-20.
Peterson, et al., (2016). Long-term multi-lineage engraftment of genome-edited hematopoietic stem cells after autologous transplantation in nonhuman primates. Blood.
Phan The Tran and Bender, (1960). Exp. Cell Res. 20, 651.
Phan The Tran and Bender, (1960). Proc. Soc. Exp. Biol. Med. 104, 388.
Phan The Tran and Bender, (1961). In Radiobiology, Proceedings of the Third Australian Conference on Radiobiology, IIbery ed., Butterworth, London, p. 59.
Ploemacher, et al., (1989). Blood 74, 2755-2763.
Radtke, et al., (2015). British journal of haematology 169, 868-878.
Rapatz et al., (1968). Cryobiology 5(1), 18-25.
Reimann, et al., (1994). Cytometry 17, 102-108.
Reisner et al., (1980). Proc. Natl. Acad. Sci. USA 77, 1164.
Rich, (2015). Methods Mol Biol 1235, 7-17.
Rinfret, (1960). Ann. N.Y. Acad. Sci. 85, 576.
Rosenfeld, et al., (1991). Science 252, 431-434.
Rosenfeld, et al., (1992). Cell 68, 143-155.
Rosnet, et al., (1996). Leukemia 10, 238-248.
Rous and Turner, (1916). J. Exp. Med. 23, 219.
Rowe, (1966). Cryobiology 3(1),12-18.
Rowe et al., (1962). Fed. Proc. 21, 157.
Rowe and Rinfret, (1962). Blood 20, 636.
Santoriello and Zon, (2012).. J Clin Invest 122, 2337-2343.
Sartor, et al., (2007). Bone Marrow Transplant 40(9), 851-857.
Schmidt, et al., (2001).Annals of the New York Academy of Sciences 938, 146-155; discussion 155-146.
Schmitt and Zuniga-Pflucker (2002). Immunity 17, 749-756.
Shepherd, et al., (2007). Blood 110, 1806-1813.
Shoulars, et al., (2016). Blood 127(19), 2346-2354.
Simione, (1992). J. Parenter. Sci. Technol. 46(6), 226-32.
Sloviter and Ravdin, (1962). Nature 196, 548.
Smith, et al., (1959). J. Thorac. Cardiovasc. Surg. 38, 573.
Snodgrass, et al., (1987).EMBO Journal 6, 3955-3960.
Spitzer et al., (1980). Cancer 45, 3075-3085.
Stewart, et al., (1995). Leukocyte integrins. Curr Opin Cell Biol 7, 690-696.
Stiff et al., (1983). Cryobiology 20, 17-24.
Stocks, et al., (1990). The Biochemical journal 268, 275-280.
Takasaki, et al., (2000). Cell biology international 24, 101-108.
Tanner, et al., (2014). Stem Cells Dev 23(1), 76-82.
Taussig, et al., (2005). Blood 106, 4086-4092.
Terstappen, et al., (1991). Blood 77, 1218-1227.
Testa, et al., (2014). Biomark Res 2, 4.
Tricot et al., (2008). Haematologica 93(11), 1739-1742.
Trobridge, et al., (2008). Blood 111, 5537-5543.
Trobridge and Kiem (2010). Gene Ther 17(8), 939-948.
Vos, (1998). Curr. Op. Genet. Dev. 8, 351-359.
Walsh, et al., (1993). Proc. Soc. Exp. Bioi. Med. 204, 289-300.
Wang, et al., (1997). Blood 89, 3919-3924.
Watts, et al., (2012). Exp Hematol 40(3), 187-196.
Weaver et al., (2001). Bone Marrow Transplantation 27(2), S23-S29.
Williams et al., (1985). J. Immunol. 135, 1004.
Wu, et al., (2014). Cell Stem Cell 14, 486-499.
Yin, et al., (1997). Blood 90, 5002-5012.
Younan, et al., (2015). Molecular therapy : the journal of the American Society of Gene Therapy 23, 943-951.
Zerbino, et al., (2015). Genome biology 16, 56.
Zhang et al., (2014). Bioinformatics 30, 614-620.
Zonari, et al., (2017). Stem Cell Reports, 8(4), 977-990.

## Claims

1. A method for isolating a stem cell population for the development of a clinical treatment, the method comprising:
isolating a stem cell population that is CD34+/CD45RA-/CD90+; wherein the isolating does not utilize CD38 or CD49f, and wherein the isolating does not utilize markers other than CD34, CD45RA, CD90, CD117, CD123, and/or CD133.

2. The method of claim 1, wherein the stem cell is isolated from a stem cell source and wherein the stem cell source includes umbilical cord blood, placental blood, bone marrow, or peripheral blood.

3. The method of claim 1, wherein the isolating includes magnetic-assisted cell sorting (MACS) with an anti-CD34 antibody.

4. The method of claim 1, wherein the isolating includes magnetic separation; fluorescence activated cell sorting; nanosorting based on fluorophore expression; affinity chromatography; the use of cytotoxic agents joined to a monoclonal antibody or used in conjunction with a monoclonal antibody; panning with an antibody attached to a solid matrix; selective agglutination using a lectin; immunomagnetic bead-based sorting; or combinations thereof.

5. The method of claim 1, further comprising removing red blood cells.

6. The method of claim 1, wherein the isolated stem cell population has predictive engraftment potential as evidenced by a Spearman's Rank Correlation Coefficient (R²) score having an absolute value of 0.5 or more, 0.6 or more, 0.7 or more, 0.8 or more, or 0.9 or more.

7. A method for isolating and genetically-modifying a stem cell population for the development of a clinical treatment, the method comprising:
isolating a stem cell population that is CD34+/CD45RA-/CD90+; wherein the isolating does not utilize CD38 or CD49f, and wherein the isolating does not utilize markers other than CD34, CD45RA, CD90, CD117, CD123, and/or CD133; and
genetically-modifying the isolated stem cell population.

8. A method for isolating a stem cell population for the development of a clinical treatment and formulating the isolated stem cell population for administration to a subject, the method comprising:
isolating a stem cell population that is CD34+/CD45RA-/CD90+; wherein the isolating does not utilize CD38 or CD49f, and wherein the isolating does not utilize markers other than CD34, CD45RA, CD90, CD117, CD123, and/or CD133; and
formulating the isolated stem cell population for administration to a subject.

## Patentansprüche

1. Verfahren zum Isolieren einer Stammzellpopulation zur Entwicklung einer klinischen Behandlung, wobei das Verfahren umfasst:
Isolieren einer Stammzellpopulation, die CD34+/CD45RA-/CD90+ ist; wobei das Isolieren kein CD38 oder CD49f nutzt, und wobei das Isolieren keine Marker außer CD34, CD45RA, CD90, CD117, CD123 und/oder CD133 nutzt.

2. Verfahren nach Anspruch 1, wobei die Stammzelle aus einer Stammzellquelle isoliert wird und wobei die Stammzellquelle Nabelschnurblut, Plazentablut, Knochenmark oder peripheres Blut einschließt.

3. Verfahren nach Anspruch 1, wobei das Isolieren magnetische Zellsortierung (MACS) mit einem Anti-CD34-Antikörper einschließt.

4. Verfahren nach Anspruch 1, wobei das Isolieren magnetische Trennung; Fluoreszenz-aktivierte Zellsortierung; Nanosortierung auf der Basis von Fluorophor-Expression; Affinitätschromatographie; die Verwendung von Zytotoxika, die mit einem monoklonalen Antikörper verbunden sind oder in Verbindung mit einem monoklonalen Antikörper verwendet werden; Panning mit einem Antikörper, der an eine feste Matrix angelagert ist; selektive Agglutination unter Verwendung eines Lektins; Sortierung auf der Basis immunomagnetischer Beads oder Kombinationen davon einschließt.

5. Verfahren nach Anspruch 1, ferner umfassend ein Entfernen von roten Blutkörperchen.

6. Verfahren nach Anspruch 1, wobei die isolierte Stammzellpopulation ein prädiktives Engraftment-Potenzial, wie durch einen Score des Spearman's Rank Correlation Coefficient (R²) belegt, mit einem Absolutwert von 0,5 oder mehr, 0,6 oder mehr, 0,7 oder mehr, 0,8 oder mehr oder 0,9 oder mehr aufweist.

7. Verfahren zum Isolieren und gentechnischen Verändern einer Stammzellpopulation zur Entwicklung einer klinischen Behandlung, wobei das Verfahren umfasst:
Isolieren einer Stammzellpopulation, die CD34+/CD45RA-/CD90+ ist; wobei das Isolieren kein CD38 oder CD49f nutzt, und wobei das Isolieren keine Marker außer CD34, CD45RA, CD90, CD117, CD123 und/oder CD133 nutzt; und
gentechnisches Verändern der isolierten Stammzellpopulation.

8. Verfahren zum Isolieren einer Stammzellpopulation zur Entwicklung einer klinischen Behandlung und Formulieren der isolierten Stammzellpopulation zur Verabreichung an einen Probanden, wobei das Verfahren umfasst:
Isolieren einer Stammzellpopulation, die CD34+/CD45RA-/CD90+ ist; wobei das Isolieren kein CD38 oder CD49f nutzt, und wobei das Isolieren keine Marker außer CD34, CD45RA, CD90, CD117, CD123 und/oder CD133 nutzt; und
Formulieren der isolierten Stammzellpopulation zur Verabreichung an einen Probanden.

## Revendications

1. Procédé d'isolement d'une population de cellules souches pour le développement d'un traitement clinique, le procédé comprenant :
l'isolement d'une population de cellules souches qui est CD34+/CD45RA-/CD90+ ; l'isolement n'utilisant pas CD38 ou CD49f, et l'isolement n'utilisant pas de marqueurs autres que CD34, CD45RA, CD90, CD117, CD123 et/ou CD133.

2. Procédé selon la revendication 1, dans lequel la cellule souche est isolée à partir d'une source de cellules souches et dans lequel la source de cellules souches comprend du sang de cordon ombilical, du sang placentaire, de la moelle osseuse ou du sang périphérique.

3. Procédé selon la revendication 1, dans lequel l'isolement comprend un tri cellulaire magnétique (MACS) avec un anticorps anti-CD34.

4. Procédé selon la revendication 1, dans lequel l'isolement comprend une séparation magnétique ; un tri cellulaire activé par fluorescence ; un tri à l'échelle nanométrique basé sur l'expression de fluorophores ; une chromatographie d'affinité ; l'utilisation d'agents cytotoxiques joints à un anticorps monoclonal ou utilisés conjointement avec un anticorps monoclonal ; un panning avec un anticorps fixé à une matrice solide ; une agglutination sélective à l'aide d'une lectine ; un tri immunomagnétique avec des billes ; ou une combinaison de ceux-ci.

5. Procédé selon la revendication 1, comprenant en outre une élimination des globules rouges.

6. Procédé selon la revendication 1, dans lequel la population de cellules souches isolée présente un potentiel de greffe prédictif mis en évidence par une note de coefficient de corrélation de rang de Spearman (R²) ayant une valeur absolue de 0,5 ou plus, 0,6 ou plus, 0,7 ou plus, 0,8 ou plus, ou 0,9 ou plus.

7. Procédé d'isolement et de modification génétique d'une population de cellules souches pour le développement d'un traitement clinique, le procédé comprenant :
l'isolement d'une population de cellules souches qui est CD34+/CD45RA-/CD90+ ; l'isolement n'utilisant pas CD38 ou CD49f, et l'isolement n'utilisant pas de marqueurs autres que CD34, CD45RA, CD90, CD117, CD123 et/ou CD133 ; et
une modification génétique de la population de cellules souches isolée.

8. Procédé d'isolement d'une population de cellules souches pour le développement d'un traitement clinique et de formulation de la population de cellules souches isolée pour une administration à un sujet, le procédé comprenant :
l'isolement d'une population de cellules souches qui est CD34+/CD45RA-/CD90+ ; l'isolement n'utilisant pas CD38 ou CD49f, et l'isolement n'utilisant pas de marqueurs autres que CD34, CD45RA, CD90, CD117, CD123 et/ou CD133 ; et
une formulation de la population de cellules souches isolée pour une administration à un sujet.
